# EUROPEAN PATENT APPLICATION

(11) **EP 4 620 948 A1**
(43) Date of publication of application: **24.09.2025**
(21) Application number: 23891848.6
(22) Date of filing: 20.10.2023
(51) Int. Cl.: C07C 275/32, C07C 335/08, C07D 207/337, C07D 233/90, C07D 209/18, C07D 239/52, C07D 473/34, C07D 473/30, C07D 239/47, A61K 9/51

(54) **LIPID COMPOUND AND COMPOSITION FOR DELIVERY OF ACTIVE SUBSTANCE**

(30) Priority: 18.11.2022 KR 20220155135
(71) Applicant: Korea Research Institute of Bioscience and Biotechnology, Daejeon 34141 (KR)
(72) Inventor: KIM, Yoonkyung, Daejeon 34141 (KR); JEON, Hae-Geun, Daejeon 34141 (KR); JUNG, Hye-youn, Daejeon 34141 (KR); HO, Viet Cuong, Daejeon 34141 (KR); LEE, Ji Yoon, Daejeon 34141 (KR)
(74) Representative: Vossius & Partner Patentanwälte Rechtsanwälte mbB
(86) International application number: PCT/KR2023/016429
(87) International publication number: WO 2024/106781

(57) **Abstract**

Disclosed are a new lipid compound and a new lipid nanoparticle (LNP) composition comprising the lipid compound. The lipid compound according to the present disclosure may enhance the structural stability of an active substance by directly or indirectly binding to the active substance through multivalent interactions to encapsulate the active substance. In addition, the lipid nanoparticle composition comprising the lipid compound may significantly improve the intracellular delivery efficiency and activity of the active substance, therefore may be useful for the treatment and prevention of the disease.

## Description

### Technical Field

The present disclosure relates to a lipid compound delivering an active substance into a cell in vivo and a lipid nanoparticle composition comprising the lipid compound.

### Background Art

Due to the urgent circumstances caused by the recent COVID-19 pandemic and the successive emergence of new variant viruses, technologies related to mRNA vaccines-which allow for faster development and greater versatility than traditional vaccine approaches-have advanced rapidly. In particular, mRNA vaccines have been the focus of intensive research over the past decade. In addition to SARS-CoV-2, they are being developed as vaccines for influenza, Zika virus, rabies, respiratory syncytial virus (RSV), cytomegalovirus (CMV), and for cancer. Compared to traditional vaccines that contain pathogens (such as viruses), mRNA vaccines contain only the mRNA encoding the genetic information of the virus, making them significantly safer due to their non-infectious nature.

In the case of COVID-19 mRNA vaccines, they primarily contain mRNA encoding the spike protein on the surface of SARS-CoV-2. The mRNA administered in vivo is delivered into the cytoplasm of cells to express the spike protein, and this protein functions as an antigen to produce antibodies against the virus. Since mRNA vaccines can be manufactured by modifying the mRNA nucleotide sequence according to the genetic information of the virus, the development and production of these vaccines are very fast, making them efficient in responding to variant viruses. Accordingly, compared to traditional vaccines, mRNA vaccines offer advantages in terms of versatility, flexibility, production speed, and cost-effectiveness.

However, mRNA is structurally very vulnerable and can be easily degraded by enzymes such as RNase (ribonuclease), and its genetic information may be compromised depending on the storage conditions. Therefore, it is essential to have a delivery system that fully protects the structure of mRNA to maximize vaccine efficacy and enhances mRNA delivery into the cytoplasm through high intracellular delivery efficiency and efficient endosomal escape. As mRNA delivery systems, viral vectors and lipid nanoparticles (LNPs) are commonly used. However, viral vectors raise concerns regarding immune responses, potential long-term toxicity, and limited cargo capacity (*i.e.,* mRNA size). As a result, lipid nanoparticles (LNPs) are gaining attention as the most promising mRNA delivery system.

In particular, the recent experience with COVID-19 has led to the growing demand for next-generation LNP delivery systems that can enhance the structural stability of mRNA improve the stability of mRNA-LNPs, and increase vaccine efficacy and safety, and enable targeted delivery to specific tissues or sites within the body. To meet these needs, research is actively being conducted around the world to develop new lipid compounds and LNP formulations containing them.

### Detailed Description of the Invention

### Technical Problem

An aspect of the present disclosure is to provide a lipid compound that can enhance binding affinity with active substances and stability, and a lipid nanoparticle composition comprising the lipid compound.

Another aspect of the present disclosure is to provide a lipid nanoparticle composition comprising the lipid compound.

Another aspect of the present disclosure is to provide a method for preparing active substance-lipid nanoparticles.

Another aspect of the present disclosure is to provide a pharmaceutical composition, including the lipid nanoparticle composition and a pharmaceutically acceptable carrier.

Another aspect of the present disclosure is to provide a method for prevention or treatment of a disease, including the step of administering the lipid nanoparticle composition to a subject in need of the prevention or treatment of the disease.

### Technical Solution

To achieve the above goal, the present disclosure provides a lipid compound of the following Formula 1, an isomer thereof, or a salt thereof:

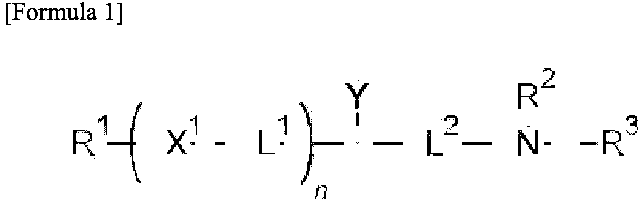

Wherein,
R¹ is hydrogen, hydroxy, C₁₋₆ alkyl, C₂₋₆ alkenyl, C₁₋₆ alkoxy, C₂₋₆ alkenyloxy, - CN, - NO₂, -N(R)₂, -C(=O)R, -C(=O)OR, -OC(=O)R, -C(=O)N(R)₂, -NRC(=O)R, - NRC(=O)N(R)₂, -NRC(=S)N(R)₂, C₃₋₁₄ cycloalkyl, 5- to 10-membered heteroaryl, 3- to 14-membered heterocyclyl, -O-5- to 10-membered heteroaryl, or -O-3- to 14-membered heterocyclyl, wherein C₁₋₆ alkyl, C₂₋₆ alkenyl, C₁₋₆ alkoxy, C₂₋₆ alkenyloxy, C₃₋₁₄ cycloalkyl, 5- to 10-membered heteroaryl, 3- to 14-membered heterocyclyl, -O-5- to 10-membered heteroaryl, or -O-3- to 14-membered heterocyclyl may be each independently unsubstituted or substituted with 1 to 3 halogens or C₁₋₆ alkyl;
X¹ is a simple bond, -CO-C₁₋₆ alkyl, -CO-C₂₋₆ alkenyl, -C(=O)NR-, -NRC(=O)-, - NRC(=O)NR-, -NRC(=S)NR-, C₆₋₁₀ aryl, C₃₋₁₄ cycloalkyl, 5- to 10-membered heteroaryl, 3- to 14-membered heterocyclyl, a nucleobase, an amino acid monomer, or an amino acid oligomer, wherein -CO-C₁₋₆ alkyl, -CO-C₂₋₆ alkenyl, C₆₋₁₀ aryl, C₃₋₁₄ cycloalkyl, 5- to 10-membered heteroaryl, 3- to 14-membered heterocyclyl, a nucleobase, an amino acid monomer, and an amino acid oligomer may be each independently unsubstituted or substituted with 1 to 3 halogens or C₁₋₆ alkyl;
L¹ is C₁₋₁₄ alkylene, C₂₋₁₄ alkenylene, M, R^{a}M, MR^{a}, MR^{a}M¹, or R^{a}MR^{b};
*n* is an integer from 1 to 5, and in case *n* is from 2 to 5, X¹ and L¹ are each independently selected;
L² is C₁₋₁₄ alkylene, C₂₋₁₄ alkenylene, M, R^{a}M, MR^{a}, MR^{a}M¹, or R^{a}MR^{b}, and L² is the same as or different from L¹;
Y is hydrogen, -(CH₂)ₘ OH, -(CH₂)ₘ SH, or -(CH₂)ₘ SeH, wherein m is an integer from 0 to 5;
R² and R³ are each independently C₁₋₃₀ alkyl, C₂₋₃₀ alkenyl, or R^{c}MR^{d}, wherein C₁₋₃₀ alkyl and C₂₋₃₀ alkenyl are each independently unsubstituted or substituted with 1 to 3 C₁₋₁₆ alkyl or C₂₋₁₆ alkenyl;
M and M¹ are each independently -NHC(=O)-, -C(=O)NH-, -C(=O)O-, -OC(=O)-, - C(=O)-, -NH-, -S-, -SS-, -O-, -S(O)₂-, -C(=O)S-, -SC(=O)-, -NHC(=O)NH-, - NHC(=O)O-, or -OC(=O)NH-;
R is each independently hydrogen, C₁₋₆ alkyl, C₂₋₆ alkenyl, C₃₋₁₄ cycloalkyl, 5- to 10-membered heteroaryl, or 3- to 14-membered heterocyclyl;
R^{a} and R^{b} are independently -(CH₂)₁-, -C₃₋₂₀ cycloalkyl-(CH₂)₁-, -(CH₂)₁-C₃₋₂₀ cycloalkyl-, -C₆₋₂₀ aryl-(CH₂)₁-, -(CH₂)₁-C₆₋₂₀ aryl-, -NH-(CH₂)₁-, or -(CH₂)-, wherein 1 is an integer from 0 to 10;
R^{c} is C₁₋₁₄ alkylene or C₂₋₁₄ alkenylene;
R^{d} is C₁₋₂₀ alkyl, C₂₋₂₀ alkenyl, or hydrogen, wherein C₁₋₂₀ alkyl and C₂₋₂₀ alkenyl are each independently unsubstituted or substituted with 1 to 3 C₁₋₂₀ alkyl or C₂₋₂₀ alkenyl;

The heteroaryl is an aromatic heterocycle compromising 1 to 6 heteroatoms selected from N, O, and S, and the heterocycle is an aliphatic heterocycle compromising 1 to 6 heteroatoms selected from N, O, and S.

### Advantageous Effects

The lipid compound according to the present disclosure may enhance the structural stability of an active substance by directly or indirectly binding to the active substance through multivalent interactions to encapsulate the active substance. The lipid nanoparticle composition comprising the lipid compound may significantly improve the intracellular delivery efficiency and activity of the active substance.

### Brief Description of Drawings

FIG. 1 illustrates the structure of an mRNA-LNP and examples of the structures of conventional lipid compounds that constitute the mRNA-LNP.
FIG. 2 illustrates the binding profile between mRNA and a lipid compound according to an embodiment of the present disclosure.
FIG. 3 is a series of graphs illustrating the results of measuring the apparent p*K*ₐ of mRNA-LNPs prepared according to an embodiment of the present disclosure.
FIG. 4 is a series of graphs illustrating the results of measuring the hydrodynamic diameter and polydispersity index (PDI) of mRNA-LNPs prepared according to an embodiment of the present disclosure.
FIG. 5 is a series of graphs illustrating the results of measuring the surface charge (zeta potential) of mRNA-LNPs prepared according to an embodiment of the present disclosure.
FIG. 6 is a series of whole-body images of mice intramuscularly injected with fLuc mRNA-LNPs prepared according to an embodiment of the present disclosure. The images were taken at various time points after administration using an in vivo imaging system for small animals (IVIS Lumina III).
FIG. 7 is a graph illustrating the results of quantitative analysis of the luminescence images obtained from the experiment shown in FIG. 6.
FIG. 8 is a series of graphs illustrating the measured mRNA levels of four markers related to immune responses induced by LNPs in cells treated with mRNA-LNPs prepared according to an embodiment of the present disclosure.
FIG. 9 is a series of graphs illustrating the results of analyzing five toxicity markers related to the liver and kidneys. These markers were measured from blood samples collected after a certain period of time following the in vivo administration of mRNA-LNPs prepared according to an embodiment of the present disclosure.
FIG. 10 is a series of graphs illustrating the measured size, polydispersity index (PDI), and encapsulation efficiency of LNP samples according to the elapsed time after preparation and the temperature conditions of mRNA-LNPs prepared according to an embodiment of the present disclosure.

### Best Mode for Carrying out the Invention

To achieve the goal, the present disclosure provides a lipid compound of the following Formula 1, an isomer thereof, or a salt thereof:

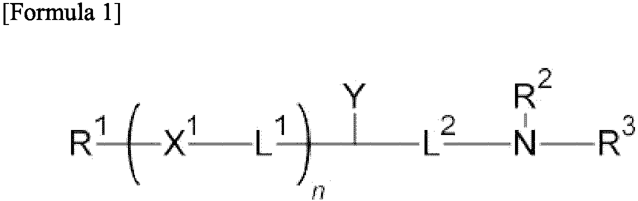

Wherein,
R¹ is hydrogen, hydroxy, C₁₋₆ alkyl, C₂₋₆ alkenyl, C₁₋₆ alkoxy, C₂₋₆ alkenyloxy, - CN, - NO₂, -N(R)₂, -C(=O)R, -C(=O)OR, -OC(=O)R, -C(=O)N(R)₂, -NRC(=O)R, - NRC(=O)N(R)₂, -NRC(=S)N(R)₂, C₃₋₁₄ cycloalkyl, 5- to 10-membered heteroaryl, 3- to 14-membered heterocyclyl, -O-5- to 10-membered heteroaryl, or -O-3- to 14-membered heterocyclyl, wherein C₁₋₆ alkyl, C₂₋₆ alkenyl, C₁₋₆ alkoxy, C₂₋₆ alkenyloxy, C₃₋₁₄ cycloalkyl, 5- to 10-membered heteroaryl, 3- to 14-membered heterocyclyl, -O-5- to 10-membered heteroaryl, or -O-3- to 14-membered heterocyclyl may be each independently unsubstituted or substituted with 1 to 3 halogens or C₁₋₆ alkyl;
X¹ is a simple bond, -CO-C₁₋₆ alkyl, -CO-C₂₋₆ alkenyl, -C(=O)NR-, -NRC(=O)-, - NRC(=O)NR-, -NRC(=S)NR-, C₆₋₁₀ aryl, C₃₋₁₄ cycloalkyl, 5- to 10-membered heteroaryl, 3- to 14- membered heterocyclyl, a nucleobase, an amino acid monomer, or an amino acid oligomer, wherein -CO-C₁₋₆ alkyl, -CO-C₂₋₆ alkenyl, C₆₋₁₀ aryl, C₃₋₁₄ cycloalkyl, 5- to 10-membered heteroaryl, 3- to 14-membered heterocyclyl, a nucleobase, an amino acid monomer, and an amino acid oligomer may be each independently unsubstituted or substituted with 1 to 3 halogens or C₁₋₆ alkyl;
L¹ is C₁₋₁₄ alkylene, C₂₋₁₄ alkenylene, M, R^{a}M, MR^{a}, MR^{a}M¹, or R^{a}MR^{b};
*n* is an integer from 1 to 5, and in case *n* is from 2 to 5, X¹ and L¹ are each independently selected;
L² is C₁₋₁₄ alkylene, C₂₋₁₄ alkenylene, M, R^{a}M, MR^{a}, MR^{a}M¹ or R^{a}MR^{b}, and L² is the same as or different from L¹;
Y is hydrogen, -(CH₂)ₘOH, -(CH₂)ₘSH, or -(CH₂)ₘSeH, wherein m is an integer from 0 to 5;
R² and R³ are each independently C₁₋₃₀ alkyl, C₂₋₃₀ alkenyl, or R^{c}MR^{d}, wherein C₁₋₃₀ alkyl and C₂₋₃₀ alkenyl are each independently unsubstituted or substituted with 1 to 3 C₁₋₁₆ alkyl or C₂₋₁₆ alkenyl;
M and M¹ are each independently -NHC(=O)-, -C(=O)NH-, -C(=O)O-, -OC(=O)-, - C(=O)-, -NH-, -S-, -SS-, -O-, -S(O)₂-, -C(=O)S-, -SC(=O)-, -NHC(=O)NH-, - NHC(=O)O-, or -OC(=O)NH-;
R is each independently hydrogen, C₁₋₆ alkyl, C₂₋₆ alkenyl, C₃₋₁₄ cycloalkyl, 5- to 10-membered heteroaryl, or 3- to 14-membered heterocyclyl;
R^{a} and R^{b} are independently -(CH₂)₁-, -C₃₋₂₀ cycloalkyl-(CH₂)₁-, -(CH₂)₁-C₃₋₂₀ cycloalkyl- , -C₆₋₂₀ aryl-(CH₂)₁-, -(CH₂)₁-C₆₋₂₀ aryl-, -NH-(CH₂)₁ -, or -(CH₂)-, wherein 1 is an integer from 0 to 10;
R^{c} is C₁₋₁₄ alkylene or C₂₋₁₄ alkenylene;
R^{d} is C₁₋₂₀ alkyl, C₂₋₂₀ alkenyl, or hydrogen, wherein C₁₋₂₀ alkyl and C₂₋₂₀ alkenyl are each independently unsubstituted or substituted as 1 to 3 C₁₋₂₀ alkyls or C₂₋₂₀ alkenyl;

The heteroaryl is an aromatic heterocycle compromising 1 to 6 heteroatoms selected from N, O, and S, and the heterocycle is an aliphatic heterocycle compromising 1 to 6 heteroatoms selected from N, O, and S.

In another aspect, the present disclosure provides a lipid nanoparticle composition comprising a lipid compound of the Formula 1, an isomer thereof, or a salt thereof.

### Mode for Carrying out the Invention

Below, a detailed description will be given of the present disclosure.

Unless otherwise stated, the term "halogen" as used herein refers to F, Cl, Br, or I.

The term "hydroxy" refers to -OH group.

The term "alkyl" as used herein refers to a linear or branched saturated hydrocarbon functional group. Specifically, "C₁₋₆ alkyl" contains 1 to 6 carbon atoms. Specifically, C₁₋₆ alkyl includes, but is not limited to, methyl, ethyl, n-propyl, isopropyl, n-butyl, isobutyl, tert-butyl, sec-butyl, n-pentyl, isopentyl, neopentyl, tert-pentyl, 1-methylbutyl, n-hexyl, 1-methylpentyl, 2-methylpentyl, 4-methyl-2-pentyl, 3,3-dimethylbutyl, 2-ethylbutyl, and the like. The alkyl group may be independently substituted with one or more substituents, for example, a methyl group may be substituted with 1 to 3 halogens or a functional group such as alcohol, thiol, selenol, C₁₋₆ alkyl, and other hydrocarbon groups.

The term "alkylene"(-CH₂CH₂-) refers to a linear or branched saturated hydrocarbon chain without hydrogen at both ends such as ethylene (-CH₂CH₂-) and propylene (-CH₂CH₂CH₂-). The alkylene group may be substituted with one or more substituents.

The term "alkenyl" refers to a linear or branched unsaturated hydrocarbon group comprising one or more double bonds, and the alkenyl group may be independently substituted with one or more substituents. For example, "C₂₋₆ alkenyl" contains 2 to 6 carbon atoms. Specifically, C₁₋₆ alkenyl includes, but is not limited to, ethenyl (vinyl group), n-propenyl, n-butenyl, 1-methyl-2-propenyl, 2-methyl-2-propenyl, n-pentenyl, 1-methyl-2-butenyl, 2-methyl-2-butenyl, n-hexenyl, and the like.

The term "alkenylene" refers to a linear or branched unsaturated hydrocarbon chain without hydrogen at both ends such as propenylene (-CH=CHCH₂-) and 2-pentenylene (-CH₂CH=CHCH₂CH₂-). The alkenylene group may be independently substituted with one or more substituents.

The term "alkoxy" refers to chemical formula '-O-alkyl' and the alkoxy group may be independently substituted with one or more substituents. For example, C₁₋₆ alkoxy includes, but is not limited to, methoxy, ethoxy, n-propyloxy, isopropyloxy, n-butoxy, isobutoxy, tert-butoxy, n-pentoxy, isopentoxy, tert-pentoxy, sec-pentoxy, neopentoxy, hexyloxy, and the like. In an embodiment, alkoxy group may be substituted with one or more substituents, for instance, 1 to 3 halogens, hydroxy, C₁₋₆ alkyl, and other hydrocarbon groups.

The term "alkenyloxy" may refer to chemical formula '-O-alkenyl', and the alkenyloxy may be independently substituted with one or more substituents. For example, C₂₋₆ alkenyloxy includes, but is not limited to, ethenyloxy, n-propenyloxy, isopropenyloxy, n-butenyloxy, 1- methyl-2-propenyloxy, 2-methyl-2-propenyloxy, n-pentenyloxy, 1-methyl-2-butenyloxy, 2-methyl-2-butenyloxy, n-hexenyloxy, and the like. In an embodiment, alkenyloxy group may be substituted with one or more substituents, for instance, 1 to 3 halogens, hydroxy, C₁₋₆ alkyl, and other hydrocarbon groups.

The term "cycloalkyl" refers to one or more saturated rings or one or more non-aromatic ring hydrocarbon groups, wherein the non-aromatic rings may contain some degree of unsaturation. Unless otherwise defined, the cycloalkyl may be a simple ring or polycyclic ring. Wherein, the polycyclic ring includes all of the fused rings, bridged rings, and spiro rings. The cycloalkyl group may be arbitrarily substituted with one or more substituents. In an embodiment of the present disclosure, "C₃₋₁₄ cycloalkyl" refers to cycloalkyl of 3 to 14 carbon atoms forming the ring. In an embodiment, 0, 1, 2, 3, or 4 atoms of each ring of the cycloalkyl group may be substituted with a substituent. Representative example of C₃₋₁₄ cycloalkyl group includes, but is not limited to, cyclopropyl, cyclobutyl, cyclopentyl, cyclohexyl, cycloheptyl, cyclopentenyl, cyclopentadienyl, cyclohexenyl, cyclohexadienyl, cyclooctyl, bicyclo[1.1.0]butyl, bicyclo[2.2.1]heptyl, adamantyl, and the like.

The term "aryl" refers to a monocyclic or bicyclic aromatic ring group. In other words, unless otherwise defined, the present description may include phenyl, naphthyl, biaryl, and the like. In an embodiment of the present disclosure, C₆₋₁₀ aryl refers to an aromatic ring of 6 to 10 carbon atoms. In an embodiment, 0, 1, 2, 3, 4, 5, or 6 atoms of each ring of the aryl group may be substituted with a substituent.

The term "heteroaryl" refers to aromatic 5- to 10- membered monocyclic or bicyclic heterocycle comprising 1 to 6 heteroatoms selected from N, O, and S. In other words, heteroaryl may be aromatic 5- or 6-membered aromatic heterocycle comprising 1 to 6 heteroatoms selected from N, O, and S, or the heteroaryl ring may be a bicyclic ring fused with a benzene ring or other heteroaryl rings. In an embodiment, 0, 1, 2, 3, or 4 atoms of each ring of the heteroaryl group may be substituted with a substituent. Representative example of the heteroaryl is, but not is limited to, pyrrolyl, furanyl, thiophenyl, pyrazolyl, imidazolyl, isoxazolyl, oxazolyl, isothiazolyl, thiazolyl, 1,2,3-triazolyl, 1,3,4-triazolyl, 1-oxa-2,3-diazolyl, 1-oxa-2,4-diazolyl, 1-oxa-2,5-diazolyl, 1-oxa-3,4-diazolyl, 1-thia-2,3-diazolyl, 1-thia-2,4-diazolyl, 1-thia-2,5-diazolyl, 1-thia-3,4-diazolyl, tetrazolyl, pyridinyl, pyridazinyl, pyrimidinyl, pyrazinyl, indolyl, isoindolyl, benzofuranyl, benzothiophenyl, indazolyl, benzimidazolyl, benzotriazolyl, pyrrolo[2,3-b]pyridinyl, pyrrolo[2,3-c]pyridinyl, pyrrolo[3,2-c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-c]pyridinyl, pyrazolo[3,4-c]pyridinyl, pyrazolo[3,4-b]pyridinyl, purinyl, indolizinyl, imidazo[1,2-a]pyridinyl, imidazo[1,5-a]pyridinyl, pyrazolo[1,5-a]pyridinyl, pyrrolo[1,2-b]pyridazinyl, imidazo[1,2-c]pyrimidinyl, quinolinyl, isoquinolinyl, cinnolinyl, azaquinazolinyl, quinoxalinyl, phthalazinyl, 1,6-naphthyridinyl, 1,7-naphthyridinyl, 1,8-naphthyridinyl, 1,5-naphthyridinyl, 2,6-naphthyridinyl, 2,7-naphthyridinyl, pyrido[3,2-d]pyrimidinyl, pyrido[4,3-d]pyrimidinyl, pyrido[3,4-d]pyrimidinyl, pyrido[2,3-b]pyrimidinyl, pyrido[2,3-b]pyrazinyl, pyrido[3,4-b]pyrazinyl, pyrimido[5,4-d]pyrimidinyl, pyrazino[2,3-b]pyrazinyl, pyrimido[4,5-d]pyrimidinyl, and the like.

The term "heterocyclyl" refers to a saturated or partially unsaturated ring comprising 1 to 4 heteroatoms selected from N, O, and S, besides carbon atoms. Unless otherwise defined, the heterocyclyl may be a simple ring or polycyclic ring. For example, "3- to 14-membered heterocyclyl" refers to aliphatic heterocycle comprising 3 to 14 atoms forming a ring, and 3- to 6-membered aliphatic heterocyclic or the heterocyclyl ring may include a bicyclic ring fused with a benzene ring or other heterocyclyl rings. In an embodiment, 0, 1, 2, 3, or 4 atoms of each ring of the heterocyclyl group may be substituted with a substituent. For example, heterocyclyl may be, but is not limited to, azetidinyl, pyrrolidinyl, tetrahydrofuranyl, tetrahydro-thienyl, pyrazolidinyl, imidazolidinyl, oxazolidinyl, isoxazolidinyl, thiazolidinyl, dioxolyl, piperidinyl, tetrahydropyranyl, tetrahydrothiopyranyl, piperazinyl, morpholinyl, thiomorpholinyl, 1,1-dioxothiomorpholin-4-yl, azepanyl, diazepanyl, homopiperazinyl, oxazepanyl, indolyl, isoindolyl, dihydroindolyl, dioxoisoindolinyl, dihydrofuryl, dihydroimidazolinyl, dihydrooxazolyl, dihydrobenzodioxynyl, tetrahydropyridinyl, dihydropyranyl, dihydrobenzofuranyl, benzodioxolyl, benzodioxanyl, and the like.

The term "nucleobase" is one of the components of a nucleotide, a unit of RNA or DNA nucleic acid, and is a biological compound comprising nitrogen. Specifically, the main nucleobases found in RNA are adenine, guanine, cytosine, and uracil. The nucleobases may be either naturally occurring forms or modified forms. In certain embodiments, the nucleobases may include any atoms or groups of atoms capable of forming hydrogen bonds with nucleobases of another nucleic acid. For instance, the naturally occurring forms of nucleobases include adenine (A) and guanine (G) as purine nucleobases, and thymine (T), cytosine (C), and uracil(U) as pyrimidine nucleobases. In addition, various modified forms of nucleobases and their mimetics are known to those skilled in the art.

The term "amino acid" is a compound or a structural unit of proteins that contains an amino group (-NH₂) and a carboxyl group (-COOH), and can be represented by the general formula, NH₂CHRₙCOOH.

The term "substitution" refers to the replacement of an atom or a functional group in a molecular structure with another atom or a functional group (i.e., a substituent), so that the specified atoms becoming a chemically stable compound by this substitution without exceeding the valence. For example, "Group A is substituted by a substituent B" may mean that an atom bound to another atom such as carbon comprising the skeleton of Group A is substituted with a substituent B, and Group A and substituent B form covalent bonds.

The term "substituent" may refer to another group bound to the parent scaffold group and the substituent may be one or more. If there are multiple substituents, each substituent may be identical or different from each other. In the case that both the parent scaffold group and the substituent are hydrocarbon groups, the number of carbon atoms of the substituent is not included in the number of carbon atoms of the parent scaffold group. For example, a butyl group (-C₄H₉) with a methoxy group (-OCH₃) as a substituent is classified as a C1 alkoxy group and a C4 alkyl group.

The term "isomers" refers to multiple stereoisomers. In embodiments of the present disclosure, various stereoisomers may be generated during the production process. Unless specific stereoisomers are indicated, all stereoisomers that can be formed during the reaction may be included.

The term "active substances" means biologically active substances that, when administered to a subject, exert therapeutic, diagnostic, and/or prophylactic effects, and/or induce desired biological and/or pharmacological effects. These active substances include, but are not limited to, cytotoxins, radioactive ions, chemical therapeutics, small-molecule drugs, proteins, nucleic acids, and the like. The active substances, when delivered to cells or organs, may show desirable changes in cells, organs, or other body tissues or systems and may be useful for the treatment of one or more diseases, disorders, or pathologies. Specific examples of the active substances may include siRNA, shRNA, rRNA, tRNA, mRNA, miRNA, saRNA, circRNA, DNA, cDNA, plasmid, DNAzyme, ribozyme, PNA, aptamer, antisense oligonucleotide, CRISPR, protein, carbohydrate, or drug, and the like.

In an embodiment of the present invention, the following abbreviations are used throughout: "Ac" refers to acetyl, "AcO" or "OAc" refers to acetoxy, "ACN" refers to acetonitrile, "aq" refers to aqueous, "BOC", "Boc" or "boc" refers to N-tert-butoxycarbonyl, "Bn" refers to benzyl, "Bu" refers to butyl, "nBu" refers to normal-butyl, "tBu" refers to tert-butyl, "Cbz" refers to benzyloxycarbonyl, "DCC" refers to N,N'-dicyclohexyl carbodiimide, "DCM" refers to methylene chloride (CH₂Cl₂), "DEA" refers to diethyl amine, "DIPEA" refers to N,N-diisopropyl ethylamine, "DMF" refers to N,N-dimethyl formamide, "DMSO" refers to dimethylsulfoxide, "EDC" refers to 1-ethyl-3-(3-dimethylaminopropyl)carbodiimide, "EDTA" refers to ethylenediaminetetraacetic acid, "Et" refers to ethyl, "EtOAc" refers to ethyl acetate, "EtOH" refers to ethanol, "HATU" refers to (1-[bis(dimethylamino)methylene]-1*H-*1,2,3-triazolo[4,5-*b*]pyridinium 3-oxide hexafluorophosphate, "HOAc" or "AcOH" refers to acetic acid, "IPA" refers to isopropyl alcohol, "LAH" refers to lithium aluminum hydride, "mCPBA" refers to meta-chloroperoxybenzoic acid, "Me" refers to methyl, "MeOH" refers to methanol, "MS" refers to mass spectrometry, "MTBE" refers to methyl tert-butyl ether, "NHS" refers to N-hydroxysuccinimide, "Ph" refers to phenyl, "PyBOP" refers to benzotriazole-1-yloxytripyrrolidinophosphonium hexafluorophosphate, "TFA" refers to trifluoroacetic acid, "THF" refers to tetrahydrofuran, "TLC" refers to thin layer chromatography, "R_{f}" refers to retention factor, "rt" refers to retention time, "r.t." refers to room temperature, "h" refers to hour, "min" refers to minute, "s" refers to second, "equiv." refers to equivalent, and "sat." refers to saturated.

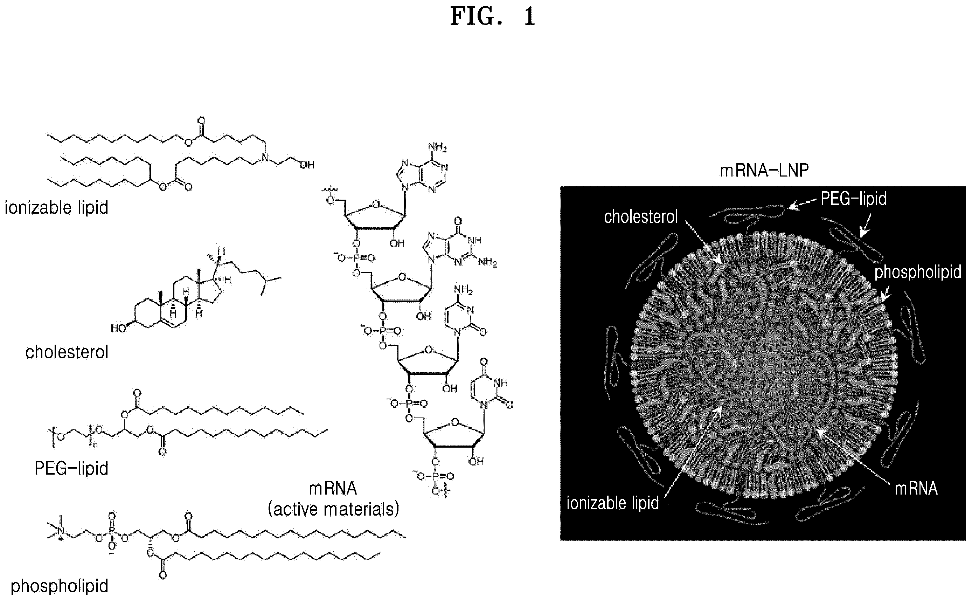

FIG. 1 shows the structure of an mRNA-LNP and examples of the structures of conventional lipid compounds that constitute the mRNA-LNP. Referring to FIG. 1, a lipid nanoparticle (LNP) carrier is generally composed of four types of lipids that surround the active substances, including (A) ionizable lipids, which become cationic in a (slightly) acidic environment, (B) phospholipids, which form a layer at the outermost surface of the LNP, (C) cholesterol, which increases structural rigidity by filling empty spaces inside the LNP, and (D) PEG-lipids ((poly(ethyleneglycol)-lipids), which are mainly distributed on the surface, contributing to LNP stabilization and increasing water solubility. Wherein, the ionizable lipids interact with the active substances in an (slightly) acidic environment, and may serve to stabilize the active substances by surrounding them. The active substances may contain siRNA, shRNA, rRNA, tRNA, mRNA, miRNA, saRNA, circRNA, DNA, cDNA, plasmid, DNAzyme, ribozyme, PNA, aptamer, antisense oligonucleotide, CRISPR, protein, carbohydrate, drug, and the like.

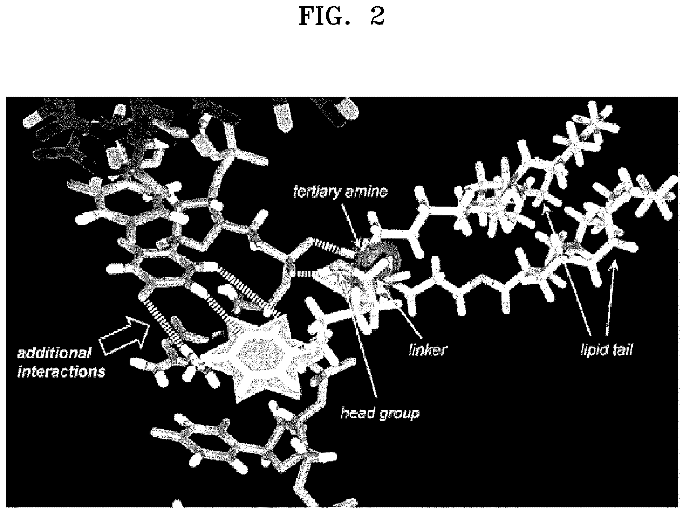

FIG. 2 illustrates the binding profile between mRNA and a lipid compound according to an embodiment of the present disclosure.

Referring to FIG. 2, the conventional ionizable lipid contains a hydrophobic lipid tail, a polar head group (e.g., hydroxy group), and a cationically ionizable tertiary amine in the middle. In contrast, the ionizable lipid of the present disclosure contains an additional interactive group (represented as a yellow star-shaped moiety).

In the ionizable lipid according to an embodiment, the tertiary amine moiety may become cationic (i.e., protonated) in an (slightly) acidic environment, enabling electrostatic interactions with anionic moiety of the active substances. The additional interactive group may interact additionally with another part of the active substances. The additional interactions may involve noncovalent interactions, such as hydrogen bonding and π-π interactions.

In other words, noncovalent interactions, such as electrostatic interactions, hydrogen bonding, and π-π interaction, can form between the ionizable lipid and the active substance. Accordingly, these interactions may enhance the stability of both the ionizable lipid-active substance complex and the lipid nanoparticle (LNP) containing them.

The present disclosure provides a lipid compound of the following Formula 1, an isomer thereof, or a salt thereof: 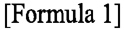 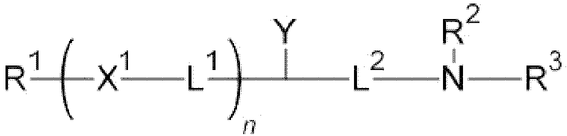 Wherein,
R¹ is hydrogen, hydroxy, C₁₋₆ alkyl, C₂₋₆ alkenyl, C₁₋₆ alkoxy, C₂₋₆ alkenyloxy, - CN, - NO₂, -N(R)₂, -C(=O)R, -C(=O)OR, -OC(=O)R, -C(=O)N(R)₂, -NRC(=O)R, - NRC(=O)N(R)₂, -NRC(=S)N(R)₂, C₃₋₁₄ cycloalkyl, 5- to 10-membered heteroaryl, 3- to 14-membered heterocyclyl, -O-5- to 10-membered heteroaryl, or -O-3- to 14-membered heterocyclyl, wherein C₁₋₆ alkyl, C₂₋₆ alkenyl, C₁₋₆ alkoxy, C₂₋₆ alkenyloxy, C₃₋₁₄ cycloalkyl, 5- to 10-membered heteroaryl, 3- to 14-membered heterocyclyl, -O-5- to 10-membered heteroaryl, or -O-3- to 14-membered heterocyclyl may be each independently unsubstituted or substituted with 1 to 3 halogens or C₁₋₆ alkyl;
X¹ is a simple bond, -CO-C₁₋₆ alkyl, -CO-C₂₋₆ alkenyl, -C(=O)NR-, -NRC(=O)-, - NRC(=O)NR-, -NRC(=S)NR-, C₆₋₁₀ aryl, C₃₋₁₄ cycloalkyl, 5- to 10-membered heteroaryl, 3- to 14-membered heterocyclyl, a nucleobase, an amino acid monomer, or an amino acid oligomer, wherein -CO-C₁₋₆ alkyl, -CO-C₂₋₆ alkenyl, C₆₋₁₀ aryl, C₃₋₁₄ cycloalkyl, 5- to 10-membered heteroaryl, 3- to 14-membered heterocyclyl, a nucleobase, an amino acid monomer, and an amino acid oligomer may be each independently unsubstituted or substituted with 1 to 3 halogens or C₁₋₆ alkyl;
L¹ is C₁₋₁₄ alkylene, C₂₋₁₄ alkenylene, M, R^{a}M, MR^{a}, MRaM¹, or R^{a}MR^{b};
*n* is an integer of 1 to 5, and in case *n* is from 2 to 5, X¹ and L1 are each independently selected;
L² is C₁₋₁₄ alkylene, C₂₋₁₄ alkenylene, M, R^{a}M, MR^{a}, MR^{a}M¹, or R^{a}MR^{b}, and L² is the same as or different from L¹;
Y is hydrogen, -(CH₂)ₘ OH, -(CH₂)ₘ SH, or -(CH₂)ₘ SeH, wherein m is an integer from 0 to 5;
R² and R³ are each independently C₁₋₃₀ alkyl, C₂₋₃₀ alkenyl, or R^{c}MR^{d}, wherein C₁₋₃₀ alkyl and C₂₋₃₀ alkenyl are each independently unsubstituted or substituted with 1 to 3 C₁₋₁₆ alkyl or C₂₋₁₆ alkenyl;
M and M¹ are each independently -NHC(=O)-, -C(=O)NH-, -C(=O)O-, -OC(=O)-, - C(=O)-, -NH-, -S-, -SS-, -O-, -S(O)₂-, -C(=O)S-, -SC(=O)-, -NHC(=O)NH-, - NHC(=O)O-, or -OC(=O)NH-;
R is each independently hydrogen, C₁₋₆ alkyl, C₂₋₆ alkenyl, C₃₋₁₄ cycloalkyl, 5- to 10-membered heteroaryl, or 3- to 14-membered heterocyclyl;
R^{a} and R^{b} are independently -(CH₂)₁-, -C₃₋₂₀ cycloalkyl-(CH₂)₁-, -(CH₂)₁-C₃₋₂₀ cycloalkyl- , -C₆₋₂₀ aryl-(CH₂)₁-, -(CH₂)₁-C₆₋₂₀ aryl-, -NH-(CH₂)₁-, or -(CH₂)-, wherein 1 is an integer from 0 to 10;
R^{c} is C₁₋₁₄ alkylene or C₂₋₁₄ alkenylene;
R^{d} is C₁₋₂₀ alkyl, C₂₋₂₀ alkenyl, or hydrogen, wherein C₁₋₂₀ alkyl and C₂₋₂₀ alkenyl are each independently unsubstituted or substituted as 1 to 3 C₁₋₂₀ alkyls or C₂₋₂₀ alkenyl;

The heteroaryl is an aromatic heterocycle compromising 1 to 6 heteroatoms selected from N, O, and S, and the heterocycle is an aliphatic heterocycle compromising 1 to 6 heteroatoms selected from N, O, and S.

As an embodiment, in the compound of the Formula 1, R¹ may be hydrogen, hydroxy, C₁₋₆ alkyl, C₁₋₆ alkoxy, -CN, -NO₂, -NH₂, -C(=O)-C₁₋₃ alkyl, -C(=O)NH₂, -NHC(=O)-C₁₋₃ alkyl, -NHC(=O)NH₂, or -NHC(=S)NH₂.

As an embodiment, in the compound of the Formula 1, X¹ may be a simple bond, -CO-C₁₋₆ alkyl-, -CO-C₂₋₆ alkenyl-, -C(=O)NH-, -C(=O)N(CH₃)-, -NHC(=O)-, - N(CH₃)C(=O)-, -NHC(=O)NH-, -N(CH₃)C(=O)NH-, -NHC(=S)NH-, or - N(CH₃)C(=S)NH-.

As another embodiment, X¹ may be 5- to 10-membered heteroaryl.

Specifically, the X¹ may be, but is not limited to, pyrrolyl, furanyl, thiophenyl, pyrazolyl, imidazolyl, isoxazolyl, oxazolyl, isothiazolyl, thiazolyl, 1,2,3-triazolyl, 1,3,4-tria zolyl, 1-oxa-2,3-diazolyl, 1-oxa-2,4-diazolyl, 1-oxa-2,5-diazolyl, 1- oxa-3,4-diazolyl, 1-thia-2,3-diazolyl, 1-thia-2,4-diazolyl, 1-thia-2,5- diazolyl, 1-thia-3,4-diazolyl, tetrazolyl, pyridinyl, pyridazinyl, pyrimidinyl, pyrazinyl, indolyl, isoindolyl, benzofuranyl, benzothiophenyl, indazolyl, benz imidazolyl, benzotriazolyl, pyrrolo[2,3-b]pyridinyl, pyrrolo[2,3-c]pyridinyl, pyrrolo[3,2-c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-c]pyridinyl, pyrazolo[3,4-c]pyridinyl, pyrazolo[3,4-b]pyridinyl, purinyl, indolizinyl, imidazo[1,2-a] pyridinyl, imidazo[1,5-a]pyridinyl, pyrazolo[1,5-a]pyridinyl, pyrrolo[1,2-b]pyridazinyl, imidazo[1,2-c]pyrimidinyl, quinolinyl, isoquinolinyl, cinnolinyl, azaquinazolinyl, quinoxalinyl, phthalazinyl, 1,6-naphthyridinyl, 1,7-naphthyridinyl, 1,8-naphthyridinyl, 1,5-naphthyridinyl, 2,6-naphthyridinyl, 2,7-naphthyridinyl, pyrido[3,2- d]pyrimidinyl, pyrido[4,3-d]pyrimidinyl, pyrido[3,4-d]pyrimidinyl, pyrido[2,3-b] pyrimidinyl, pyrido[2,3-b]pyrazinyl, pyrido[3,4-b]pyrazinyl, pyrimido[5,4-d]pyri midinyl, pyrazino[2,3-b]pyrazinyl, or pyrimido[4,5-d]pyrimidinyl.

As another embodiment, X¹ may be nucleobases. The nucleobases may be adenine, guanine, cytosine, or uracil, and may include substituent or modification thereof.

As another embodiment, X¹ may be an amino acid monomer. The amino acid monomer may be alanine, valine, isoleucine, leucine, methionine, phenylalanine, tyrosine, tryptophan, lysine, arginine, histidine, aspartic acid, glutamic acid, serine, threonine, asparagine, glutamine, cysteine, glycine, or proline.

As another embodiment, X¹ may be an amino acid oligomer. The amino acid oligomer may be an oligomer/polymer composed of 2 to 20 amino acid monomers. Wherein, each amino acid monomer may be independently selected from the group consisting of alanine, valine, isoleucine, leucine, methionine, phenylalanine, tyrosine, tryptophan, lysine, arginine, histidine, aspartic acid, glutamic acid, serine, threonine, asparagine, glutamine, cysteine, glycine, or proline.

As an embodiment, in the compound of the Formula 1, L¹ may be C₁₋₁₄ alkylene, C₂₋₁₄ alkenylene, M, R^{a}M, MR^{a}, MR^{a}M¹, or R^{a}MR^{b}.

The M and M₁ may be independently -NHC(=O)-, -C(=O)NH-, -C(=O)O-, -OC(=O)-, -C(=O)-, -NH-, -S-, -SS-, -O-, -S(O)₂-, -C(=O)S-, -SC(=O)-, -NHC(=O)NH-, - NHC(=O)O-, or -OC(=O)NH-yl. In other words, the M and M₁ may be selected identically with or differently from each other at both ends of R^{a}.

The R^{a} and R^{b} are independently -(CH₂)₁-, -C₃₋₂₀ cycloalkyl-(CH₂)₁-, -(CH₂)₁-C₃₋₂₀ cycloalkyl-, -C₄₋₂₀ aryl-(CH₂)₁-, -(CH₂)₁-C₄₋₂₀ aryl-, wherein 1 may be an integer from 0 to 10. In other words, the R^{a} and R^{b} may be selected identically with or differently from each other at both ends of M.

As an embodiment, in the compound of the Formula 1, *n* may be an integer from 1 to 5. In case that *n* is 2 to 5, the X¹ and the L¹ may be independently selected in each case. Accordingly, each X¹ may be selected all identically or differently, each L¹ may be selected all identically or differently.

As an embodiment, in the compound of the Formula 1, L² may be C₁₋₁₄ alkylene, C₂₋₁₄ alkenylene, M, R^{a}M, MR^{a}, MR^{a}M¹ or R^{a}MR^{b}. The L² may be identical to or different from L¹. The M, M¹, R^{a}, and R^{b} comprising the L² are the same as described above.

As an embodiment, in the compound of the Formula 1, Y is hydrogen, -(CH₂)ₘ OH, - (CH₂)ₘ SH, or -(CH₂)ₘ SeH, wherein m may be an integer from 0 to 5.

As an embodiment, in the compound of the Formula 1, R² and R³ may be each independently C₁₋₃₀ alkyl, C₂₋₃₀ alkenyl, or R^{c}MR^{d}. Wherein, the C₁₋₃₀ alkyl and C₂₋₃₀ alkenyl may be each independently substituted with one or more substituents, for instance, C₁₋₁₆ alkyl or C₂₋₁₆ alkenyl.

The M may be independently -NHC(=O)-, -C(=O)NH-, -C(=O)O-, -OC(=O)-, -C(=O)- , -NH-, -S-, -SS-, -O-, -S(O)₂-, -C(=O)S-, -SC(=O)-, -NHC(=O)NH-, -NHC(=O)O-, or -OC(=O)NH-yl.

The R^{c} may be C₁₋₁₄ alkylene or C₂₋₁₄ alkenylene.

The R^{d} is C₁₋₂₀ alkyl, C₂₋₂₀ alkenyl, or hydrogen, wherein the C₁₋₂₀ alkyl and C₂₋₂₀ alkenyl may be each independently unsubstituted or substituted with 1 to 3 C₁₋₂₀ alkyl or C₂₋₂₀ alkenyl.

Salt of the Formula 1 should be less toxic to mammals, including humans, and should not have any negative effect on the biological activity and physicochemical properties of the parent compound. For example, the salt may be an acid addition salt formed by a free acid.

An inorganic acid or an organic acid may be used as the free acid, wherein the inorganic acid may be hydrochloric acid, sulfuric acid, nitric acid, phosphoric acid, perchloric acid, bromic acid, and the like, and the organic acid may be acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid, glutamic acid, and the like.

The acid addition salt may be produced by a conventional method, for example, dissolving the compound of the Formula 1 in an excessive amount of an aqueous solution of the acid and precipitating the salt using a water-miscible organic solvent, for example, methanol, ethanol, acetone, or acetonitrile.

In addition, the salt may be an alkali metal salt (e.g., a sodium salt) or an alkaline earth metal salt (e.g., a potassium salt).

The alkali metal salt or alkaline earth metal salt may be obtained by dissolving the compound of the Formula 1 in an excessive amount of alkali metal hydroxide or alkaline earth metal hydroxide solution, filtering the decomposed compound salt, and evaporating and drying the filtrate.

In another embodiment of the present disclosure, the compound of the Formula 1 may be the compound of the following Formula 2: 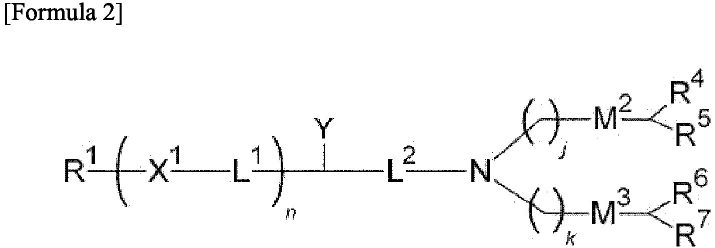 Wherein,
*j* and *k* are independently an integer of 1 to 12;
M² and M³ are each independently -NHC(=O)-, -C(=O)NH-, -C(=O)O-, -OC(=O)-, - C(=O)-, -NH-, -S-, -SS-, -O-, -S(O)₂-, -C(=O)S-, -SC(=O)-, -NHC(=O)NH-, - NHC(=O)O- or -OC(=O)NH-;
R⁴, R⁵, R⁶, and R⁷ are each independently hydrogen, C₁₋₂₄ alkyl, or C₂₋₂₄ alkenyl, wherein the C₁₋₂₄ alkyl and C₂₋₂₄ alkenyl are each independently unsubstituted or substituted with 1 to 3 C₁₋₂₀ alkyl or C₂₋₂₀ alkenyl.

As an embodiment, in the compound of the Formula 2, R¹, n, X¹, L¹, L², and Y are the same as described in the compound of the Formula 1.

As an embodiment, in the compound of the Formula 2, *j* and *k* may be each independently integer of 1 to 12.

As an embodiment, in the compound of the Formula 2, M² and M³ may be each independently -NHC(=O)-, -C(=O)NH-, -C(=O)O-, -OC(=O)-, -C(=O)-, -NH-, -S-, - SS-, -O-, -S(=O)₂-, -C(=O)S-, -SC(=O)-, -NHC(=O)NH-, -NHC(=O)O-, or - OC(=O)NH-.

As an embodiment, in the compound of the Formula 2, R⁴, R⁵, R⁶, and R⁷ are each independently hydrogen, C₁₋₂₄ alkyl, or C₂₋₂₄ alkenyl, wherein the C₁₋₂₄ alkyl and C₂₋₂₄ alkenyl are each independently unsubstituted or may be substituted with 1 to 3 C₁₋₂₀ alkyl or C₂₋₂₀ alkenyl.

The detailed example of compounds of the Formula 1 according to the present disclosure is as below, but it is not limited to:
(1) Heptadecan-9-yl 8-((6-acetamido-2-hydroxyhexyl)(6-oxo-6-(undecyloxy) hexyl)amino)octanoate;
(2) Heptadecan-9-yl 8-((2-hydroxy-6-ureidohexyl)(6-oxo-6-(undecyloxy)hexyl)amino)octanoate;
(3) Heptadecan-9-yl 8-((2-hydroxy-6-(3-methylureido)hexyl)(6-oxo-6-(undecyloxy)hexyl)amino)octanoate;
(4) Heptadecan-9-yl 17-hydroxy-3,11-dioxo-19-(6-oxo-6-(undecyloxy)hexyl)-7-oxa-2,4,10,12,19-pentaazaheptacosan-27-oate;
(5) Heptadecan-9-yl 25-hydroxy-3,11,19-trioxo-27-(6-oxo-6-(undecyloxy)hexyl)-7,15-dioxa-2,4,10,12,18,20,27-heptaazapentatriacontan-35-oate;
(6) Heptadecan-9-yl 8-((2-hydroxy-6-(3-methylthioureido)hexyl)(6-oxo-6-(undecyloxy)hexyl)amino)octanoate;
(7) Heptadecan-9-yl 8-((6-(cyclopentanecarboxamido)-2-hydroxyhexyl)(6-oxo-6-(undecyloxy)hexyl)amino)octanoate;
(8) Heptadecan-9-yl 8-((2-hydroxy-6-((*R*)-pyrrolidine-3-carboxamido)hexyl)(6-oxo-6-(undecyloxy)hexyl)amino)octanoate;
(9) Heptadecan-9-yl 8-((2-hydroxy-6-((*S*)-pyrrolidine-3-carboxamido)hexyl)(6-oxo-6-(undecyloxy)hexyl)amino)octanoate;
(10) Heptadecan-9-yl 8-((2-hydroxy-6-((*R*)-1-methylpyrrolidine-3-carboxamido)hexyl)(6-oxo-6-(undecyloxy)hexyl)amino)octanoate;
(11) Heptadecan-9-yl 8-((2-hydroxy-6-((*S*)-1-methylpyrrolidine-3-carboxamido)hexyl)(6-oxo-6-(undecyloxy)hexyl)amino)octanoate;
(12) Heptadecan-9-yl 8-((6-(cyclohexanecarboxamido)-2-hydroxyhexyl)(6-oxo-6-(undecyloxy)hexyl)amino)octanoate;
(13) Heptadecan-9-yl 8-((2-hydroxy-6-(piperazine-2-carboxamido)hexyl)(6-oxo-6-(undecyloxy)hexyl)amino)octanoate;
(14) Heptadecan-9-yl 8-((6-(1,4-dimethylpiperazine-2-carboxamido)-2-hydroxy hexyl)(6-oxo-6-(undecyloxy)hexyl)amino)octanoate;
(15) Heptadecan-9-yl 8-((6-(2-(1,4-dimethylpiperazine-2-yl)acetamido)-2-hydroxyhexyl)(6-oxo-6-(undecyloxy)hexyl)amino)octanoate;
(16) Heptadecan-9-yl 8-((2-hydroxy-6-(1*H*-pyrrole-2-carboxamido)hexyl)(6-oxo-6-(undecyloxy)hexyl)amino)octanoate;
(17) Heptadecan-9-yl 8-((2-hydroxy-6-(1*H*-pyrrole-3-carboxamido)hexyl)(6-oxo-6-(undecyloxy)hexyl)amino)octanoate;
(18) Heptadecan-9-yl 8-((2-hydroxy-6-(1-methyl-1*H*-pyrrole-2-carboxamido)hexyl) (6-oxo-6-(undecyloxy)hexyl)amino)octanoate;
(19) Heptadecan-9-yl 8-((2-hydroxy-6-(1-methyl-1*H*-pyrrole-3-carboxamido)hexyl) (6-oxo-6-(undecyloxy)hexyl)amino)octanoate;
(20) Heptadecan-9-yl 8-((2-hydroxy-6-(4-nitro-1*H*-pyrrole-2-carboxamido)hexyl)(6-oxo-6-(undecyloxy)hexyl)amino)octanoate;
(21) Heptadecan-9-yl 8-((2-hydroxy-6-(3-hydroxy-1*H*-pyrrole-2-carboxamido)hexyl)(6-oxo-6-(undecyloxy)hexyl)amino)octanoate;
(22) Heptadecan-9-yl 8-((2-hydroxy-6-(4-hydroxy-1*H*-pyrrole-2-carboxamido)hexyl)(6-oxo-6-(undecyloxy)hexyl)amino)octanoate;
(23) Heptadecan-9-yl 8-((2-hydroxy-6-(5-hydroxy-1*H*-pyrrole-2-carboxamido)hexyl)(6-oxo-6-(undecyloxy)hexyl)amino)octanoate;
(24) Heptadecan-9-yl 8-((2-hydroxy-6-(3-hydroxy-1-methyl-1*H*-pyrrole-2-carboxamido)hexyl)(6-oxo-6-(undecyloxy)hexyl)amino)octanoate;
(25) Heptadecan-9-yl 8-((2-hydroxy-6-(4-hydroxy-1-methyl-1*H*-pyrrole-2-carboxamido)hexyl)(6-oxo-6-(undecyloxy)hexyl)amino)octanoate;
(26) Heptadecan-9-yl 8-((2-hydroxy-6-(5-hydroxy-1-methyl-1*H*-pyrrole-2-carboxamido)hexyl)(6-oxo-6-(undecyloxy)hexyl)amino)octanoate;
(27) Heptadecan-9-yl 8-((2-hydroxy-6-(4-hydroxy-1*H*-pyrrole-3-carboxamido)hexyl)(6-oxo-6-(undecyloxy)hexyl)amino)octanoate;
(28) Heptadecan-9-yl 8-((2-hydroxy-6-(5-hydroxy-1*H*-pyrrole-3-carboxamido)hexyl)(6-oxo-6-(undecyloxy)hexyl)amino)octanoate;
(29) Heptadecan-9-yl 8-((2-hydroxy-6-(2-hydroxy-1*H*-pyrrole-3-carboxamido)hexyl)(6-oxo-6-(undecyloxy)hexyl)amino)octanoate;
(30) Heptadecan-9-yl 8-((2-hydroxy-6-(4-hydroxy-1-methyl-1*H*-pyrrole-3-carboxamido)hexyl)(6-oxo-6-(undecyloxy)hexyl)amino)octanoate;
(31) Heptadecan-9-yl 8-((2-hydroxy-6-(5-hydroxy-1-methyl-1*H*-pyrrole-3-carboxamido)hexyl)(6-oxo-6-(undecyloxy)hexyl)amino)octanoate;
(32) Heptadecan-9-yl 8-((2-hydroxy-6-(2-hydroxy-1-methyl-1*H*-pyrrole-3-carboxamido)hexyl)(6-oxo-6-(undecyloxy)hexyl)amino)octanoate;
(33) Heptadecan-9-yl 8-((2-hydroxy-6-(1*H*-imidazole-2-carboxamido)hexyl)(6-oxo-6-(undecyloxy)hexyl)amino)octanoate;
(34) Heptadecan-9-yl 8-((2-hydroxy-6-(1*H*-imidazole-4-carboxamido)hexyl)(6-oxo-6-(undecyloxy)hexyl)amino)octanoate;
(35) Heptadecan-9-yl 8-((2-hydroxy-6-(2-hydroxy-1*H*-imidazole-4-carboxamido)hexyl)(6-oxo-6-(undecyloxy)hexyl)amino)octanoate;
(36) Heptadecan-9-yl 8-((2-hydroxy-6-(1-methyl-1*H*-imidazole-2-carboxamido) hexyl)(6-oxo-6-(undecyloxy)hexyl)amino)octanoate;
(37) Heptadecan-9-yl 8-((2-hydroxy-6-(1-methyl-1*H*-imidazole-4-carboxamido) hexyl)(6-oxo-6-(undecyloxy)hexyl)amino)octanoate;
(38) Heptadecan-9-yl 8-((2-hydroxy-6-(1-methyl-1*H*-imidazole-5-carboxamido) hexyl)(6-oxo-6-(undecyloxy)hexyl)amino)octanoate;
(39) Heptadecan-9-yl 8-((2-hydroxy-6-(2-hydroxy-1-methyl-1*H*-imidazole-4-carboxamido)hexyl)(6-oxo-6-(undecyloxy)hexyl)amino)octanoate;
(40) Heptadecan-9-yl 8-((2-hydroxy-6-(2-hydroxy-1-methyl-1*H*-imidazole-5-carboxamido)hexyl)(6-oxo-6-(undecyloxy)hexyl)amino)octanoate;
(41) Heptadecan-9-yl 8-((2-hydroxy-6-(1*H*-pyrazole-4-carboxamido)hexyl)(6-oxo-6-(undecyloxy)hexyl)amino)octanoate;
(42) Heptadecan-9-yl 8-((2-hydroxy-6-(1*H*-pyrazole-5-carboxamido)hexyl)(6-oxo-6-(undecyloxy)hexyl)amino)octanoate;
(43) Heptadecan-9-yl 8-((2-hydroxy-6-(1-methyl-1*H*-pyrazole-4-carboxamido)hexyl)(6-oxo-6-(undecyloxy)hexyl)amino)octanoate;
(44) Heptadecan-9-yl 8-((2-hydroxy-6-(1-methyl-1*H*-pyrazole-5-carboxamido)hexyl)(6-oxo-6-(undecyloxy)hexyl)amino)octanoate;
(45) Heptadecan-9-yl 8-((2-hydroxy-6-(1-methyl-1*H*-pyrazole-3-carboxamido)hexyl)(6-oxo-6-(undecyloxy)hexyl)amino)octanoate;
(46) Heptadecan-9-yl 8-((2-hydroxy-6-(isoxazole-5-carboxamido)hexyl)(6-oxo-6-(undecyloxy)hexyl)amino)octanoate;
(47) Heptadecan-9-yl 8-((2-hydroxy-6-(isoxazole-4-carboxamido)hexyl)(6-oxo-6-(undecyloxy)hexyl)amino)octanoate;
(48) Heptadecan-9-yl 8-((2-hydroxy-6-(oxazole-5-carboxamido)hexyl)(6-oxo-6-(undecyloxy)hexyl)amino)octanoate;
(49) Heptadecan-9-yl 8-((2-hydroxy-6-(oxazole-4-carboxamido)hexyl)(6-oxo-6-(undecyloxy)hexyl)amino)octanoate;
(50) Heptadecan-9-yl 8-((6-(furan-3-carboxamido)-2-hydroxyhexyl)(6-oxo-6-(undecyloxy)hexyl)amino)octanoate;
(51) Heptadecan-9-yl 8-((2-hydroxy-6-(thiophene-3-carboxamido)hexyl)(6-oxo-6-(undecyloxy)hexyl)amino)octanoate;
(52) Heptadecan-9-yl 8-((2-hydroxy-6-(picolinamido)hexyl)(6-oxo-6-(undecyloxy)hexyl)amino)octanoate;
(53) Heptadecan-9-yl 8-((2-hydroxy-6-(nicotinamido)hexyl)(6-oxo-6-(undecyloxy)hexyl)amino)octanoate;
(54) Heptadecan-9-yl 8-((2-hydroxy-6-(isonicotinamido)hexyl)(6-oxo-6-(undecyloxy)hexyl)amino)octanoate;
(55) Heptadecan-9-yl 8-((2-hydroxy-6-(pyridazine-3-carboxamido)hexyl)(6-oxo-6-(undecyloxy)hexyl)amino)octanoate;
(56) Heptadecan-9-yl 8-((2-hydroxy-6-(pyridazine-4-carboxamido)hexyl)(6-oxo-6-(undecyloxy)hexyl)amino)octanoate;
(57) Heptadecan-9-yl 8-((2-hydroxy-6-(pyrazine-2-carboxamido)hexyl)(6-oxo-6-(undecyloxy)hexyl)amino)octanoate;
(58) Heptadecan-9-yl 8-((2-hydroxy-6-(pyrimidine-4-carboxamido)hexyl)(6-oxo-6-(undecyloxy)hexyl)amino)octanoate;
(59) Heptadecan-9-yl 8-((2-hydroxy-6-(pyrimidine-5-carboxamido)hexyl)(6-oxo-6-(undecyloxy)hexyl)amino)octanoate;
(60) Heptadecan-9-yl 8-((2-hydroxy-6-(pyrimidine-2-carboxamido)hexyl)(6-oxo-6-(undecyloxy)hexyl)amino)octanoate;
(61) Heptadecan-9-yl 8-((2-hydroxy-6-(1*H*-indole-2-carboxamido)hexyl)(6-oxo-6-(undecyloxy)hexyl)amino)octanoate;
(62) Heptadecan-9-yl 8-((2-hydroxy-6-(1-methyl-1*H*-indole-2-carboxamido)hexyl)(6-oxo-6-(undecyloxy)hexyl)amino)octanoate;
(63) Heptadecan-9-yl 8-((2-hydroxy-6-(1*H*-indole-3-carboxamido)hexyl)(6-oxo-6-(undecyloxy)hexyl)amino)octanoate;
(64) Heptadecan-9-yl 8-((2-hydroxy-6-(1-methyl-1*H*-indole-3-carboxamido)hexyl)(6-oxo-6-(undecyloxy)hexyl)amino)octanoate;
(65) Heptadecan-9-yl 8-((6-(4-amino-2-oxo-1,2-dihydropyrimidine-1-carboxamido)-2-hydroxyhexyl)(6-oxo-6-(undecyloxy)hexyl)amino)octanoate;
(66) Heptadecan-9-yl 8-((6-(2,4-dioxo-1,2,3,4-tetrahydropyrimidine-1-carboxamido)-2-hydroxyhexyl)(6-oxo-6-(undecyloxy)hexyl)amino)octanoate;
(67) Heptadecan-9-yl 8-((6-(6-amino-9*H*-purine-9-carboxamido)-2-hydroxyhexyl)(6-oxo-6-(undecyloxy)hexyl)amino)octanoate;
(68) Heptadecan-9-yl 8-((6-(2-amino-6-oxo-6,9-dihydro-1*H*-purine-9-carboxamido)-2-hydroxyhexyl)(6-oxo-6-(undecyloxy)hexyl)amino)octanoate;
(69) Heptadecan-9-yl 8-((6-(2-(4-amino-2-oxopyrimidin-1(2*H*)-yl)acetamido)-2-hydroxyhexyl)(6-oxo-6-(undecyloxy)hexyl)amino)octanoate;
(70) Heptadecan-9-yl 8-((6-(2-(2,4-dioxo-3,4-dihydropyrimidin-1(2*H*)-yl)acetamido)-2-hydroxyhexyl)(6-oxo-6-(undecyloxy)hexyl)amino)octanoate;
(71) Heptadecan-9-yl 8-((6-(2-(6-amino-9*H*-purin-9-yl)acetamido)-2-hydroxyhexyl)(6-oxo-6-(undecyloxy)hexyl)amino)octanoate;
(72) Heptadecan-9-yl 8-((6-(2-(2-amino-6-oxo-1,6-dihydro-9*H*-purin-9-yl) acetamido)-2-hydroxyhexyl)(6-oxo-6-(undecyloxy)hexyl)amino)octanoate;
(73) Heptadecan-9-yl 8-((6-((*S*)-2-amino-5-guanidinopentanamido)-2-hydroxyhexyl)(6-oxo-6-(undecyloxy)hexyl)amino)octanoate;
(74) Heptadecan-9-yl 8-((6-((*S*)-2-acetamido-5-guanidinopentanamido)-2-hydroxyhexyl)(6-oxo-6-(undecyloxy)hexyl)amino)octanoate;
(75) Heptadecan-9-yl 8-((6-((*S*)-2-amino-3-(1*H*-imidazol-5-yl)propanamido)-2-hydroxyhexyl)(6-oxo-6-(undecyloxy)hexyl)amino)octanoate;
(76) Heptadecan-9-yl 8-((6-((*S*)-2-acetamido-3-(1*H*-imidazol-5-yl)propanamido)-2-hydroxyhexyl)(6-oxo-6-(undecyloxy)hexyl)amino)octanoate;
(77) Heptadecan-9-yl 8-((6-((*S*)-2-amino-3-(1*H*-indole-3-yl)propanamido)-2-hydroxyhexyl)(6-oxo-6-(undecyloxy)hexyl)amino)octanoate;
(78) Heptadecan-9-yl 8-((6-((*S*)-2-acetamido-3-(1*H*-indole-3-yl)propanamido)-2-hydroxyhexyl)(6-oxo-6-(undecyloxy)hexyl)amino)octanoate;
(79) Heptadecan-9-yl 8-((2-hydroxy-6-((*S*)-pyrrolidine-2-carboxamido)hexyl)(6-oxo-6-(undecyloxy)hexyl)amino)octanoate;
(80) Heptadecan-9-yl 8-((2-hydroxy-6-((*S*)-1-methylpyrrolidine-2-carboxamido) hexyl)(6-oxo-6-(undecyloxy)hexyl)amino)octanoate;
(81) Heptadecan-9-yl (6*S*,9*S*)-1,6-diamino-9-(3-guanidinopropyl)-16-hydroxy-1-imino-7,10-dioxo-18-(6-oxo-6-(undecyloxy)hexyl)-2,8,11,18-tetraazahexacosan-26-oate;
(82) Heptadecan-9-yl 8-((6-((*S*)-2-((*S*)-2-amino-3-(1*H*-imidazol-5-yl)propanamido)-5-guanidinopentanamido)-2-hydroxyhexyl)(6-oxo-6-(undecyloxy)hexyl)amino)octanoate;
(83) Heptadecan-9-yl 8-((6-((*S*)-2-((*S*)-2-amino-3-(1*H*-imidazol-5-yl)propanamido)-3-(1*H*-imidazol-5-yl)propanamido)-2-hydroxyhexyl)(6-oxo-6-(undecyloxy)hexyl)amino)octanoate;
(84) Heptadecan-9-yl (6*R*,9*S*)-9-((1*H*-imidazol-5-yl)methyl)-1,6-diamino-16-hydroxy-1-imino-7,10-dioxo-18-(6-oxo-6-(undecyloxy)hexyl)-2,8,11,18-tetraazahexacosan-26-oate;
(85) Heptadecan-9-yl (6*R*,9*S*,12*S*)-1,6-diamino-9,12-bis(3-guanidinopropyl)-19-hydroxy-1-imino-7,10,13-trioxo-21-(6-oxo-6-(undecyloxy)hexyl)-2,8,11,14,21-pentaazanonacosan-29-oate;
(86) Heptadecan-9-yl (6*S*,9*S*)-1-amino-6-((*R*)-2-amino-3-(1*H*-imidazol-5-yl)propanamido)-9-(3-guanidinopropyl)-16-hydroxy-1-imino-7,10-dioxo-18-(6-oxo-6-(undecyloxy)hexyl)-2,8,11,18-tetraazahexacosan-26-oate;
(87) Heptadecan-9-yl (2*R*,5*S*,8*S*)-5-((1*H*-imidazol-5-yl)methyl)-2-amino-8-(3-guanidinopropyl)-15-hydroxy-1-(1*H*-imidazol-5-yl)-3,6,9-trioxo-17-(6-oxo-6-(undecyloxy)hexyl)-4,7,10,17-tetraazapentacosan-25-oate;
(88) Heptadecan-9-yl (6*R*,9*S*,12*S*)-9-((1*H*-imidazol-5-yl)methyl)-1,6-diamino-12-(3-guanidinopropyl)-19-hydroxy-1-imino-7,10,13-trioxo-21-(6-oxo-6-(undecyloxy)hexyl)-2,8,11,14,21-pentaazanonacosan-29-oate;
(89) Heptadecan-9-yl (2*R*,5*S*,8*S*)-5,8-bis((1*H*-imidazol-5-yl)methyl)-2-amino-15-hydroxy-1-(1*H*-imidazol-5-yl)-3,6,9-trioxo-17-(6-oxo-6-(undecyloxy)hexyl)-4,7,10,17-tetraazapentacosan-25-oate;
(90) Heptadecan-9-yl (6*R*,9*S*,12*S*)-9,12-bis((1*H*-imidazol-5-yl)methyl)-1,6-diamino-19-hydroxy-1-imino-7,10,13-trioxo-21-(6-oxo-6-(undecyloxy)hexyl)-2,8,11,14,21-pentaazanonacosan-29-oate;
(91) Heptadecan-9-yl (6*R*,9*S*,12*S*)-12-((1H-imidazol-5-yl)methyl)-1,6-diamino-9-(3-guanidinopropyl)-19-hydroxy-1-imino-7,10,13-trioxo-21-(6-oxo-6-(undecyloxy)hexyl)-2,8,11,14,21-pentaazanonacosan-29-oate;
(92) Heptadecan-9-yl (6*S*,9*S*)-9-((1*H*-imidazol-5-yl)methyl)-1-amino-6-((*R*)-2-amino-3-(1*H*-imidazol-5-yl)propanamido)-16-hydroxy-1-imino-7,10-dioxo-18-(6-oxo-6-(undecyloxy)hexyl)-2,8,11,18-tetraazahexacosan-26-oate;
(93) Heptadecan-9-yl 1-(4-amino-2-oxopyrimidin-1(2*H*)-yl)-9-(2-(6-amino-9*H*-purin-9-yl)acetyl)-3-(2-aminoethyl)-17-hydroxy-2,5,11-trioxo-19-(6-oxo-6-(undecyloxy)hexyl)-3,6,9,12,19-pentaazaheptacosan-27-oate;
(94) Heptadecan-9-yl 9-(2-(4-amino-2-oxopyrimidin-1(2*H*)-yl)acetyl)-1-(2-amino-6-oxo-1,6-dihydro-9*H*-purin-9-yl)-15-(2-(6-amino-9H-purin-9-yl)acetyl)-3-(2-aminoethyl)-23-hydroxy-2,5,11,17-tetraoxo-25-(6-oxo-6-(undecyloxy)hexyl)-3,6,9,12,15,18,25-heptaazatritriacontan-23-oate;
(95) Heptadecan-9-yl 15-(2-(4-amino-2-oxopyrimidin-1(2*H*)-yl)acetyl)-9-(2-(2-amino-6-oxo-1,6-dihydro-9*H*-purin-9-yl)acetyl)-21-(2-(6-amino-9*H*-purin-9-yl)acetyl)-3-(2-aminoethyl)-1-(2,4-dioxo-3,4-dihydropyrimidin-1(2*H*)-yl)-29-hydroxy-2,5,11,17,23-pentaoxo-31-(6-oxo-6-(undecyloxy)hexyl)-3,6,9,12,15,18,21,24,31-nonaazanonatriacontan-39-oate;
(96) Heptadecan-9-yl 21-(2-(4-amino-2-oxopyrimidin-1(2*H*)-yl)acetyl)-15-(2-(2-amino-6-oxo-1,6-dihydro-9*H*-purin-9-yl)acetyl)-1-(2-(6-amino-9*H*-purin-9-yl)-27-(2-(6-amino-9*H*-purin-9-yl)acetyl)-3-(2-aminoethyl)-9-(2-(2,4-dioxo-3,4-dihydropyrimidin-1(2*H*)-yl)acetyl)-35-hydroxy-2,5,11,17,23,29-hexaoxo-37-(6-oxo-6-(undecyloxy)hexyl)-3,6,9,12,15,18,21,24,27,30,37-undecaazapentatetracontan-45-oate;
(97) Heptadecan-9-yl 8-((6-(4-(4-amino-1-methyl-1*H*-pyrrole-2-carboxamido)-1-methyl-1*H*-pyrrole-2-carboxamido)-2-hydroxyhexyl)(6-oxo-6-(undecyloxy)hexyl)amino)octanoate;
(98) Heptadecan-9-yl 8-((6-(4-(4-(4-amino-1-methyl-1*H*-imidazole-2-carboxamido)-1-methyl-1*H*-pyrrole-2-carboxamido)-1-methyl-1*H*-pyrrole-2-carboxamido)-2-hydroxyhexyl)(6-oxo-6-(undecyloxy)hexyl)amino)octanoate;
(99) Heptadecan-9-yl 8-((2-hydroxy-6-(1-methyl-4-(1-methyl-4-(1-methyl-4-(1-methyl-1*H*-imidazole-2-carboxamido)-1*H*-imidazole-2-carboxamido)-1*H*-pyrrole-2-carboxamido)-1*H*-pyrrole-2-carboxamido)hexyl)(6-oxo-6-(undecyloxy)hexyl)amino)octanoate;
(100) Heptadecan-9-yl 8-((2-hydroxy-4-(3-methylureido)butyl)(6-oxo-6-(undecyloxy)hexyl)amino)octanoate;
(101) Heptadecan-9-yl 8-((2-hydroxy-5-(3-methylureido)pentyl)(6-oxo-6-(undecyloxy)hexyl)amino)octanoate;
(102) Heptadecan-9-yl 8-((2-hydroxy-7-(3-methylureido)heptyl)(6-oxo-6-(undecyloxy)hexyl)amino)octanoate;
(103) Heptadecan-9-yl 8-((2-hydroxy-8-(3-methylureido)octyl)(6-oxo-6-(undecyloxy)hexyl)amino)octanoate;
(104) Heptadecan-9-yl 9-hydroxy-3-oxo-11-(6-oxo-6-(undecyloxy)hexyl)-7-oxa-2,4,11-triazanonadecan-19-oate;
(105) Heptadecan-9-yl 12-hydroxy-3-oxo-14-(6-oxo-6-(undecyloxy)hexyl)-7,10-dioxa-2,4,14-triazadocosan-22-oate;
(106) Heptadecan-9-yl 15-hydroxy-3-oxo-17-(6-oxo-6-(undecyloxy)hexyl)-7,10,13-trioxa-2,4,17-triazapentacosan-25-oate;
(107) Heptadecan-9-yl 8-((2-(hydroxymethyl)-6-(3-methylureido)hexyl)(6-oxo-6-(undecyloxy)hexyl)amino)octanoate;
(108) Heptadecan-9-yl 8-((2-(2-hydroxyethyl)-6-(3-methylureido)hexyl)(6-oxo-6-(undecyloxy)hexyl)amino)octanoate;
(109) Heptadecan-9-yl 8-((2-(3-hydroxypropyl)-6-(3-methylureido)hexyl)(6-oxo-6-(undecyloxy)hexyl)amino)octanoate;
(110) (6Z,9Z,28Z,31Z)-heptatriaconta-6,9,28,31-tetraen-19-yl 4-((2-hydroxy-6-(3-methylureido)hexyl)(methyl)amino)butanoate;
(111) Heptadecan-9-yl 8-((2-hydroxy-6-(piperazine-1-carboxamido)hexyl)(6-oxo-6-(undecyloxy)hexyl)amino)octanoate;
(112) Heptadecan-9-yl 8-((2-hydroxy-6-(4-methylpiperazine-1-carboxamido)hexyl)(6-oxo-6-(undecyloxy)hexyl)amino)octanoate;
(113) Heptadecan-9-yl 8-((2-hydroxy-6-(2-(piperazin-1-yl)acetamido)hexyl)(6-oxo-6-(undecyloxy)hexyl)amino)octanoate;
(114) Heptadecan-9-yl 8-((2-hydroxy-6-(2-(4-methylpiperazin-1-yl)acetamido)hexyl)(6-oxo-6-(undecyloxy)hexyl)amino)octanoate;
(115) Heptadecan-9-yl 8-((6-(furan-2-carboxamido)-2-hydroxyhexyl)(6-oxo-6-(undecyloxy)hexyl)amino)octanoate;
(116) Heptadecan-9-yl 8-((2-hydroxy-6-(thiophene-2-carboxamido)hexyl)(6-oxo-6-(undecyloxy)hexyl)amino)octanoate;
(117) Heptadecan-9-yl 8-((6-(3-methylureido)hexyl)(6-oxo-6-(undecyloxy)hexyl) amino)octanoate;
(118) Heptadecan-9-yl 8-((6-(dodecan-2-yloxy)-6-oxohexyl)(2-hydroxy-6-(3-methylureido)hexyl)amino)octanoate;
(119) Heptadecan-9-yl 8-((2-hydroxy-6-(3-methylureido)hexyl)(6-oxo-6-(tridecan-3-yloxy)hexyl)amino)octanoate;
(120) 5-((8-(Heptadecan-9-yloxy)-8-oxooctyl)(2-hydroxy-6-(3-methylureido) hexyl)amino)pentyl dodecanoate;
(121) (9Z,28Z)-heptatriaconta-9,28-dien-19-yl 4-((2-hydroxy-6-(3-methyl ureido)hexyl)(methyl)amino)butanoate;
(122) Heptadecan-9-yl 8-((2-hydroxy-6-(1,3,5-triazine-2-carboxamido)hexyl)(6-oxo-6-(undecyloxy)hexyl)amino)octanoate;
(123) Heptadecan-9-yl 8-((2-hydroxy-4-(1*H*-pyrrole-3-carboxamido)butyl)(6-oxo-6-(undecyloxy)hexyl)amino)octanoate;
(124) Heptadecan-9-yl 8-((2-hydroxy-5-(1*H*-pyrrole-3-carboxamido)pentyl)(6-oxo-6-(undecyloxy)hexyl)amino)octanoate;
(125) Heptadecan-9-yl 8-((2-hydroxy-7-(1*H*-pyrrole-3-carboxamido)heptyl)(6-oxo-6-(undecyloxy)hexyl)amino)octanoate;
(126) Heptadecan-9-yl 8-((2-hydroxy-8-(1*H*-pyrrole-3-carboxamido)octyl)(6-oxo-6-(undecyloxy)hexyl)amino)octanoate;
(127) Heptadecan-9-yl 8-((3-(2-(1*H*-pyrrole-3-carboxamido)ethoxy)-2-hydroxypropyl)(6-oxo-6-(undecyloxy)hexyl)amino)octanoate;
(128) Heptadecan-9-yl 10-hydroxy-1-oxo-12-(6-oxo-6-(undecyloxy)hexyl)-1-(1*H-*pyrrol-3-yl)-5,8-dioxa-2,12-diazacosan-20-oate;
(129) Heptadecan-9-yl 13-hydroxy-1-oxo-15-(6-oxo-6-(undecyloxy)hexyl)-1-(1*H-*pyrrol-3-yl)-5,8,11-trioxa-2,15-diazatricosan-23-oate;
(130) Heptadecan-9-yl 8-((2-(hydroxymethyl)-6-(1*H*-pyrrole-3-carboxamido)hexyl) (6-oxo-6-(undecyloxy)hexyl)amino)octanoate;
(131) Heptadecan-9-yl 8-((2-(2-hydroxyethyl)-6-(1*H*-pyrrole-3-carboxamido)hexyl)(6-oxo-6-(undecyloxy)hexyl)amino)octanoate;
(132) Heptadecan-9-yl 8-((2-(3-hydroxypropyl)-6-(1*H*-pyrrole-3-carboxamido)hexyl)(6-oxo-6-(undecyloxy)hexyl)amino)octanoate;
(133) Heptadecan-9-yl 8-((6-(1*H*-pyrrole-3-carboxamido)hexyl)(6-oxo-6-(undecyloxy)hexyl)amino)octanoate;
(134) Heptadecan-9-yl 8-((6-(dodecan-2-yloxy)-6-oxohexyl)(2-hydroxy-6-(1*H-*pyrrole-3-carboxamido)hexyl)amino)octanoate;
(135) Heptadecan-9-yl 8-((2-hydroxy-6-(1*H*-pyrrole-3-carboxamido)hexyl)(6-oxo-6-(tridecan-3-yloxy)hexyl) amino)octanoate;
(136) 5-((8-(Heptadecan-9-yloxy)-8-oxooctyl)(2-hydroxy-6-(1*H*-pyrrole-3-carboxamido)hexyl)amino)pentyl dodecanoate;
(137) (9Z,28Z)-heptatriaconta-9,28-dien-19-yl 4-((2-hydroxy-6-(1*H*-pyrrole-3-carboxamido)hexyl)(methyl)amino)butanoate;
(138) (6Z,9Z,28Z,31Z)-heptatriaconta-6,9,28,31-tetraen-19-yl 4-((2-hydroxy-6-(1*H-*pyrrole-3-carboxamido)hexyl)(methyl)amino)butanoate;
(139) Heptadecan-9-yl 8-((6-(heptan-2-yloxy)-6-oxohexyl)(2-hydroxy-6-(1*H*-pyrrole-3-carboxamido)hexyl)amino)octanoate;
(140) Heptadecan-9-yl 8-((2-hydroxy-6-(1*H*-pyrrole-3-carboxamido)hexyl)(6-(octan-2-yloxy)-6-oxohexyl)amino)octanoate;
(141) Heptadecan-9-yl 8-((2-hydroxy-6-(1*H*-pyrrole-3-carboxamido)hexyl)(6-(nonan-2-yloxy)-6-oxohexyl)amino)octanoate;
(142) Heptadecan-9-yl 8-((2-hydroxy-6-(1*H*-pyrrole-3-carboxamido)hexyl)(6-oxo-6-(tridecan-2-yloxy)hexyl)amino)octanoate;
(143) Heptadecan-9-yl 8-((2-hydroxy-6-(1*H*-pyrrole-3-carboxamido)hexyl)(6-oxo-6-(tetradecan-2-yloxy)hexyl)amino)octanoate;
(144) Heptadecan-9-yl 8-((2-hydroxy-6-(1*H*-pyrrole-3-carboxamido)hexyl)(6-(octan-3-yloxy)-6-oxohexyl)amino)octanoate;
(145) Heptadecan-9-yl 8-((2-hydroxy-6-(1*H*-pyrrole-3-carboxamido)hexyl)(6-oxo-6-(undecan-3-yloxy)hexyl)amino)octanoate;
(146) Heptadecan-9-yl 8-((2-hydroxy-6-(1*H*-pyrrole-3-carboxamido)hexyl)(6-(nonan-4-yloxy)-6-oxohexyl)amino)octanoate;
(147) Heptadecan-9-yl 8-((2-hydroxy-6-(1*H*-pyrrole-3-carboxamido)hexyl)(6-oxo-6-(undecan-4-yloxy)hexyl)amino)octanoate;
(148) Heptadecan-9-yl 8-((6-(decan-5-yloxy)-6-oxohexyl)(2-hydroxy-6-(1*H*-pyrrole-3-carboxamido)hexyl)amino)octanoate;
(149) Heptadecan-9-yl 8-((2-hydroxy-6-(1*H*-pyrrole-3-carboxamido)hexyl)(6-oxo-6-(undecan-5-yloxy)hexyl)amino)octanoate;
(150) Heptadecan-9-yl 8-((2-hydroxy-6-(1*H*-pyrrole-3-carboxamido)hexyl)(6-oxo-6-(undecan-6-yloxy)hexyl)amino)octanoate;
(151) Heptadecan-9-yl 8-((2-hydroxy-6-(1*H*-pyrrole-3-carboxamido)hexyl)(6-((2-methylpentyl)oxy)-6-oxohexyl)amino)octanoate;
(152) Heptadecan-9-yl 8-((2-hydroxy-6-(1*H*-pyrrole-3-carboxamido)hexyl)(6-((2-methyloctyl)oxy)-6-oxohexyl)amino)octanoate;
(153) Heptadecan-9-yl 8-((2-hydroxy-6-(1*H*-pyrrole-3-carboxamido)hexyl)(6-((2-methylnonyl)oxy)-6-oxohexyl)amino)octanoate;
(154) Heptadecan-9-yl 8-((2-hydroxy-6-(1*H*-pyrrole-3-carboxamido)hexyl)(6-((2-methyldecyl)oxy)-6-oxohexyl)amino)octanoate;
(155) Heptadecan-9-yl 8-((2-hydroxy-6-(1*H*-pyrrole-3-carboxamido)hexyl)(6-((2-methylundecyl)oxy)-6-oxohexyl)amino)octanoate;
(156) Heptadecan-9-yl 8-((2-hydroxy-6-(1*H*-pyrrole-3-carboxamido)hexyl)(6-((2-methyldodecyl)oxy)-6-oxohexyl)amino)octanoate;
(157) Heptadecan-9-yl 8-((2-hydroxy-6-(1*H*-pyrrole-3-carboxamido)hexyl)(6-((2-methyltridecyl)oxy)-6-oxohexyl)amino)octanoate;
(158) Heptadecan-9-yl 8-((2-hydroxy-6-(1*H*-pyrrole-3-carboxamido)hexyl)(6-((2-methyltetradecyl)oxy)-6-oxohexyl)amino)octanoate;
(159) Heptadecan-9-yl 8-((6-((2-ethylhexyl)oxy)-6-oxohexyl)(2-hydroxy-6-(1*H-*pyrrole-3-carboxamido)hexyl)amino)octanoate;
(160) Heptadecan-9-yl 8-((6-((2-butyloctyl)oxy)-6-oxohexyl)(2-hydroxy-6-(1*H-*pyrrole-3-carboxamido)hexyl)amino)octanoate;
(161) Heptadecan-9-yl 8-((6-((2-hexyloctyl)oxy)-6-oxohexyl)(2-hydroxy-6-(1*H-*pyrrole-3-carboxamido)hexyl)amino)octanoate;
(162) Heptadecan-9-yl 8-((2-hydroxy-6-(1*H*-pyrrole-3-carboxamido)hexyl)(6-((2-octyldodecyl)oxy)-6-oxohexyl)amino)octanoate;
(163) Heptadecan-9-yl 8-((2-hydroxy-6-(1*H*-pyrrole-3-carboxamido)hexyl)(6-((6-methylnonyl)oxy)-6-oxohexyl)amino)octanoate;
(164) Heptadecan-9-yl 8-((2-hydroxy-6-(1*H*-pyrrole-3-carboxamido)hexyl)(6-((6-methyloctyl)oxy)-6-oxohexyl)amino)octanoate;
(165) Heptadecan-9-yl 8-((2-hydroxy-6-(1*H*-pyrrole-3-carboxamido)hexyl)(6-((7-methylnonyl)oxy)-6-oxohexyl)amino)octanoate;
(166) Heptadecan-9-yl 8-((2-hydroxy-6-(1*H*-pyrrole-3-carboxamido)hexyl)(6-((8-methyldecyl)oxy)-6-oxohexyl)amino)octanoate;
(167) Heptadecan-9-yl 8-((2-hydroxy-6-(1*H*-pyrrole-3-carboxamido)hexyl)(6-((7-methyloctyl)oxy)-6-oxohexyl)amino)octanoate;
(168) Heptadecan-9-yl 8-((2-hydroxy-6-(1*H*-pyrrole-3-carboxamido)hexyl)(6-((8-methylnonyl)oxy)-6-oxohexyl)amino)octanoate;
(169) Heptadecan-9-yl 8-((2-hydroxy-6-(1*H*-pyrrole-3-carboxamido)hexyl)(6-((9-methyldecyl)oxy)-6-oxohexyl)amino)octanoate;
(170) Heptadecan-9-yl 8-((2-hydroxy-6-(1*H*-pyrrole-3-carboxamido)hexyl)(6-((10-methylundecyl)oxy)-6-oxohexyl)amino)octanoate;
(171) Heptadecan-9-yl 8-((2-hydroxy-6-(1*H*-pyrrole-3-carboxamido)hexyl)(6-((11-methyldodecyl)oxy)-6-oxohexyl)amino)octanoate;
(172) Heptadecan-9-yl 8-((2-hydroxy-6-(1*H*-pyrrole-3-carboxamido)hexyl)(6-((12-methyltridecyl)oxy)-6-oxohexyl)amino)octanoate;
(173) Heptadecan-9-yl 8-((2-hydroxy-6-(1*H*-pyrrole-3-carboxamido)hexyl)(6-((5-methylheptan-2-yl)oxy)-6-oxohexyl)amino)octanoate;
(174) Heptadecan-9-yl 8-((2-hydroxy-6-(1*H*-pyrrole-3-carboxamido)hexyl)(6-((5-methyloctan-2-yl)oxy)-6-oxohexyl)amino)octanoate;
(175) Heptadecan-9-yl 8-((2-hydroxy-6-(1*H*-pyrrole-3-carboxamido)hexyl)(6-((6-methylheptan-2-yl)oxy)-6-oxohexyl)amino)octanoate;
(176) Heptadecan-9-yl 8-((2-hydroxy-6-(1*H*-pyrrole-3-carboxamido)hexyl)(6-((2-methyloctan-3-yl)oxy)-6-oxohexyl)amino)octanoate;
(177) Heptadecan-9-yl 8-((6-((2,6-dimethylheptan-4-yl)oxy)-6-oxohexyl)(2-hydroxy-6-(1*H*-pyrrole-3-carboxamido)hexyl)amino)octanoate;
(178) Heptadecan-9-yl 8-((6-((7-ethyl-2-methylundecan-4-yl)oxy)-6-oxohexyl)(2-hydroxy-6-(1*H*-pyrrole-3-carboxamido)hexyl)amino)octanoate.

The lipid compound according to the present disclosure may be advantageously used in a lipid nanoparticle composition to deliver active substances to mammalian cells or organs. For example, the produced lipid nanoparticle composition comprising the lipid compounds described herein has excellent safety when administered in the body and has excellent long-term stability at refrigeration and room temperature.

In another aspect, the present invention provides a method for producing the lipid compound of the Formula 1. Specifically, the lipid compound of the Formula 1 may be produced by the method shown in the reaction formula described in the embodiment, but is not limited to being produced by the method. In particular, those skilled in the art may fully understand that the lipid compound of the Formula 1 of the present invention may be produced by various methods using well-known technologies in the art.

In another aspect, the present disclosure provides a lipid nanoparticle composition comprising a lipid compound of the Formula 1, an isomer thereof, or a salt thereof.

Specifically, the composition is a lipid compound of the compound of the Formula 1, an isomer thereof, or a salt thereof; phospholipid; a structural lipid (e.g., cholesterol), a PEGylated lipid (PEG-lipid), and active substances may be included.

In the present disclosure, the "phospholipid" is a lipid capable of comprising phosphate residues and one or more carbon chains, for instance, an unsaturated fatty acid chain. Phospholipids may include one or more unsaturated (e.g., double or triple) bonds. Certain phospholipids may promote fusion to the membrane. For example, cationic phospholipids may interact with one or more negatively charged phospholipids in a membrane (e.g., cell or endocytic membrane). The fusion of phospholipids to the membrane may allow one or more elements of the lipid-containing composition to pass through the membrane, for example, enabling the delivery of one or more elements to the cell.

The lipid nanoparticle composition according to the present disclosure may include one or more phospholipids, for example, one or more (poly)unsaturated lipids. Phospholipids may be assembled to one or more lipid bilayers. Generally, phospholipids may include phospholipid residues and one or more fatty acid residues. Phospholipid residues may be selected from, but are not limited to, a group consisting of phosphatidyl choline, phosphatidyl ethanolamine, phosphatidyl glycerol, phosphatidyl serine, phosphatidic acid, 2-lysophosphatidyl choline, and sphingomyelin. The fatty acid residues may be selected from, but not limited to, a group consisting of, for example, lauric acid, myristic acid, palmitic acid, palmitoleic acid, stearic acid, oleic acid, linoleic acid, alpha-linolenic acid, erucic acid, phytanic acid, arachidic acid, arachidonic acid, eicosapentaenoic acid, behenic acid, docosapentaenoic acid, and docosahexaenoic acid.

Specifically, the phospholipids may be selected from, but are not limited to, a group consisting of 1,2-distearoyl-sn-glycero-3-phosphocholine (DSPC), 1,2-dioleoyl-sn-glycero-3-phosphoethanolamine (DOPE), 1,2-dilinoleoyl-sn-glycero-3-phosphocholine (DLPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-diundecanoyl-sn-glycero-3-phosphocholine (DUPC), 1-palmitoyl-2-oleoyl-sn-glycero-3-phosphocholine (POPC), 1,2-di-O-octadecenyl-sn-glycero-3-phosphocholine (18:0 diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoylphosphatidylglycerol (DPPG), palmitoyloleoylphosphatidylethanolamine (POPE), distearoyl-phosphatidyl-ethanolamine (DSPE), dipalmitoyl phosphatidyl ethanolamine (DPPE), dimyristoylphosphoethanolamine (DMPE), 1-stearoyl-2-oleoyl-phosphatidylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine (LPE), and mixtures thereof.

According to the present disclosure, the lipid nanoparticle composition may include one or more structural lipids. Structural lipids may be selected from, but are not limited to, for example, cholesterol, pecosterol, cytosterol, ergosterol, campesterol, stigmasterol, bracicasterol, tomatidine, ursolic acid, alpha-tocopherol, and a group consisting of mixtures thereof. In some embodiments, structural lipids are cholesterol. In some embodiments, structural lipids include, but are not limited to, cholesterol and corticosteroids (e.g., prednisolone, dexamethasone, prednisone, and hydrocortisone), or a combination thereof.

According to the present disclosure, the lipid nanoparticle composition may include one or more PEGylated lipids. The term "PEGylated lipid" refers to a lipid containing polyethylene glycol (PEG) components as "PEG-lipid". PEGylated lipids are, for example, PEGylated phosphatidylethanolamine, PEGylated phosphatidic acid, PEGylated ceramide (PEG-CER), PEGylated dialkylamine, PEGylated diacylglycerol (PEG-DEG), PEGylated dialkyl glycerol, and mixtures thereof. For example, PEG-lipids may be, but are not limited to, PEG-c-DOMG, PEG-DMG, PEG-DLPE, PEG-DMPE, PEG-DPPC, or PEG-DSPE lipids.

In another aspect, the present disclosure provides a method of delivering active substances (e.g., mRNA) to a cell (e.g., a mammalian cell). The method includes the lipid compound of Formula 1, an isomer thereof, or a salt thereof; a phospholipid; a structural lipid (e.g., cholesterol); a PEG-lipid; and an active substance is administered as an assembled lipid nanoparticle (LNP) formulation to a subject, wherein the administration of LNP formulation is followed by contacting the cells in the subject, and subsequent delivery of the active substances into the cells.

In addition, the present disclosure provides a method to prepare the active substance-LNP formulation that includes a step of mixing, in the organic phase, lipid compounds of the Formula 1 as ionizable lipids, isomers there of, or salts thereof; phospholipids; structural lipids; and PEGylated lipids, at a molar ratio of (20-60):(0-25):(30-60):(0-5); and a step of preparing the aqueous phase in which the active substances are dissolved in the amount so that

the ratio of the number of nitrogen atoms capable of ionizing the lipid compound of the Formula 1, an isomer thereof, or a salt thereof to the number of phosphorus atoms from the anionic phosphodiester group in the active substances (i.e., N/P ratio) is in the range of 1.5-15; and the mass ratio of the lipid compound of the Formula 1, an isomer thereof, or a salt thereof to the active substance is (4-30):1.

Specific examples of the lipid compound of the Formula 1, an isomer thereof, or a salt thereof, a phospholipid, a structural lipid, a PEGylated lipid, and the active substances are as described above.

In addition, the present disclosure provides a pharmaceutical composition, including the above-described lipid nanoparticle composition and a pharmaceutically acceptable carrier.

In addition, the present disclosure provides a method for preventing or treating diseases, including administering the above-described lipid nanoparticle composition to a subject in need of the prevention or treatment of diseases.

The pharmaceutical composition may further include a pharmaceutically acceptable component.

The pharmaceutical composition may be administered by an oral or parenteral route. Wherein, parenteral refers to a broad route of administration, including, for example, intravenous, intraarterial, intraperitoneal, intramuscular, subcutaneous, intranasal, hypoglossal, intrathecal, inhalation, eye, rectal, vaginal, and ventricular administration.

In the case of pharmaceutically preparing the pharmaceutical composition, it is produced by using diluents or excipients such as commonly used fillers, thickeners, binders, wetting agents, disintegrants, and surfactants. Solid pharmaceutical preparations for oral administration include tablets, patients, acid preparations, granules, capsules, and trochies, and such solid preparations are prepared by mixing at least one excipient, such as starch, calcium carbonate, sucrose, lactose, or gelatin, in one or more compounds according to the disclosure. In addition, lubricants such as magnesium stearate talc are also used in addition to simple excipients. Liquid pharmaceutical preparations for oral administration include suspensions, solutions, emulsions, or syrups. In addition to water and liquid paraffin, which are commonly used diluents, various excipients, such as wetting agents, sweeteners, air fresheners, and preservatives, may be included.

Pharmaceutical preparations for parenteral administration include sterilized aqueous solutions, water-insoluble solutions, suspensions, emulsions, freeze-dried agents, suppositories, and the like. As the non-aqueous solvent and the suspensions, vegetable oils such as propylene glycol, polyethylene glycol, olive oil, injectable esters such as ethylolate, and the like may be used. As the suppository base, witepsol, macrogol, tween 61, cacaoji, laurinji, glycerol, gelatin, and the like may be used.

The pharmaceutical composition or the lipid nanoparticle composition according to the present disclosure may be administered in a pharmaceutically effective amount. In the present disclosure, "pharmacologically effective amount" refers to an amount sufficient to treat a disease at a reasonable benefit/risk ratio applicable to medical treatment, and the effective dose level may be determined according to the patient's disease type, severity, drug activity, drug sensitivity, administration time, administration route and discharge rate, treatment period, factors including concurrent drugs, and other factors well known in the medical field. The composition of the present disclosure may be administered as an individual therapeutic agent or in combination with another therapeutic agent, may be administered sequentially or simultaneously with a conventional therapeutic agent, and may be administered once or multiple times. Considering all of the above factors, it is important to administer an amount capable of obtaining the maximum effect in a minimum amount without side effects, which may be easily determined by those skilled in the art.

Hereinafter, embodiments and experimental examples of the present disclosure will be described in detail. However, the embodiments and experimental examples are for illustratively describing the present disclosure, and the scope of the present invention is not limited thereto.

### [Example]

### Example 1. Production of heptadecan-9-yl 8-((6-acetamido-2-hydroxyhexyl)(6-oxo-6-(undecyloxy)hexyl)amino)octanoate (compound 1)

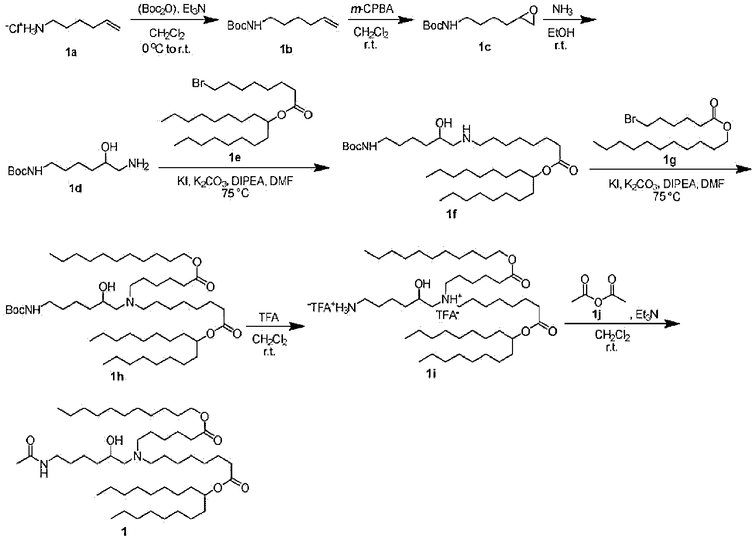

### Step 1: Synthesis of tert-butylhex-5-en-1-ylcarbamate (compound 1b)

1-amino-5-hexen hydrochloride (compound 1a, CAS# 848650-01-1, 4.87 g, 35.9 mmol) and triethylamine (42 mL, 301 mmol) were dissolved in anhydrous CH₂Cl₂ (65 ml) and Boc₂O (20.0 g, 91.5 mmol) was added in an ice bath at 0 °C. The reaction mixture was stirred at room temperature for 14 h, and the reaction solvent was concentrated under reduced pressure. The residue was purified using silica gel chromatography (CHCl₃ 100%) to give *tert-*butyl hex-5-en-1-ylcarbamate (compound 1b, 2.07 g, 29%) as a colerless liquid.

¹H NMR (400 MHz, DMSO-*d*₆): δ 6.77 (t, *J* = 5.7 Hz, 1H), 5.78 (ddt, *J* = 16.9, 10.2, 6.7 Hz, 1H), 5.09-4.83 (m, 2H), 2.90 (q, *J* = 6.4 Hz, 2H), 2.00 (q, *J* = 6.8 Hz, 2H), 1.37 (m, 13H).

### Step 2: Synthesis of tert-butyl (4-(oxiran-2-yl)butyl)carbamate (compound 1c)

*Tert-*butyl hex-5-en-1-ylcarbamate (compound 1b, 2.03 g, 10.2 mmol) was dissolved in anhydrous CH₂Cl₂ (15 mL) in a 250 mL flask. Separately, a solution of mCPBA (77%, 9.16 g, 40.9 mmol) was prepared in anhydrous CH₂Cl₂ (65 mL) and slowly added to the flask with stirring in an ice bath at 0 °C. The reaction mixture was then stirred at room temperature for 19 h. After completion, 10% aqueous sodium sulfite (50 mL) and 10% aqueous sodium bycarbonate (50 mL) were added and stirred vigorously for 30 minutes. The mixture was transferred to a separating funnel, and the organic layer was washed with brine and dried over anhydrous sodium sulfate. The mixture was concentrated and purified using silica gel chromatography (hexane/EtOAc, 3:1) to give *tert-*butyl (4-(oxiran-2-yl)butyl)carbamate (compound 1c, 1.65 g, 75%) as a colorless liquid. ¹H NMR (600 MHz, CDCl₃): δ 4.53 (s, 1H), 3.13 (q, *J* = 6.6 Hz, 2H), 2.96-2.82 (m, 1H), 2.74 (dd, *J* = 5.0, 3.9 Hz, 1H), 2.46 (dd, *J* = 5.0, 2.7 Hz, 1H), 1.65-1.44 (m, 15H); HRMS (ESI): m/z [M+Na]⁺ calcd for C₁₁H₂₁NO₃Na⁺ 238.1419; found 238.1413.

### Step 3: Synthesis of tert-butyl (6-amino-5-hydroxyhexyl)carbamate (compound 1d)

*Tert-*butyl (4-(oxiran-2-yl)butyl)carbamate (compound 1c, 1.61 g, 7.48 mmol) was dissolved in ethanol (100 mL) in a 500 mL flask. Aqueous ammonia (33%, 220 mL) was slowly added to flask with stirring and the reaction mixture was stirred at room temperature for 18 h. After removal of the solvent under reduced pressure, the crude product was dried under vacuum to give *tert-*butyl (6-amino-5-hydroxyhexyl)carbamate (1d, 1.62 g, 93%) as a white solid. ¹H NMR (400 MHz, DMSO-*d*₆): δ 6.75 (t, *J* = 5.7 Hz, 1H), 4.36 (s, 1H), 2.88 (q, *J* = 6.4 Hz, 2H), 2.49-2.29 (m, 2H), 1.37 (m, 15H); HRMS (ESI) m/z: [M+H]⁺ calcd for C₁₁H₂₅N₂O₃⁺ 233.1865, found 233.1854.

### Step 4: Synthesis of heptadecan-9-yl 8-((6-((tert-butoxycarbonyl)amino)-2-hydroxyhexyl) amino)octanoate (compound 1f)

*Tert-*butyl (6-amino-5-hydroxyhexyl)carbamate (compound 1d, 1.53 g, 6.59 mmol) and heptadecan-9-yl 8-bromooctanoate(patent document 1[PCT/US2017/033403 (BENENATO, K.; HOGE, S.; MARTINI, P.; MCFADYEN, I.; PRESNYAK, V.; KUMARASINGHE, E. S.) 2017.05.18]) (compound 1e, 1.20 g, 2.60 mmol) were dissolved in ethanol (25 mL) in a 50 mL flask. The reaction mixture was refluxed at 70 °C for 32 h. The mixture was concentrated and purified using silica gel chromatography (DCM/MeOH/NH₄OH (33%), 140:10:1) to give heptadecan-9-yl 8-((6-((*tert-*butoxycarbonyl)amino)-2-hydroxyhexyl)amino)octanoate (compound 1f, 1.24 g, 78%) as a white solid.

¹H NMR (600 MHz, acetone-*d*₆): δ 4.85 (m, 1H), 3.55 (m, 1H), 3.06 (q, *J* = 6.5 Hz, 2H), 2.65-2.37 (m, 4H), 2.28 (t, *J* = 7.3 Hz, 2H), 1.64-1.22 (m, 53H), 1.00-0.76 (t, *J* = 6.9 Hz, 6H); HRMS (ESI) m/z: [M+H]⁺ calcd for C₃₆H₇₃N₂O₃⁺ 613.5519, found 613.5507.

### Step 5: Synthesis of heptadecan-9-yl 8-((6-((tert-butoxycarbonyl)amino)-2-hydroxyhexyl)(6-oxo-6-(undecyloxy)hexyl)amino)octanoate (compound 1h)

Heptadecan-9-yl 8-((6-((*tert*-butoxycarbonyl) amino)-2- hydroxyhexyl) amino)octanoate (compound 1f, 651 mg, 1.06 mmol) and undecyl 6-bromohexanoate (patent document 2[PCT/US2018/022717 (BENENATO, K. E.; KUMARASINGHE, E. S.; CORNEBISE, M.) 2018.03.15]) (compound 1g, 552 mg, 1.58 mmol) were dissolve in anhydride DMF (3.0 mL) in the an oven dried flask, and DIPEA (0.2 mL, 1.7 mmol), K₂CO₃ (586 mg, 4.24 mmol), and KI (199 mg, 1.20 mmol) were added. The reaction mixture was stirred at 75 °C for 18 h and concentrated. The mixture was diluted with CH₂Cl₂, washed with brine, and dried over sodium sulfate. The mixture was concentrated and purified using silica gel chromatography (CH₂Cl₂/CH₃OH/NH₄OH (33%), 200:10:1) to give heptadecan-9-yl 8-((6-((*tert*-butoxycarbonyl)amino)-2-hydroxyhexyl)(6-oxo-6-(undecyloxy)hexyl)amino)octanoate (compound 1h, 539 mg, 58%) as a white solid.

¹H NMR (400 MHz, CDCl₃): δ 4.92-4.80 (m, 1H), 4.54 (br, 1H), 4.05 (t, *J* = 6.8 Hz, 2H), 3.53 (br, 1H), 3.12 (t, *J* = 5.76 Hz, 2H), 2.71-2.07 (m, 10H), 1.74-1.06 (m, 79H), 0.88 (td, *J* = 6.9, 1.6 Hz, 9H).

### Step 6: Synthesis of heptadecan-9-yl 8-((6-amino-2-hydroxyhexyl)(6-oxo-6-(undecyloxy)hexyl)amino)octanoate trifluoroacetate salt (compound 1i)

Heptadecan-9-yl 8-((6-((*tert*-butoxycarbonyl)amino)-2-hydroxyhexyl)(6-oxo-6-(undecyloxy)hexyl)amino)octanoate (compound 1h, 170 mg, 0.192 mmol) was dissolved in anhydrous CH₂Cl₂ (0.4 mL) in an oven dried flask, and TFA (1.6 mL) was added at 0 °C. The reaction mixture was stirred at room temperature for 40 min, and the solvent was removed under vacuum to give heptadecan-9-yl 8-((6-amino-2-hydroxyhexyl)(6-oxo-6-(undecyloxy)hexyl)amino)octanoate trifluoroacetate salt (compound 1i, 198 mg, 58%) as a white solid.

¹H NMR (400 MHz, CDCl₃): δ 9.56 (s, 1H), 7.87 (s, 3H), 4.85 (qu, *J* = 6.2 Hz, 1H), 4.04 (t, *J* = 6.8 Hz, 3H), 3.42-2.75 (m, 8H), 2.41-2.18 (m, 4H), 1.89-1.03 (m, 68H), 1.03-0.65 (m, 9H).

### Step 7: Synthesis of heptadecan-9-yl 8-((6-acetamido-2-hydroxyhexyl)(6-oxo-6-(undecyloxy)hexyl)amino)octanoate (compound 1)

Heptadecan-9-yl 8-((6-amino-2-hydroxyhexyl)(6-oxo-6-(undecyloxy)hexyl)amino)octanoate trifluoroacetate salt (compound 1i, 18.9 mg, 0.0187 mmol) was dissolved in anhydrous CH₂Cl₂ (0.5 mL) in an oven dried flask. Et₃N (10 µL) and acetic anhydride (1j, 1.6 µL) were added in order, and the reaction mixture was stirred at room temperature for 20 h. The mixture was concentrated and purified using silica gel chromatography (Hexane/EtOAc/MeOH/NH₄OH (33%), 70:40:10:1) to give heptadecan-9-yl 8-((6-acetamido-2-hydroxyhexyl)(6-oxo-6-(undecyloxy)hexyl)amino)octanoate (compound 1, 11.9 mg, 77%) as colorless liquid.

¹H NMR (400 MHz, acetone-*d*₆): δ 6.96 (s, 1H), 4.87 (qu, *J* = 5.9 Hz, 1H), 4.04 (t, *J* = 6.9 Hz, 2H), 3.57-3.52 (m, 1H), 3.15 (q, *J* = 6.5 Hz, 2H), 2.57-2.24 (m, 10H), 1.65-1.22 (m, 68H), 0.88 (t, *J* = 6.9 Hz, 9H); HRMS (ESI) m/z: [M+H]⁺ calcd for C₅₀H₉₉N₂O₆⁺ 823.7498, found 823.7491.

### Example 2. Production of heptadecan-9-yl 8-((2-hydroxy-6-ureidohexyl)(6-oxo-6-(undecyloxy)hexyl)amino)octanoate (compound 2)

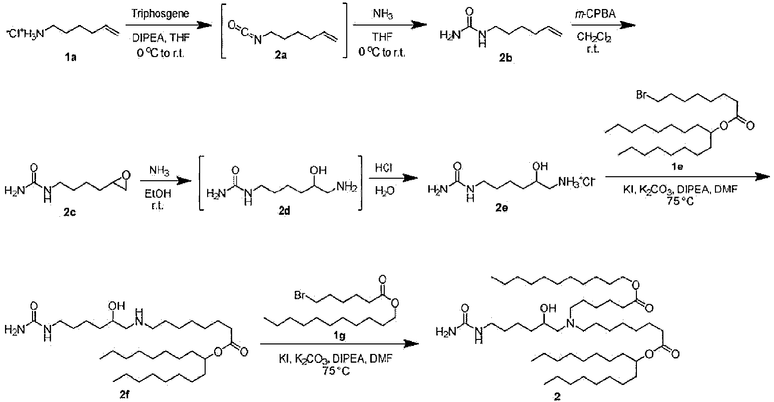

### Step 1: Synthesis of 1-(hex-5-en-1-yl)urea (compound 2b)

To a stirred solution of 1-amino-5-hexin hydrochloride (1a, 400 mg, 2.95 mmol) and DIPEA (1.14 g, 1.54 mL, 8.85 mmol) in anhydrous THF (15 mL), a solution of triphosgene (306 mg, 1.03 mmol) in anhydrous THF (4.9 mL) was slowly added at 0°C in a sealable flask. The reaction mixture was stirred at room temperature for 1 h, and an intermediate product 2a was formed. Ammonia solution (0.4 M, dioxane solution, 22.1 mL, 8.85 mmol) was added to reaction mixture at 0 °C, followed by stirring at room temperature for 20 h. The mixture was concentrated and diluted with CH₂Cl₂. The organic layer was washed with brine, dried over sodium sulfate, and concentrated. The residue was purified using silica gel chromatography (CH₂Cl₂/CH₃OH, 10:1) to give 1-(hex-5-en-1-yl)urea (compound 2b, 302 mg, 72%) as a white solid.

¹H NMR (400 MHz, CDCl₃): δ 5.78 (ddt, *J* = 17.0, 10.2, 6.7 Hz, 1H), 5.00 (dd, *J* = 17.1, 1.7 Hz, 1H), 4.95 (dd, *J* = 10.2, 0.8, Hz, 1H), 4.90 (br, 1H), 4.47 (br, 2H), 3.15 (t, *J* = 6.7 Hz, 2H), 2.06 (dt, *J* = 14.1, 7.0 Hz, 2H), 1.49 (tt, *J* = 6.9, 6.7 Hz, 2H), 1.44 (tt, *J* = 7.1, 7.0 Hz, 2H); ¹³C NMR (150 MHz, CDCl₃): δ 159.0, 138.6, 114.9, 40.7, 33.5, 29.6, 26.2; HRMS (ESI) m/z: [M+H]⁺ calcd for C₇H₁₅N₂O⁺ 143.1179, found 143.1185.

### Step 2: Synthesis of 1-(4-(oxiran-2-yl)butyl)urea (compound 2c)

1-(hex-5-en-1-yl)urea (compound 1c, 302 mg, 2.12 mmol) was dissolved in anhydrous CH₂Cl₂ (14 mL) in a 50 mL flask. Separately, a solution of mCPBA (77%, 1.43 g, 6.37 mmol) was prepared in anhydrous CH₂Cl₂ (28 mL) and slowly added to the flask with stirring at 0°C. The reaction mixture was stirred at room temperature for 48 h, and concentrated. The residue was purified using silica gel chromatography (eluted with 10% AcOH in CH₂Cl₂, removed *m*-chlorobenzoic acid, and then eluted with 10% CH₃OH in CH₂Cl₂) to give 1-(4-(oxiran-2-yl)butyl)urea (compound 2c, 188 mg, 56%) as a white solid.

¹H NMR (600 MHz, CDCl₃): δ 5.57 (s, 1H), 4.49 (s, 2H), 3.11 (qd, *J* = 6.7, 1.4 Hz, 2H), 2.85-2.82 (m, 1H), 2.63 (dd, *J* = 5.1, 4.1, Hz, 1H), 2.39 (dd, *J* = 5.2, 2.6 Hz, 1H), 1.55-1.40 (m, 6H).

### Step 3: Synthesis of 2-hydroxy-6-ureidohexan-1-aminium chloride (compound 2e)

1-(4-(oxiran-2-yl)butyl)urea (compound 2c, 156 mg, 0.986 mmol) was dissolved in EtOH (13.2 mL) and aqueous ammonia (30-33%, 6.53 mL) solution was added. The reaction mixture was stirred at room temperature for 40 h, and an intermediate product 2d was formed. The reaction mixture was concentrated, and diluted with CH₂Cl₂ (20 mL), and then transferred to a separating funnel. Water (20 mL) and aqueous HCl (1.0 M, 1.1 mL) were added to convert the intermediate product 2d into chlorinated salt, which was extracted in the aqueous layer. The aqueous layer was evaporated under reduced pressure and dried under vacuum to give 2-hydroxy-6-ureidohexan-1-aminium chloride (compound 2e, 205 mg, 98%) as a white solid.

¹H NMR (600 MHz, DMSO-*d*₆): δ 7.71 (s, 3H), 5.92 (s, 1H), 5.36 (s, 2H), 5.18 (s, 1H), 3.60 (s, 1H), 2.94 (q, *J* = 4.9 Hz, 2H), 2.87-2.81 (m, 1H), 2.63-2.57 (m, 1H), 1.40-1.30 (m, 5H), 1.29-1.21 (m, 1H).

### Step 4: Synthesis of heptadecan-9-yl 8-((2-hydroxy-6-ureidohexyl)amino)octanoate (compound 2f)

2-hydroxy-6-ureidohexan-1-aminium chloride (compound 2e, 118 mg, 0.557 mmol) and heptadecan-9-yl 8-bromooctanoate (compound 1e, 129 mg, 0.279 mmol) were dissolved in anhydrous DMF (2.8 mL) in a 5 mL flask. DIPEA (108 mg, 0.84 mmol, 0.15 mL), K₂CO₃ (231 mg, 1.67 mmol), and KI (51 mg, 0.31 mmol) were added to reaction mixture. The reaction mixture was stirred at 75 °C for 42 h, and concentrated. The residue was purified using silica gel chromatography (CH₂Cl₂/CH₃OH/aqueous ammonia (33%), 80:20:2) to give heptadecan-9-yl 8-((2-hydroxy-6-ureidohexyl)amino)octanoate (compound 2f, 84 mg, 54%) as a white solid.

¹H NMR (600 MHz, CDCl₃): δ 5.66 (br, 1H), 5.08 (s, 2H), 4.85 (qu, *J* = 6.0 Hz, 1H), 3.74 (s, 1H), 3.14 (q, *J* = 4.9 Hz, 2H), 2.77 (d, *J* = 11.8 Hz, 1H), 2.74-2.65 (m, 2H), 2.57 (dd, *J* = 11.7, 9.7 Hz, 1H), 2.27 (t, *J* = 7.5 Hz, 2H), 1.63-1.21 (m, 44H), 0.87 (t, *J* = 7.0 Hz, 6H); HRMS (ESI) m/z: [M+H]⁺ calcd for C₃₂H₆₆N₃O₄⁺ 556.5048, found 556.5053.

### Step 5: Synthesis of heptadecan-9-yl 8-((2-hydroxy-6-ureidohexyl)(6-oxo-6-(undecyloxy)hexyl)amino)octanoate (compound 2)

Heptadecan-9-yl 8-((2-hydroxy-6-ureidohexyl)amino)octanoate (compound 2f, 48 mg, 0.086 mmol) and undecyl 6-bromohexanoate (compound 1g, 32 mg, 0.090 mmol) were dissolved in anhydrous DMF (0.86 mL) in 4 mL vial. DIPEA (33 mg, 0.26 mmol, 45 uL), K₂CO₃ (36 mg, 0.26 mmol), and KI (16 mg, 0.094 mmol) were added. The reaction mixture was stirred at 75 °C for 18 h, and concentrated. The residue was purified using silica gel chromatography (CH₂Cl₂/CH₃OH/NH₄OH (33%), 15:1:0.1) to give heptadecan-9-yl 8-((2-hydroxy-6-ureidohexyl)(6-oxo-6-(undecyloxy)hexyl)amino)octanoate (compound 2, 44 mg, 62%) as a colorless oil.

¹H NMR (400 MHz, CDCl₃): δ 4.90 (br, 1H), 4.85 (qu, *J* = 6.2 Hz, 1H), 4.51 (br, 2H), 4.04 (t*, J* = 6.8 Hz, 2H), 3.56 (br, 1H), 3.17 (q, *J* = 5.8 Hz, 2H), 2.56-2.22 (m, 9H), 1.69-1.17 (m, 68H), 0.87 (t, *J* = 6.7 Hz, 9H); HRMS (ESI) m/z: [M+H]⁺ calcd for C₄₉H₉₈N ₃O₆⁺ 824.7450, found 824.7454.

### Example 3. Production of heptadecan-9-yl 8-((2-hydroxy-6-(3-methylureido)hexyl)(6-oxo-6-(undecyloxy)hexyl)amino)octanoate (compound 3)

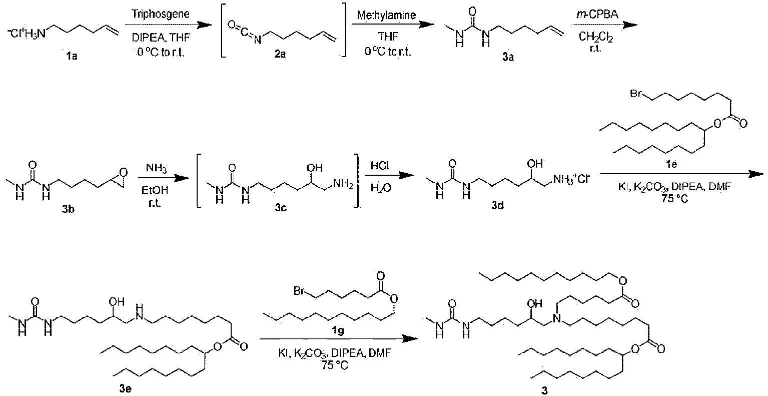

### Step 1: Synthesis of 1-(hex-5-en-1-yl)-3-methylurea (compound 3a)

To a stirred solution of 1-amino-5-hexin hydrochloride (1a, 400 mg, 2.95 mmol) and DIPEA (1.14 g, 1.54 mL, 8.85 mmol) in anhydrous THF (15 mL), a solution of triphosgene (306 mg, 1.03 mmol) in anhydrous THF (4.9 mL) was slowly added at 0 °C in a sealable flask. The reaction mixture was stirred at room temperature for 1 h, and an intermediate product 2a was formed. Methylamine (2.0 M, THF solution, 4.4 mL, 8.85 mmol) was added to the reaction mixture at 0 °C, followed by stirring at room temperature for 20 h. The mixture was concentrated and diluted with CH₂Cl₂. The organic layer was washed with brine, dried over sodium sulfate, and concentrated. The residue was purified using silica gel chromatography (CH₂Cl₂/CH₃OH, 10:1) to give 1-(hex-5-en-1-yl)-3-methylurea (compound 3a, 351 mg, 76%) as a white solid.

¹H NMR (400 MHz, CDCl₃): δ 5.79 (ddt, *J* =17.4, 10.3, 6.7 Hz, 1H), 5.00 (dd, *J* = 17.1, 1.6 Hz, 1H), 4.95 (dd, *J* = 10.2, 0.8, Hz, 1H), 4.34 (Br, s, 2H), 3.17 (t, *J* = 7.0 Hz, 2H), 2.77 (s, 3H), 2.07 (dt, *J* =14.1, 7.0 Hz, 2H), 1.51 (tt, *J* = 8.6, 7.1 Hz, 2H), 1.42 (tt, *J* = 7.9, 6.9 Hz, 2H); ¹³C NMR (150 MHz, CDCl₃): δ 159.0, 138.6, 114.9, 40.7, 33.5, 29.8, 27.4, 26.3; HRMS (ESI) m/z: [M+H]⁺ calcd for C₈H₁₇N₂O⁺ 157.1335, found 157.1340.

### Step 2: Synthesis of 1-methyl-3-(4-(oxiran-2-yl)butyl)urea (compound 3b)

1-(hex-5-en-1-yl)-3-methylurea (compound 3a, 100 mg, 0.640 mmol) was dissolved in anhydrous CH₂Cl₂ (4.3 mL) in an oven dried flask. Separately, a solution of mCPBA (77%, 430 mg, 1.92 mmol) was prepared in anhydrous CH₂Cl₂ (8.5 mL), and slowly added to the flask with stirring at 0 °C. The reaction mixture was stirred at room temperature for 48 h, and concentrated. The residue was purified using silica gel chromatography (eluted with 10% AcOH in CH₂Cl₂, removed m-chlorobenzoic acid, and then eluted with 10% CH₃OH in CH₂Cl₂) to give 1-methyl-3-(4-(oxiran-2-yl)butyl)urea (compound 3b, 34 mg, 31%) as a white solid.

¹H NMR (600 MHz, acetone-*d*₆): δ 5.38 (s, 1H), 5.19 (s, 1H), 3.11 (qd, *J* = 6.9, 1.6 Hz, 2H), 2.84-2.82 (m, 1H), 2.64 (d, *J* = 4.7 Hz, 3H), 2.63 (dd, *J* = 5.2, 1.2, Hz, 1H), 2.39 (dd, *J* = 5.3, 2.6 Hz, 1H), 1.55-1.39 (m, 6H).

### Step 3: Synthesis of 2-hydroxy-6-(3-methylureido)hexan-1-aminium chloride (compound 3d)

1-methyl-3-(4-(oxiran-2-yl)butyl)urea (compound 3b, 41 mg, 0.238 mmol) was dissolved in EtOH (3.2 mL), and aqueous ammonia (30-33%, 1.6 mL) solution was added. The reaction mixture was stirred at room temperature for 40 h, and an intermediate product 3c was formed. The reaction mixture was concentrated, and diluted with CH₂Cl₂ (10 mL), and then transferred to a separating funnel. Water (10 mL) and aqueous HCl (1.0 M, 0.3 mL) were added to convert the intermediate product 3c into chlorinated salt, which was extracted in the aqueous layer. The aqueous layer was evaporated under reduced pressure and dried under vacuum to give 2-hydroxy-6-(3-methylureido)hexan-1-aminium chloride (compound 3d, 53 mg, 99%) as a white solid.

¹H NMR (600 MHz, DMSO-*d*₆): δ 7.80 (s, 3H), 5.90 (s, 1H), 5.71 (s, 2H), 5.17 (s, 1H), 3.61 (s, 1H), 2.96 (t, *J* = 6.1 Hz, 2H), 2.86-2.80 (m, 1H), 2.63-2.56 (m, 1H), 2.52 (s, 3H), 1.42-1.30 (m, 5H), 1.29-1.21 (m, 1H).

### Step 4: Sythesis of heptadecan-9-yl 8-((2-hydroxy-6-(3-methylureido)hexyl)amino)octanoate (compound 3e)

2-hydroxy-6-(3-methylureido)hexan-1-aminium chloride (compound 3d, 38 mg, 0.17 mmol) and heptadecan-9-yl 8-bromooctanoate (compound 1e, 39 mg, 0.084 mmol) were dissolved in anhydrous DMF (1.7 mL) in an oven dried flask. DIPEA (33 mg, 0.25 mmol, 0.044 mL), K₂CO₃ (35 mg, 0.25 mmol), and KI (15 mg, 0.093 mmol) were added to reaction mixture. The reaction mixture was stirred at 75 °C for 42 h, and concentrated. The residue was purified using silica gel chromatography (CH₂Cl₂/CH₃OH/NH₄OH (33%), 80:20:2) to give heptadecan-9-yl 8-((2-hydroxy-6-(3-methylureido)hexyl)amino)octanoate (compound 3e, 19 mg, 40%) as a white solid.

¹H NMR (400 MHz, CDCl₃): δ 5.61 (s, 1H), 5.42 (s, 1H), 4.84 (qu, *J* = 6.2 Hz, 1H), 4.50 (Br, 1H), 3.93 (s, 1H), 3.14 (d, *J* = 4.8 Hz, 2H), 2.92 (d, *J* = 11.7 Hz, 1H), 2.87 (t, *J* = *7.8* Hz, 2H), 2.77 (d, *J* = 11.2 Hz, 1H), 2.72 (d, *J* = 4.44 Hz, 3H), 2.26 (t, *J* = 7.5 Hz, 2H), 1.72 (s, 2H), 1.59 (qu, *J* = 6.8 Hz, 2H), 1.55-1.21 (m, 40H), 0.87 (t, *J*= 6.8 Hz, 6H); HRMS (ESI) m/z: [M+H]⁺ calcd for C₃₃H₆₈N₃O₄⁺, 570.5204, found 570.5207.

### Step 5: Synthesis of heptadecan-9-yl 8-((2-hydroxy-6-(3-methylureido)hexyl)(6-oxo-6-(undecyloxy)hexyl)amino)octanoate (compound 3)

Heptadecan-9-yl 8-((2-hydroxy-6-(3-methylureido)hexyl)amino)octanoate (compound 3e, 25 mg, 0.086 mmol) and undecyl 6-bromohexanoate (compound 1g, 32 mg, 0.090 mmol) were dissolved in anhydrous DMF (0.86 mL) in a 4 mL vial. DIPEA (33 mg, 0.26 mmol, 45 uL), K₂CO₃ (36 mg, 0.26 mmol), and KI (16 mg, 0.094 mmol) were added. The reaction mixture was stirred at 75 °C for 18 h, and concentrated. The residue was purified using silica gel chromatography (CH₂Cl₂/CH₃OH/NH₄OH (33%), 15:1:0.1) to give heptadecan-9-yl 8-((2-hydroxy-6-(3-methylureido)hexyl)(6-oxo-6-(undecyloxy)hexyl)amino)octanoate (compound 3, 44 mg, 62%) as colorless oil.

¹H NMR (400 MHz, CDCl₃): δ 4.85 (qu, *J* = 6.1 Hz, 1H), 4.52 (t, *J* = 5.2 Hz, 1H), 4.42 (q, *J* = 4.3 Hz, 1H), 4.05 (t, *J* = 6.7 Hz, 2H), 3.58 (br, 1H), 3.18 (q, *J* = 6.0 Hz, 2H), 2.76 (d, *J* = 4.8 Hz, 3H), 2.61-2.20 (m, 10H), 1.68-1.11 (m, 68H), 0.87 (t, *J* = 6.5 Hz, 9H); HRMS (ESI) m/z: [M+H]⁺ calcd for C₅₀H₁₀₀N₃O₆⁺ 838.7607, found 838.7609.

### Example 4. Production of heptadecan-9-yl 8-((6-(cyclopentanecarboxamido)-2-hydroxyhexyl)(6-oxo-6-(undecyloxy)hexyl)amino)octanoate(compound 7)

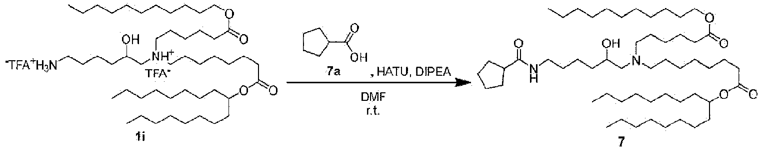

### Step 1: Synthesis of heptadecan-9-yl 8-((6-(cyclopentanecarboxamido)-2-hydroxyhexyl) (6-oxo-6-(undecyloxy)hexyl)amino)octanoate (compound 7)

Heptadecan-9-yl 8-((6-amino-2-hydroxyhexyl)(6-oxo-6-undecyloxy)hexyl)amino)octanoate trifluoroacetate salt (compound 1i, 35.7 mg, 0.0353 mmol), cyclopentanecarboxylic acid (7a, 14.9 mg, 0.130 mmol), and DIPEA (30 uL, 0.23 mmol) were dissolved in anhydrous DMF (2.0 mL) in an oven dried flask. HATU (26.6 mg, 0.0700 mmol) was added at 0 °C, and the reaction mixture was stirred at room temperature for 4 h. The mixture was concentrated and purified using silica gel chromatography (hexane/EtOAc/CH₃OH/NH₄OH (33%), 70:40:10:1) to give heptadecan-9-yl 8-((6-cyclopentanecarboxamido)-2-hydroxyhexyl)(6-oxo-6-(undecyloxy)hexyl)amino)octanoate (compound 7, 21.4 mg, 69%) as colorless liquid.

¹H NMR (600 MHz, acetone-*d*₆): δ 6.89 (s, 1H), 4.87 (qu, *J* = 6.2 Hz, 1H), 4.04 (t, *J* = 6.7 Hz, 2H), 3.55 (br, 1H), 3.17 (q, *J* = 6.7 Hz, 2H), 2.62-2.23 (m, 11H), 1.80-1.15 (m, 76H), 0.88 (t, *J* = 6.9 Hz, 9H).

### Example 5. Production of heptadecan-9-yl 8-((2-hydroxy-6-((R)-pyrrolidine-3-carboxamido)hexyl)(6-oxo-6-(undecyloxy)hexyl)amino)octanoate (compound 8)

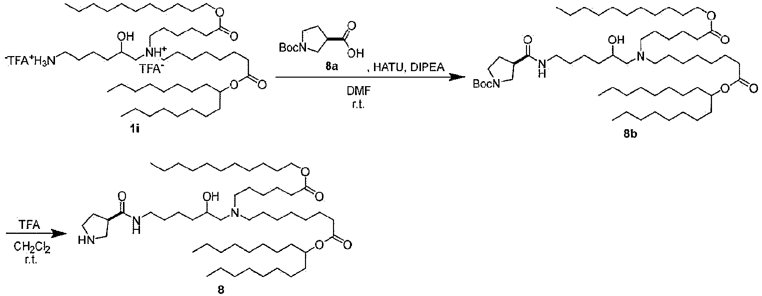

### Step 1: Synthesis of tert-butyl (3R)-3-((6-((8-(heptadecan-9-yloxy)-8-oxooctyl)(6-oxo-6-(ubdecyloxy)hexyl)amino)-5-hydroxyhexyl)carbamoyl)pyrrolidine-1-carboxylate (compound 8b)

Heptadecan-9-yl 8-((6-amino-2-hydroxyhexyl)(6-oxo-6-undecyloxy)hexyl)amino)octanoate trifluoroacetate salt (compound 1i, 34.7 mg, 0.0327 mmol), (*R*)-1-(*tert*-butoxycarbonyl)pyrrolidine-3- carboxylic acid (8a, 14.3 mg, 0.0644 mmol), and DIPEA (50 uL, 0.278 mmol) were dissolved in anhydrous DMF (1.0 mL) in an oven dried flask. HATU (15.5 mg, 0.0399 mmol) was added in an ice bath at 0°C, and the reaction mixture was stirred at room temperature for 2 h. The mixture was diluted with CH₂Cl₂, the organic layer was washed with brine, dried over sodium sulfate, and concentrated. The residue was purified using silica gel chromatography(hexane/EtOAc/CH₃OH/NH₄OH (33%), 130:40:10:1) to give *tert-*butyl (3*R*)-3-((6-((8-(heptadecan-9-yloxy)-8-oxooctyl)(6-oxo-6-(undecyloxy)hexyl)amino)-5-hydroxyhexyl)carbamoyl)pyrrolidine-1-carboxylate (compound 8b, 21.6 mg, 66%) as colorless liquid.

¹H NMR (600 MHz, acetone-*d*₆): δ 7.15 (s, 1H), 4.87 (qu, *J* = 6.2 Hz, 1H), 4.04 (t, *J* = 6.7 Hz, 2H), 3.63-3.11 (m, 8H), 2.95 (dt, *J* = 22.0, 7.9 Hz, 1H), 2.58-2.22 (m, 12H), 1.80-1.15 (m, 77H), 0.88 (t, *J* = 6.9 Hz, 9H); HRMS (ESI) m/z: [M+H]⁺ calcd for C₅₈H₁₁₂N₃O₈⁺ 978.8444, found 978.8461.

### Step 2: Synthesis of heptadecan-9-yl 8-((2-hydroxy-6-((R)-pyrrolidine-3-carboxamido)hexyl)(6-oxo-6-(undecyloxy)hexyl)amino)octanoate (compound 8)

*Tert-*butyl (3*R*)-3-((6-((8-(heptadecan-9-yloxy)-8-oxooctyl)(6-oxo-6-(undecyloxy)hexyl)amino)-5-hydroxyhexyl)carbamoyl)pyrrolidine-1-carboxylate (compound 8b, 21.1 mg, 0.0209 mmol) was dissolved in anhydrous CH₂Cl₂ in a 5 mL flask. TFA (0.8 mL) was added, and stirred at room temperature for 30 min. The reaction solvent was evaporated under reduced pressure, the residue was purified using LH-20 column (CH₃OH 100%) to give heptadecan-9-yl 8-((2-hydroxy-6-((*R*)-pyrrolidine-3-carboxamido)hexyl)(6-oxo-6-(undecyloxy)hexyl)amino)octanoate trifluoroacetate salt (compound 8, 18.5 mg, 89%) as colorless liquid.

¹H NMR (600 MHz, acetone-*d*₆): δ 4.87 (qu, *J* = 6.2 Hz, 1H), 4.38-4.08 (m, 4H), 4.03 (t, *J* = 6.7 Hz, 2H), 3.70-2.70 (m, 10H), 2.54-2.10 (m, 6H), 1.90-1.05 (m, 72H), 0.88 (t, *J* = 6.9 Hz, 9H); HRMS (ESI) m/z: [M+2H]²⁺ calcd for C₅₃H₁₀₅N₃O₆²⁺ 439.8996, found 439.9000.

### Example 6. Production of heptadecan-9-yl 8-((2-hydroxy-6-((S)-pyrrolidine-3-carboxamido)hexyl)(6-oxo-6-(undecyloxy)hexyl)amino)octanoate (compound 9)

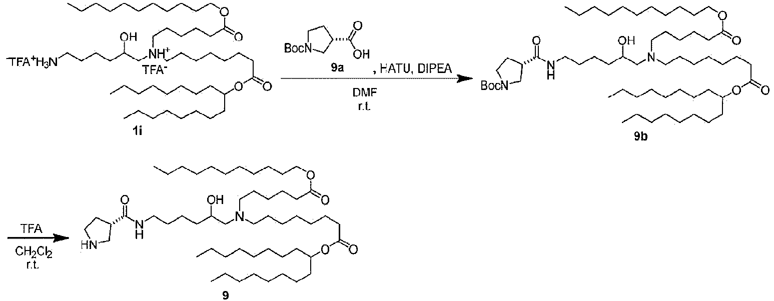

### Step 1: Synthesis of tert-butyl (3S)-3-((6-((8-(heptadecan-9-yloxy)-8-oxooctyl)(6-oxo-6-(ubdecyloxy)hexyl)amino)-5-hydroxyhexyl)carbamoyl)pyrrolidine-1-carboxylate (compound 9b)

Heptadecan-9-yl 8-((6-amino-2-hydroxyhexyl)(6-oxo-6-undecyloxy)hexyl)amino)octanoate trifluoroacetate salt (compound 1i, 36.2 mg, 0.0341 mmol), (*S*)-1-(*tert*-butyloxycarbonyl)pyrrolidine-3-carboxylic acid (9a, 14.7 mg, 0.0662 mmol), and DIPEA (50 uL, 0.278 mmol) were dissolved in anhydrous DMF (1.0 mL) in an oven dried flask. HATU (15.6 mg, 0.0402 mmol) was added at 0°C, and the reaction mixture was stirred at room temperature for 2 h. The mixture was diluted with CH₂Cl₂, the organic layer was washed with brine, dried over sodium sulfate, and concentrated. The residue was purified using silica gel chromatography (hexane/EtOAc/CH₃OH/NH₄OH (33%), 130:40:10:1) to give *tert-*butyl (3*S*)-3-((6-((8-(heptadecan-9-yloxy)-8-oxooctyl)(6-oxo-6-(undecyloxy)hexyl)amino)-5-hydroxyhexyl)carbamoyl)pyrrolidine-1-carboxylate (compound 9b, 23.7 mg, 70%) as a colorless liquid.

¹H NMR (600 MHz, acetone-*d*₆): δ 7.15 (s, 1H), 4.87 (qu, *J* = 6.2 Hz, 1H), 4.04 (t, *J* = 6.7 Hz, 2H), 3.63-3.11 (m, 8H), 2.95 (dt, *J* = 22.0, 7.9 Hz, 1H), 2.58-2.22 (m, 12H), 1.80-1.15 (m, 77H), 0.88 (t, *J* = 6.9 Hz, 9H); HRMS (ESI) m/z: [M+H]⁺ calcd for C₅₈H₁₁₂N₃O₈⁺ 978.8444, found 978.8455.

### Step 2: Synthesis of heptadecan-9-yl 8-((2-hydroxy-6-((S)-pyrrolidine-3-carboxamido)hexyl) (6-oxo-6-(undecyloxy)hexyl)amino)octanoate (compound 8)

*Tert-*butyl (3*S*)-3-((6-((8-(heptadecan-9-yloxy)-8-oxooctyl)(6-oxo-6-(undecyloxy)hexyl)amino)-5-hydroxyhexyl)carbamoyl)pyrrolidine-1-carboxylate (compound 9b, 22.5 mg, 0.0223 mmol) was dissolved in anhydrous CH₂Cl₂ in a 5 mL flask. TFA (0.8 mL) was added, and stirred at room temperature for 30 min. The reaction solvent was evaporated under reduced pressure, the residue was purified using LH-20 column (CH₃OH 100%) to give heptadecan-9-yl 8-((2-hydroxy-6-((*S*)-pyrrolidine-3-carboxamido)hexyl)(6-oxo-6-(undecyloxy)hexyl)amino)octanoate trifluoroacetate salt (compound 9, 19.0 mg, 85%) as a colorless liquid.

¹H NMR (600 MHz, acetone-*d*₆): δ 4.87 (qu, *J* = 6.2 Hz, 1H), 4.38-4.08 (m, 4H), 4.03 (t, *J* = 6.7 Hz, 2H), 3.70-2.70 (m, 10H), 2.54-2.10 (m, 6H), 1.90-1.05 (m, 72H), 0.88 (t, *J* = 6.9 Hz, 9H); HRMS (ESI) m/z: [M+2H]²⁺ calcd for C₅₃H₁₀₅N₃O₆²⁺ 439.8996, found 439.8991.

### Example 7. Production of heptadecan-9-yl 8-((2-hydroxy-6-((R)-1-methylpyrrolidine-3-carboxamido)hexyl)(6-oxo-6-(undecyloxy)hexyl)amino)octanoate (compound 10)

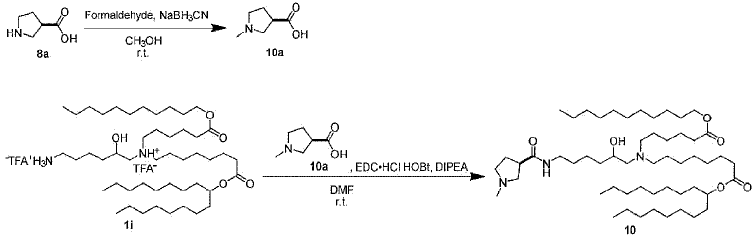

### Step 1: Synthesis of (R)-1-methylpyrrolidine-3-carboxylic acid (compound 10a)

(*R*)-pyrrolidine-3-carboxylic acid (8a, 9.0 mg, 0.078 mmol) was dissolved in CH₃OH (0.78 mL) in a 4 mL vial, and 35% formaldehyde solution (37 uL, 0.47 mmol) was added. After stirring for 5 min, NaBH₃CN (7.4 mg, 0.12 mmol) was added, and the reaction mixture was stirred for an additional 5 min. After the end of the reaction was confirmed by TLC, 1 equivalent of 1 N HCl was added, and the reaction mixture was dried under vacuum. The obtained (*R*)-1-methylpyrrolidine-3-carboxylic acid (compound 10a) was used in the next step without additional purification.

### Step 2: Synthesis of heptadecan-9-yl 8-((2-hydroxy-6-((R)-1-methylpyrrolidine-3-carboxamido)hexyl)(6-oxo-6-(undecyloxy)hexyl)amino)octanoate (compound 10)

Heptadecan-9-yl 8-((6-amino-2-hydroxyhexyl)(6-oxo-6-undecyloxy)hexyl)amino)octanoate trifluoroacetate salt (compound 1i, 26.3 mg, 0.0261 mmol), (*R*)-1-methylpyrrolidine-3-carboxylic acid (10a, products from previous step), and DIPEA (100 uL, 0.573 mmol) were dissolved in DMF (2.6 mL) in an oven dried flask. HOBt (14.1 mg, 0.104 mmol) and EDC·HCl (20.0 mg, 0.104 mmol) were added sequentially at 0 °C inice bath, and the reaction mixture was stirred at room temperature for 16 h. The mixture was diluted with CH₂Cl₂, the organic layer was washed with brine, dried over sodium sulfate, and concentrated. The residue was purified using silica gel chromatography (CH₂Cl₂/CH₃OH/NH₄OH (33%), 70:10:1) to give heptadecan-9-yl 8-((2-hydroxy-6-((*R*)-1-methylpyrrolidine-3-carboxamido)hexyl)(6-oxo-6-(undecyloxy)hexyl)amino)octanoate (compound 10, 6.7 mg, 29%) as a colorless liquid.

¹H NMR (600 MHz, acetone-*d*₆): δ 7.05 (s, 1H), 4.87 (qu, *J* = 6.3 Hz, 1H), 4.04 (t, *J* = 6.7 Hz, 2H), 3.60-3.49 (m, 1H), 3.17 (q, *J* = 6.4 Hz, 1H), 2.83 (qu, *J* = 7.0, 1.3 Hz, 2H), 2.65 (t, *J* = 8.3 Hz, 2H), 2.57-2.27 (m, 12H), 2.26 (s, 3H), 2.00-1.92 (m, 2H), 1.67-1.1% (m, 77H), 0.88 (t, *J* = 6.9 Hz, 9H); HRMS (ESI) m/z: [M+2H]²⁺ calcd for C₅₄H₁₀₇N₃O²⁺ 446.9075, found 446.9084.

### Example 8. Production of heptadecan-9-yl 8-((2-hydroxy-6-((S)-1-methylpyrrolidine-3-carboxamido)hexyl)(6-oxo-6-(undecyloxy)hexyl)amino)octanoate (compound 11)

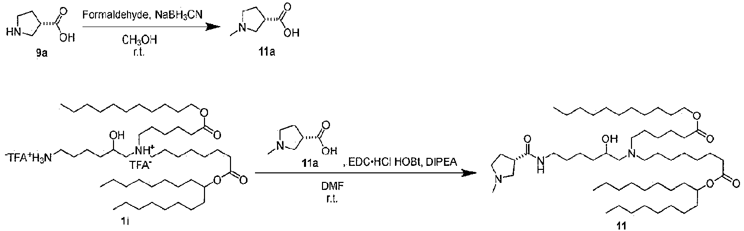

### Step 1: Synthesis of (S)-1-methylpyrrolidine-3-carboxylic acid (compound 11a)

(*S*)-pyrrolidine-3-carboxylic acid (9a, 9.5 mg, 0.083 mmol) was dissolved in CH₃OH (0.83 mL) in a 4 mL vial, and 35% formaldehyde solution (39 uL, 0.50 mmol) was added. After stirring for 5 min, NaBH₃CN (7.8 mg, 0.12 mmol) was added, and the reaction mixture was stirred for an additional 5 min. After the end of the reaction was confirmed by TLC, 1 equivalent of 1 N HCl was added, and the reaction mixture was dried under vacuum. The obtained (S)-1-methylpyrrolidine-3-carboxylic acid (compound 11a) was used in the next step without additional purification.

### Step 2: Synthesis of heptadecan-9-yl 8-((2-hydroxy-6-((S)-1-methylpyrrolidine-3-carboxamido)hexyl)(6-oxo-6-(undecyloxy)hexyl)amino)octanoate (compound 11)

Heptadecan-9-yl 8-((6-amino-2-hydroxyhexyl)(6-oxo-6-undecyloxy)hexyl)octanoate trifluoroacetate salt (compound 1i, 27.8 mg, 0.0275 mmol), (S)-1-methylpyrrolidine-3-carboxylic acid (11a, products from previous step), and DIPEA (100 uL, 0.577 mmol) were dissolved in DMF (2.8 mL) in an oven dried flask. HOBt (14.9 mg, 0.110 mmol) and EDC·HCl (21.1 mg, 0.110 mmol) were added sequentially at 0 °C in ice bath, and the reaction mixture was stirred at room temperature for 16 h. The mixture was diluted with CH₂Cl₂, the organic layer was washed with brine, dried over sodium sulfate, and concentrated. The residue was purified using silica gel chromatography (CH₂Cl₂/CH₃OH/NH₄OH (33%), 70:10:1) to give heptadecan-9-yl 8-((2-hydroxy-6-((*S*)-1-methylpyrrolidine-3-carboxamido)hexyl)(6-oxo-6-(undecyloxy)hexyl)amino)octanoate (compound 11, 8.7 mg, 36%) as a colorless liquid.

¹H NMR (600 MHz, acetone-*d*₆): δ 7.05 (s, 1H), 4.87 (qu, *J* = 6.3 Hz, 1H), 4.04 (t, *J* = 6.7 Hz, 2H), 3.60-3.49 (m, 1H), 3.17 (q, *J* = 6.4 Hz, 1H), 2.83 (qu, *J* = 7.0, 1.3 Hz, 2H), 2.65 (t, *J* = 8.3 Hz, 2H), 2.57-2.27 (m, 12H), 2.26 (s, 3H), 2.00-1.92 (m, 2H), 1.67-1.14 (m, 77H), 0.88 (t, *J* = 6.9 Hz, 9H); HRMS (ESI) m/z: [M+2H]²⁺ calcd for C₅₄H₁₀₇N₃O₆²⁺ 446.9075, found 446.9077.

### Example 9. Production of heptadecan-9-yl 8-((2-hydroxy-6-(1H-pyrrole-2-carboxamido)hexyl)(6-oxo-6-(undecyloxy)hexyl)amino)octanoate (compound 16)

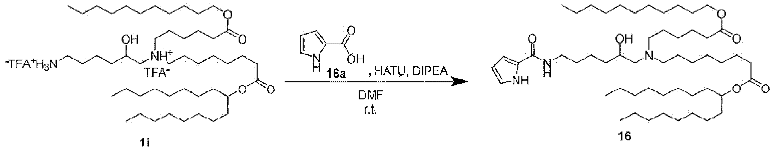

### Step 1: Synthesis of heptadecan-9-yl 8-((2-hydroxy-6-(1H-pyrrole-2-carboxamido)hexyl)(6-oxo-6-(undecyloxy)hexyl)amino)octanoate (compound 16)

1*H*-pyrrole-2-carboxylic acid (compound 16a, 25.2 mg, 0.227 mmol) and DIPEA (200 uL, 1.16 mmol) were dissolved in DMF (2.6 mL) in an oven dried flask. A solution of HATU (57.6 mg, 0.151 mmol) in anhydrous DMF (1.0 mL) was added at 0 °C in an ice bath, and the mixture was stirred at room temperature for 30 min. Separately, heptadecan-9-yl 8-((6-amino-2-hydroxyhexyl)(6-oxo-6-undecyloxy)hexyl)amino)octanoate trifluoroacetate salt (compound 1i, 73.8 mg, 0.0731 mmol) was dissolved in anhydrous DMF (1.0 mL) and added dropwise to the reaction mixture at 0 °C. The mixture was stirred at room temperature for 21 h. The reaction mixture was diluted with CH₂Cl₂, and the organic layer was washed with brine, dried over sodium sulfate, and concentrated under reduced pressure. The residue was purified using silica gel chromatography (CH₂Cl₂/CH₃OH/NH₄OH (33%), 200:10:1) to give heptadecan-9-yl 8-((2-hydroxy-6-(1*H*-pyrrole-2-carboxamido)hexyl)(6-oxo-6-(undecyloxy)hexyl)amino)octanoate (compound 16, 9.76 mg, 15%) as a colorless liquid.

¹H NMR (600 MHz, acetone-*d*₆): δ 10.54 (s, 1H), 7.31 (s, 1H), 6.92 (sext, *J* = 1.3 Hz, 1H), 6.71 (sept, *J* = 1.3 Hz, 1H), 6.11 (dt, *J* = 3.7, 2.6 Hz, 1H), 4.87 (qu, *J* = 6.2 Hz, 1H), 4.04 (t, *J* = 6.7 Hz, 2H), 3.56 (sept, *J* = 3.4 Hz, 1H), 3.45 (br, 1H), 3.33 (qd, *J* = 6.6, 2.7 Hz, 2H), 2.53 (qu, *J* = 5.6 Hz, 2H), 2.44-2.25 (m, 8H), 1.66-1.22 (m, 68H), 0.88 (t, *J* = 7.0 Hz, 9H; ¹³C NMR (150 MHz, acetone-*d*₆): δ 173.7, 173.5, 161.6, 127.9, 121.6, 109.6, 109.5, 74.1, 67.9, 64.6, 62.2, 55.2, 55.0, 39.7, 35.6, 35.01, 34.97, 34.7, 32.7, 32.6, 30.9, 30.34 (2C, overlapped), 30.30, 30.26, 30.25-29.4 (7C, overlapped with solvent residual), 28.0 (2C, overlapped), 27.8, 27.6, 26.7, 26.1, 25.9, 25.7, 24.0, 23.3 (2C, overlapped), 14.4 (2C, overlapped); HRMS (ESI) m/z: [M+H]⁺ calcd for C₅₃H₁₀₀N₃O₆⁺ 874.7607, found 874.7589.

### Example 10. Production of heptadecan-9-yl 8-((2-hydroxy-6-(1H-pyrrole-3-carboxamido)hexyl)(6-oxo-6-(undecyloxy)hexyl)amino)octanoate (compound 17)

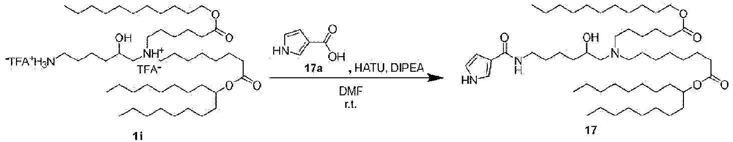

### Step 1: Synthesis of heptadecan-9-yl 8-((2-hydroxy-6-(1H-pyrrole-3-carboxamido)hexyl)(6-oxo-6-(undecyloxy)hexyl)amino)octanoate (compound 17)

1*H*-pyrrole-3-carboxylic acid (compound 17a, 25.4 mg, 0.229 mmol) and DIPEA (200 uL, 1.16 mmol) were dissolved in DMF (1.7 mL) in an oven dried flask. A solution of HATU (59.6 mg, 0.157 mmol) in anhydrous DMF (1.0 mL) was added at 0 °C in an ice bath, and the mixture was stirred at room temperature for 30 min. Separately, heptadecan-9-yl 8-((6-amino-2-hydroxyhexyl)(6-oxo-6-undecyloxy)hexyl)amino)octanoate trifluoroacetate salt (compound 1i, 74.8 mg, 0.0741 mmol) was dissolved in anhydrous DMF (1.0 mL) and added dropwise to the reaction mixture at 0 °C. The mixture was stirred at room temperature for 21 h. The reaction mixture was diluted with CH₂Cl₂, and the organic layer was washed with brine, dried over sodium sulfate, and concentrated under reduced pressure. The residue was purified using silica gel chromatography (CH₂Cl₂/CH₃OH/NH₄OH (33%), 200:10:1) to give heptadecan-9-yl 8-((2-hydroxy-6-(1*H*-pyrrole-3-carboxamido)hexyl)(6-oxo-6-(undecyloxy)hexyl)amino)octanoate (compound 17, 38.9 mg, 60%) as a colorless liquid.

¹H NMR (400 MHz, CDCl₃): δ 8.99 (s, 1H), 7.32 (s, 1H), 6.74 (d, *J* = 2.2 Hz, 1H), 6.41 (s, 1H), 5.98 (s, 1H), 4.86 (qu, *J* = 6.2 Hz, 1H), 4.05 (t, *J* = 6.8 Hz, 2H), 3.56 (br, 1H), 3.41 (sept, *J* = 5.7 Hz, 2H), 2.50 (br, 2H), 2.43-2.19 (m, 8H), 1.69-1.15 (m, 68H), 0.87 (t, *J* = 6.7 Hz, 9H); ¹³C NMR (150 MHz, acetone-*d*₆): δ 173.6, 173.5, 164.9, 121.7, 121.1, 119.1, 107.9, 74.1, 67.9, 64.6, 62.2, 55.2, 55.0, 39.6, 35.6, 35.01, 34.96, 34.7, 32.66, 32.61, 31.1, 30.35, 30.34, 30.30, 30.25, 30.24-29.4 (7C, overlapped with solvent residual), 28.01, 27.98, 27.7, 27.6, 26.7, 26.1, 25.9, 25.7, 24.0, 23.3 (2C, overlapped), 14.4 (2C, overlapped); HRMS (ESI) m/z: [M+H]⁺ calcd for C₅₃H₁₀₀N₃O₆⁺ 874.7607, found 874.7589.

### Example 11. Production of heptadecan-9-yl 8-((2-hydroxy-6-(1-methyl-1H-pyrrole-2-carboxamido)hexyl)(6-oxo-6-(undecyloxy)hexyl)amino)octanoate (compound 18)

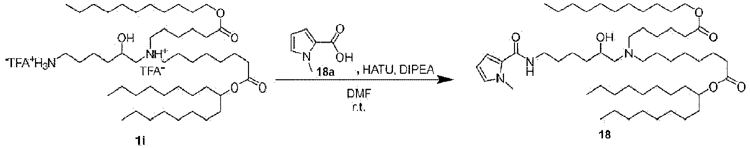

### Step 1: Synthesis of heptadecan-9-yl 8-((2-hydroxy-6-(1-methyl-1H-pyrrole-2-carboxamido)hexyl)(6-oxo-6-(undecyloxy)hexyl)amino)octanoate (compound 18)

1-Methyl-1*H*-pyrrole-2-carboxylic acid (compound 18a, 28.6 mg, 0.229 mmol) and DIPEA (0.25 mL, 1.47 mmol) were dissolved in DMF (1.7 mL) in an oven dried flask.A solution of HATU (56.0 mg, 0.147 mmol) in anhydrous DMF (1.0 mL) was added at 0 °C in an ice bath, and the reaction mixture was stirred at room temperature for 30 min. Separately, heptadecan-9-yl 8-((6-amino-2-hydroxyhexyl)(6-oxo-6-undecyloxy)hexyl)amino)octanoate trifluoroacetate salt (compound 1i, 74.1 mg, 0.0734 mmol) was dissolved in anhydrous DMF (1.0 mL) and added dropwise to the reaction mixture at 0 °C. The mixture was stirred at room temperature for 16 h. The reaction mixture was diluted with CH₂Cl₂, and the organic layer was washed with brine, dried over sodium sulfate, and concentrated under reduced pressure. The residue was purified using silica gel chromatography (CH₂Cl₂/CH₃OH/NH₄OH (33%), 200:10:1) to give heptadecan-9-yl 8-((2-hydroxy-6-(1-methyl-1*H*-pyrrole-2-carboxamido)hexyl)(6-oxo-6-(undecyloxy)hexyl)amino)octanoate (compound 18, 36.3 mg, 56%) as a colorless liquid.

¹H NMR (600 MHz, acetone-*d*₆): δ 7.23 (s, 1H), 6.78 (t, *J* = 2.1 Hz, 1H), 6.68 (dd, *J* = 3.9, 1.7 Hz, 1H), 5.98 (dd, *J* = 3.9, 2.6 Hz, 1H), 4.87 (qu, *J* = 6.2 Hz, 1H), 4.04 (t,*J* = 6.7 Hz, 2H), 3.91 (s, 3H), 3.57 (br, 1H), 3.45 (br, 1H), 3.35-3.26 (m, 2H), 2.53 (qu, *J* = 6.5 Hz, 2H), 2.43-2.25 (m, 8H), 1.66-1.22 (m, 68H), 0.88 (t, *J* = 7.0 Hz, 9H); ¹³C NMR (150 MHz, acetone-*d*₆): δ 173.6, 173.5, 162.4, 128.0, 127.3, 112.1, 107.4, 74.1, 68.0, 64.6, 62.2, 55.2, 55.0, 39.6, 36.6, 35.6, 35.0, 35.0, 34.7, 32.7, 32.6, 30.9, 30.4, 30.34, 30.30, 30.25, 30.24-29.4 (7C, overlapped with solvent residual), 28.0 (2C, overlapped), 27.8, 27.6, 26.7, 26.1, 25.9, 25.7, 24.0, 23.3 (2C, overlapped), 14.4 (2C, overlapped); HRMS (ESI) m/z: [M+H]⁺ calcd for C₅₄H₁₀₂N₃O₆⁺ 888.7763, found 888.7753.

### Example 12. Production of heptadecan-9-yl 8-((2-hydroxy-6-(1-methyl-1H-pyrrole-3-carboxamido)hexyl)(6-oxo-6-(undecyloxy)hexyl)amino)octanoate (compound 19)

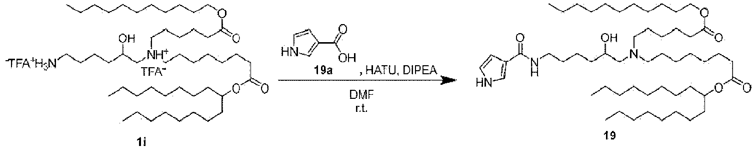

### Step 1: Synthesis of heptadecan-9-yl 8-((2-hydroxy-6-(1-methyl-1H-pyrrole-3-carboxamido)hexyl)(6-oxo-6-(undecyloxy)hexyl)amino)octanoate (compound 19)

To a solution of 1*H*-pyrrole-3-carboxylic acid (compound 19a, 29.4 mg, 0.235 mmol), and DIPEA (0.25 mL, 1.4 mmol) in anhydrous DMF (1.7 mL) in an oven-dried flask, a solution of HATU (57.3 mg, 0.151 mmol) in anhydrous DMF (1.0 mL) was added at 0 °C and stirred at room temperature for 30 min. A solution of heptadecan-9-yl 8-((6-amino-2-hydroxyhexyl) (6-oxo-6-undecyloxy)hexyl)octanoate trifluoroacetate salt (compound 1i, 74.6 mg, 0.0739 mmol) in anhydrous DMF (1.0 mL) was then added to the reaction mixture at 0 °C and stirred at room temperature for 16 h. After completion, the mixture was diluted with CH₂Cl₂, the organic layer was washed with brine, dried in sodium sulfate, and concentrated. The residue was purified using silica gel chromatography (CH₂Cl₂/CH₃OH/NH₄OH (33%), 200:10:1) to give heptadecan-9-yl 8-((2-hydroxy-6-(1*H*-pyrrole-3-carboxamido)hexyl)(6-oxo-6-(undecyloxy)hexyl)amino)octanoate (compound 19, 21.4 mg, 33%) as a colorless oil.

¹H NMR (600 MHz, DMSO-*d*₆): δ 7.62 (t, *J* = 5.7 Hz, 1H), 7.19 (t, *J* = 1.9 Hz, 1H), 6.65 (t, *J* = 4.9, 1H), 6.40 (dd, *J* = 2.7, 1.8 Hz, 1H), 4.77 (qu, *J* = 6.2 Hz, 1H), 3.98 (t, *J* = 6.5 Hz, 2H), 3.60 (s, 3H), 3.51 (s, 1H), 3.43 (br, 1H), 3.13 (q, *J=* 6.0 Hz, 2H), 2.34 (t, *J* = 7.1 Hz, 4H), 2.27-2.21 (m, 6H), 1.56-1.17 (m, 68H), 0.84 (t, *J* = 7.0 Hz, 9H); ¹³C NMR (150 MHz, DMSO-*d*₆): δ 173.3, 173.0, 163.9, 124.4, 122.6, 120.5, 108.0, 73.4, 68.1, 64.0, 61.5, 54.6, 54.4, 39.0, 36.4, 35.3, 34.3, 34.07, 34.04, 31.77, 31.72, 30.3, 29.46, 29.43, 29.40, 29.34, 29.26, 29.19, 29.11, 29.08, 29.05, 28.9, 28.6, 27.2, 27.1, 26.82, 26.75, 25.8, 25.2, 25.1, 24.9, 23.3, 22.57, 22.55, 14.4 (2C, overlapped); HRMS (ESI) m/z: [M+H]⁺ calcd for C₅₄H₁₀₂N₃O₆⁺ 888.7763, found 888.7731.

### Example 13. Production of heptadecan-9-yl 8-((2-hydroxy-6-(3-hydroxy-1H-pyrrole-2-carboxamido)hexyl)(6-oxo-6-(undecyloxy)hexyl)amino)octanoate (compound 21)

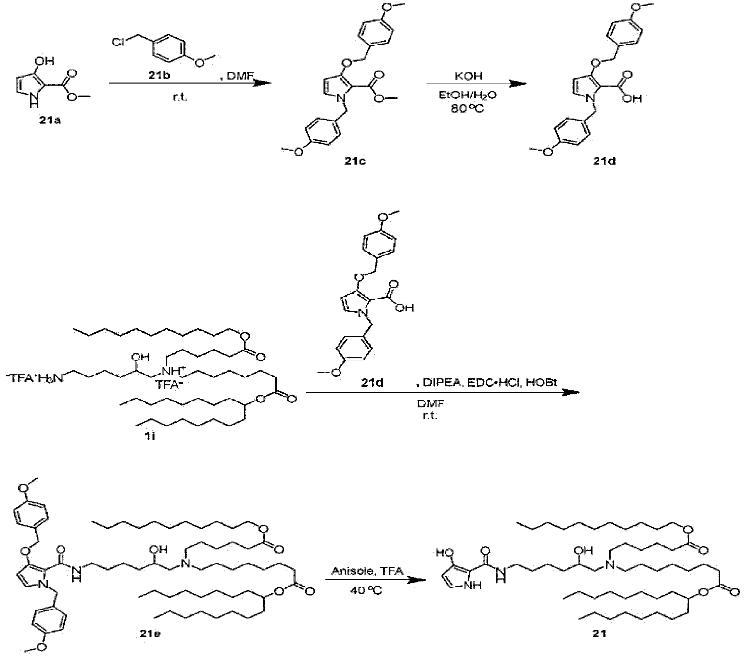

### Step 1: Synthesis of methyl 1-(4-methoxybenzyl)-3-((4-methoxybenzyl)oxy)-1H-pyrrole-2-carboxylate (compound 21c)

Methyl 3-hydroxy-1*H*-pyrrole-2- carboxylate (compound 21a, 81.5 mg, 0.578 mmol) was placed in an oven-dried flask and dissolved in anhydrous DMF (1.0 mL). The solution was cooled to 0 °C in an ice bath, and NaH (60%, 51.9 mg, 1.30 mmol) and 1-(chloromethyl)-4-methoxybenzene (compound 21b, 0.200 mL, 1.28 mmol) were added at 0 °C .The reaction mixture was allowed to warm to room temperature and stirred for 14 h. The mixture was concentrated, purified using silica gel chromatography (Hexane/EtOAc, 3:1) to afford methyl 1-(4-methoxybenzyl)-3- ((4-methoxybenzyl)oxy)-1*H*-pyrrole-2-carboxylate (compound 21c, 61.9 mg, 28%) as a white solid.

¹H NMR (400 MHz, acetone-*d*₆): δ 7.39 (d, *J* = 8.7 Hz, 2H), 7.10 (d, *J* = 8.7 Hz, 2H), 6.98 (d, *J* = 3.0 Hz, 1H), 6.92 (d, *J* = 8.7 Hz, 2H), 6.84 (d, *J* = 8.7 Hz, 2H), 5.99 (d, *J* = 3.1, 1H), 5.42 (s, 2H), 4.99 (s, 2H), 3.79 (s, 3H), 3.75 (s, 3H), 3.70 (s, 3H); HRMS (ESI) m/z: [M+H]⁺ calcd for C₂₂H₂₄NO₅⁺ 382.1649, found 382.1654.

### Step 2: Synthesis of 1-(4-methoxybenzyl)-3-((4-methoxybenzyl)oxy)-1H-pyrrole-2-carboxylic acid (compound 21d)

A solution of methyl 1-(4-methoxybenzyl)-3-((4-methoxybenzyl)oxy)-1*H*-pyrrole-2-carboxylate (compound 21c, 61.9 mg, 0.162 mmol) in absolute ethanol (2.0 mL) was treated with 2N KOH solution (293 uL, 0.585 mmol) in a sealed 4 mL vial. The mixture was stirred at 80 °C for 17 h. Reaction progress was monitored by TLC. The mixture was concentrated and dried under high vacuum to obtained 1-(4-methoxybenzyl)-3-((4-methoxybenzyl)oxy)-1*H*-pyrrole-2-carboxlic acid (compound 21d) as a crude solid, which was used in the following reaction without futher purification.

### Step 3: Synthesis of heptadecan-9-yl 8-((2-hydroxy-6-(1-(4-methoxybenzyl)-3-((4-methoxybenzyl)oxy)-1H-pyrrole-2-carboxamido)hexyl)(6-oxo-6-(undecyloxy)hexyl)amino)octanoate (compound 21e)

Heptadecan-9-yl 8-((6-amino-2-hydroxyhexyl) (6-oxo-6-undecyloxy)hexyl)octanoate trifluoroacetate salt (compound 1i, 61.3 mg, 0.0607 mmol), 1-(4-methoxybenzyl)-3-((4-methoxybenzyl)oxy)-1*H*-pyrrole-2-carboxylic acid (compound 21d, all obtained products from previous step), and DIPEA (0.100 mL, 0.574 mmol) was placed in an oven-dried flask, and dissolved in anhydrous DMF (1.0 mL). The solution was cooled to 0 °C in an ice bath, followed by the sequential addition of HOBt (23.8 mg, 0.176 mmol) and EDC·HCl (25.0 mg, 0.131 mmol). The reaction mixuter was stirred at room temperature for 20 h. The mixture was concentrated and purified using silica gel chromatography (Hexane/EtOAc/CH₃OH, 14:8:1) to give heptadecan-9-yl 8-((2-hydroxy-6-(1-(4-methoxybenzyl)-3-((4-methoxybenzyl)oxy)-1*H*-pyrrole-2-carboxamido)hexyl)(6-oxo-6-(undecyloxy)hexyl)amino)octanoate (compound 21e, 36.1 mg, 53%) as a colorless oil.

¹H NMR (600 MHz, acetone-*d*₆): δ 7.43 (d, *J =* 8.6 Hz, 2H), 7.22 (t, *J =* 5.5 Hz, 1H), 7.18 (d, *J* = 8.6 Hz, 2H), 6.96 (d, *J* = 8.7 Hz, 2H), 6.82 (d, *J* = 8.7 Hz, 2H), 6.81 (d, *J* = 3.1 Hz, 1H), 6.02 (d, *J =* 3.1 Hz, 1H), 5.56 (s, 2H), 5.06 (s, 2H), 4.87 (qu, *J* = 6.3 Hz, 1H), 4.03 (t, *J* = 6.7 Hz, 2H), 3.82 (s, 3H), 3.75 (s, 3H), 3.53 (br, 1H), 3.27 (q, *J* = 6.5 Hz, 2H), 2.58-2.21 (m, 10H), 1.67-1.15 (m, 68H), 0.88 (t, *J =* 6.9 Hz, 9H); HRMS (ESI) m/z: calcd for [M+H]⁺ C₆₉H₁₁₆N₃O₉⁺ 1130.8706, found 1130.8734.

### Step 4: Synthesis of heptadecan-9-yl 8-((2-hydroxy-6-(3-hydroxy-1H-pyrrole-2-carboxamido)hexyl)(6-oxo-6-(undecyloxy)hexyl)amino)octanoate (compound 21)

TFA (0.5 mL, 6.53 mmol) was added to solution of heptadecan-9-yl 8-((2-hydroxy-6-(1-(4-methoxybenzyl)-3-((4-methoxybenzyl)oxy)-1*H-*pyrrole-2-carboxamido)hexyl)(6-oxo-6-(undecyloxy)hexyl)amino)octanoate (compound 21e, 19.9 mg, 0.0176 mmol) in anisole (20 uL, 0.18 mmol and stirred at 40 °C for 17 h. Uppon completion, the mixture was concentrated and purified using silica gel chromatography (Hexane/EtOAc/CH₃OH/NH₄OH (33%), 140:80:10:1) to give heptadecan-9-yl 8-((2-hydroxy-6-(3-hydroxy-1H-pyrrole-2-carboxamido)hexyl)(6-oxo-6-(undecyloxy)hexyl)amino)octanoate (compound 21, 4.23 mg, 27%) as a colorless oil.

¹H NMR (600 MHz, acetone-*d*₆): δ 9.86 (s, 1H), 7.01 (s, 1H), 6.66 (d, *J=* 2.9 Hz, 1H), 5.71 (d, *J =* 2.6 Hz, 1H), 4.87 (qu, *J =* 5.8 Hz, 1H), 4.04 (t, *J =* 6.7 Hz, 2H), 3.57 (br, 1H), 3.36 (br, 2H), 2.61-2.21 (m, 10H), 1.72-1.12 (m, 68H), 0.88 (t, *J =* 6.9 Hz, 9H); HRMS (ESI) m/z: [M+H]⁺ calcd for C₅₃H₁₀₀N₃O₇⁺ 890.7556, found 890.7554.

### Example 14. Production of heptadecan-9-yl 8-((2-hydroxy-6-(3-hydroxy-1-methyl-1H-pyrrole-2-carbozamido)hezyl)(6-oxo-6-(undecyloxy)hexyl)amino)octanoate (compound 24)

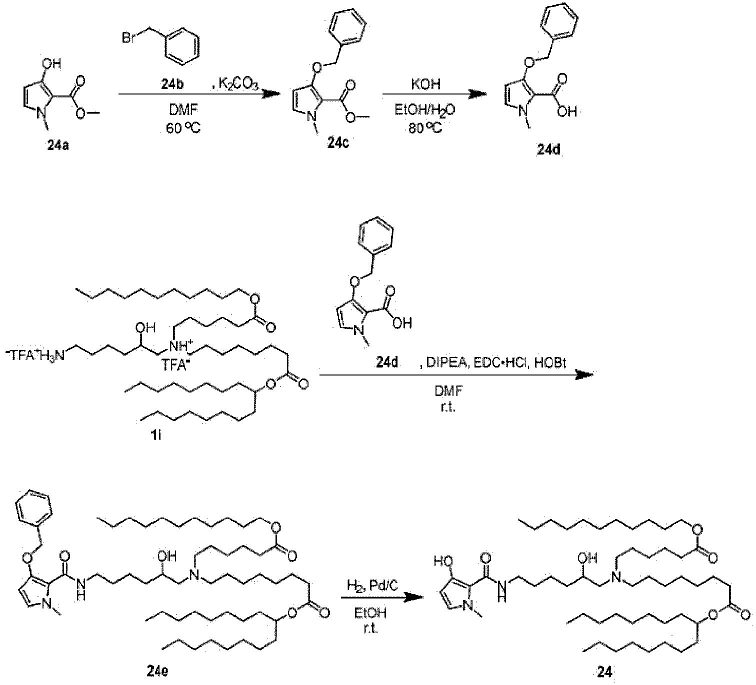

### Step 1: Synthesis of methyl 3-(benzyloxy)-1-methyl-1H-pyrrole-2-carboxylate (compound 24c)

Methyl 3-hydroxy-1-methyl-1*H*-pyrrole-2-carboxylic acid (compound 24a, 87.0 mg, 0.561 mmol) was placed in an oven-dried flask, dissolve in anhydride DMF (1.12 mL). K₂CO₃ (775 mg, 5.61 mmol) and benzyl bromide (compound 24b, 0.67 mL, 5.6 mmol) was added in order, and stirred at 60 °C for 50 h. The mixture was diluted with CH₂Cl₂, the organic layer was washed with brine, dried over anhydrous sodium sulfate, and concentrated. The residue was purified using silica gel chromatography (ether/hexane, 1:2) to give methyl 3-(benzyloxy)-1-methyl-1*H*-pyrrole-2-carboxylate (compound 24c, 104 mg, 76%) as a colorless liquid.

¹H NMR (400 MHz, acetone-*d*₆): δ 7.48 (d, *J =* 7.5 Hz, 2H), 7.37 (t, *J =* 7.4 Hz, 2H), 7.29 (t, *J* = 7.3 Hz, 1H), 6.79 (d, *J* = 2.9 Hz, 1H), 5.90 (d, *J* = 3.0 Hz, 1H), 5.07 (s, 2H), 3.81 (s, 3H), 3.75 (s, 3H); ¹³C NMR (150 MHz, acetone-*d*₆): δ 162.2, 154.3, 138.9, 129.1, 128.3, 128.1, 127.8, 109.3, 95.9, 72.7, 50.6, 37.8; HRMS (ESI) m/z: [M+H]⁺ calcd for C₁₄H₁₆NO₃⁺ 246.1125, found 246.1129.

### Step 2: Synthesis of 3-(benzyloxy)-1-methyl-1H-pyrrole-2-carboxylic acid (compound 24d)

Methyl 3-(benzyloxy)-1-methyl-1*H*-pyrrole-2-carboxylate (compound 24c, 26.4 mg, 0.108 mmol) was placed in a 4 mL vial, dissolve in absolute ethanol (0.86 mL). A solution of 2N KOH (0.22 mL, 0.43 mmol) was adde, and stirred at 80 °C for 5 h. The mixture was concentrated and dried under high vacuum to afford 3-(benzyloxy)-1-methyl-1*H*-pyrrole-2-carboxylic acid (compound 24d) as crude whit solid, which was used in the following reaction without futher purification.

### Step 3: Synthesis of heptadecan-9-yl 8-((6-(3-benzyloxy)-1-methyl-1H-pyrrole-2-carboxamido)-2-hydroxyhexyl)(6-oxo-6-(undecyloxy)hexyl)amino)octanoate (compound 24e)

Heptadecan-9-yl 8-((6-amino-2-hydroxyhexyl)(6-oxo-6-undecyloxy)hexyl)octanoate trifluoroacetate salt (1i, 54.0 mg, 0.0535 mmol), 3-(benzyloxy)-1-methyl-1*H*-pyrrole-2-carboxylic acid (24d, crude from previous step), and DIPEA (0.10 mL, 0.59 mmol) were placed in an oven-dried flask and dissolved in anhydrous DMF (1.78 mL). The mixture was cooled in an ice bath (0 °C), and HATU (26.0 mg, 0.0683 mmol) was added. The reaction mixture was stirred at room temperature for 2 h. Upon completion, the mixture was diluted with CH₂Cl₂, and the organic layer was washed with brine, dried over anhydrous sodium sulfate, and concentrated under reduced pressure. The crude product was purified by silica gel chromatography (hexane/EtOAc/MeOH/NH₄OH (33%), 140:70:10:1) to afford heptadecan-9-yl 8-((6-(3-benzyloxy)-1-methyl-1*H*-pyrrole-2-carboxamido)-2-hydroxyhexyl)(6-oxo-6-undecyloxyhexyl)amino)octanoate (24e, 37.5 mg, 71%) as a colorless oil.

¹H NMR (600 MHz, acetone-*d*₆): δ 7.49 (d, *J* = 7.5 Hz, 2H), 7.41 (t, *J =* 7.5 Hz, 2H), 7.35 (t, *J* = 7.4 Hz, 1H), 7.18 (t, *J* = 5.1 Hz, 1H), 6.64 (d, *J* = 3.0 Hz, 1H), 5.94 (d, *J* = 3.0 Hz, 1H), 5.15 (s, 2H), 4.87 (qu, *J* = 6.2 Hz, 1H), 4.03 (t, *J* = 6.7 Hz, 2H), 3.86 (s, 3H), 3.54 (br, 1H), 3.36-3.25 (m, 2H), 2.54 (q, *J* = 5.7 Hz, 2H), 2.46-2.22 (m, 8H), 1.68-1.16 (m, 68H), 0.88 (t, *J =* 7.0 Hz, 9H); ¹³C NMR (150 MHz, acetone-*d*₆): δ 173.6, 173.4, 161.9, 149.7, 138.1, 129.4, 128.9, 128.6, 125.1, 111.9, 95.0, 74.1, 73.5, 67.8, 64.6, 62.2, 55.1, 55.0, 38.9, 37.7, 35.6, 34.99, 34.96, 34.6, 32.65, 32.61, 30.9, 30.35, 30.34, 30.30, 30.26, 30.24-29.4 (7C, overlapped with solvent residual), 28.01, 27.97, 27.7, 27.6, 26.7, 26.1, 25.9, 25.7, 24.0, 23.3 (2C, overlapped), 14.4 (2C, overlapped); HRMS (ESI) m/z: [M+H]⁺ calcd for C₆₁H₁₀₈N₃O₇⁺ 994.8182, found 994.8147.

### Step 4: Synthesis of heptadecan-9-yl 8-((2-hydroxy-6-(3-hydroxy-1-methyl-1H-pyrrole-2-carboxamido)hexyl)(6-oxo-6-(undecyloxy)hexyl)amino)octanoate (compound 24)

Heptadecan-9-yl 8-((6-(3-benzyloxy)-1-methyl-1*H*-pyrrole-2-carboxamido)-2-hydroxyhexyl)(6-oxo-6-undecyloxyhexyl)amino)octanoate (24e, 19.6 mg, 0.0197 mmol) was placed in a 5 mL flask and dissolved in ethanol (2.0 mL). 10% Pd/C (wet, 55% total weight, 10 mg) was added, and the flask was charged with a hydrogen balloon. The reaction mixture was stirred at room temperature for 90 min. After completion, the mixture was filtered through Celite, concentrated under reduced pressure, and purified by silica gel chromatography (Hexane/EtOAc/MeOH/NH₄OH (33%), 70:40:10:1) to yield heptadecan-9-yl 8-((2-hydroxy-6-(3-hydroxy-1-methyl-1*H-*pyrrole-2-carboxamido)hexyl)(6-oxo-6-undecyloxyhexyl)amino)octanoate (24, 14.2 mg, 80%) as a colorless oil.

¹H NMR (600 MHz, acetone-*d*₆): δ 7.20 (s, 1H), 6.53 (d, *J* = 2.7 Hz, 1H), 5.63 (d, *J =* 2.8 Hz, 1H), 4.87 (qu, *J* = 6.2 Hz, 1H), 4.04 (t, *J* = 6.7 Hz, 2H), 3.83 (s, 3H), 3.58 (sext, *J* = 4.3 Hz, 1H), 3.35 (q, *J* = 6.6 Hz, 2H), 2.54 (dt, *J* = 12.8, 7.7 Hz, 2H), 2.44-2.26 (m, 8H), 1.64-1.23 (m, 68H), 0.88 (t, *J =* 7.0 Hz, 9H); ¹³C NMR (150 MHz, acetone-*d*₆): δ 173.6, 173.5, 162.7, 148.3, 125.2, 111.1, 96.6, 74.1, 67.9, 64.6, 62.2, 55.2, 55.0, 39.0, 37.5, 35.6, 35.0, 35.0, 34.7, 32.7, 32.6, 31.1, 30.35, 30.34, 30.30, 30.26, 30.22-29.44 (7C, overlapped with solvent residual), 28.01, 28.00, 27.8, 27.6, 26.7, 26.1, 25.9, 25.7, 24.0, 23.3 (2C, overlapped), 14.3 (2C, overlapped); HRMS (ESI) m/z: [M+H]⁺ calcd for C₅₄H₁₀₂N₃O₇⁺ 904.7712, found 904.7689.

### Example 15. Production of heptadecan-9-yl 8-((2-hydroxy-6-(1H-imidazole-2-carboxamido)hexyl)(6-oxo-6-(undecyloxy)hexyl)amino)octanoate (compound 33)

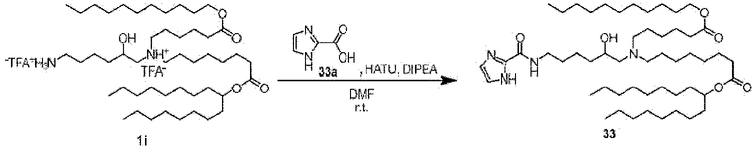

### Step 1: Synthesis of heptadecan-9-yl 8-((2-hydroxy-6-(1H-imidazole-2-carboxamido)hexyl) (6-oxo-6-(undecyloxy)hexyl)amino)octanoate (compound 33)

Heptadecan-9-yl 8-((6-amino-2-hydroxyhexyl)(6-oxo-6-undecyloxy)hexyl)octanoate trifluoroacetate salt (1i, 44.5 mg, 0.0440 mmol), 1*H*-imidazole-2-carboxylic acid (33a, 17.9 mg, 0.160 mmol), and DIPEA (0.08 mL, 0.44 mmol) were added to an oven-dried flask and dissolved in anhydrous DMF (4.4 mL). The reaction mixture was cooled in an ice bath (0 °C), followed by the addition of HATU (33.5 mg, 0.0881 mmol). The mixture was then stirred at room temperature for 3 h. After completion, the mixture was diluted with CH₂Cl₂, and the organic layer was washed with brine, dried over anhydrous sodium sulfate, and concentrated under reduced pressure. The crude product was purified by silica gel chromatography (hexane/EtOAc/CH₃OH/NH₄OH (33%), 80:40:10:1) to yield heptadecan-9-yl 8-((2-hydroxy-6-(1*H*-imidazole-2-carboxamido)hexyl)(6-oxo-6-undecyloxyhexyl)amino)octanoate (33, 14.2 mg, 37%) as a colorless oil.

¹H NMR (600 MHz, acetone-*d*₆): δ 11.91 (s, 1H), 7.69 (s, 1H), 7.27 (s, 1H), 7.03 (s, 1H), 4.87 (qu, *J* = 6.2 Hz, 1H), 4.04 (t, *J* = 6.6 Hz, 2H), 3.77 (s, 3H), 3.58 (br, 1H), 3.39 (q, *J* = 6.4 Hz, 2H), 2.52 (br, 2H), 2.46-2.24 (m, 8H), 1.69-1.15 (m, 68H), 0.88 (t, *J* = 7.0 Hz, 9H); ¹³C NMR (150 MHz, acetone-*d*₆): δ 173.67, 173.5, 159.2, 129.9, 119.8, 111.5, 74.1, 67.9, 64.6, 62.2, 55.2, 55.0, 39.6, 35.6, 35.0, 35.0, 34.7, 32.7, 32.6, 30.7, 30.34 (2C, overlapped), 30.30, 30.26, 30.24-29.4 (7C, overlapped with solvent residual), 28.0 (2C, overlapped), 27.8, 27.6, 26.7, 26.1, 25.9, 25.7, 23.9, 23.3 (2C, overlapped), 14.4 (2C, overlapped); 28.0 (2C, overlapped), 27.8, 27.6, 26.7, 26.1, 25.9, 25.7, 24.0, 23.3 (2C, overlapped), 14.4 (2C, overlapped); HRMS (ESI) m/z: [M+H]⁺ calcd for C₅₂H₉₉N₄O₆⁺ 875.7559, found 875.7530.

### Example 16. Production of heptadecan-9-yl 8-((2-hydroxy-6-(1H-imidazole-4-carboxamido)hexyl)(6-oxo-6-(undecyloxy)hexyl)amino)octanoate (compound 34)

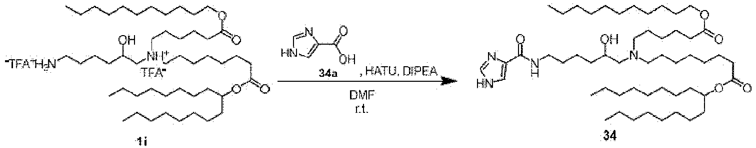

### Step 1: Synthesis of heptadecan-9-yl 8-((2-hydroxy-6-(1H-imidazole-4-carboxamido)hexyl) (6-oxo-6-(undecyloxy)hexyl)amino)octanoate (compound 34)

1*H*-Imidazole-4-carboxylic acid (34a, 16.5 mg, 0.147 mmol), HATU (31.2 mg, 0.0821 mmol), and DIPEA (0.120 mL, 0.689 mmol) were placed in an oven-dried flask and dissolved in anhydrous DMF (3.5 mL). The mixture was stirred at room temperature for 30 min. Separately, heptadecan-9-yl 8-((6-amino-2-hydroxyhexyl)(6-oxo-6-undecyloxy)hexyl)octanoate trifluoroacetate salt (1i, 41.2 mg, 0.0408 mmol) was dissolved in anhydrous DMF (1.0 mL) and added to the reaction mixture. The resulting solution was stirred at room temperature for 1 h. The reaction mixture was concentrated under reduced pressure and purified by silica gel chromatography (CH₂Cl₂/CH₃OH/NH₄OH (33%), 200:10:1) to afford heptadecan-9-yl 8-((2-hydroxy-6-(1*H*-imidazole-4-carboxamido)hexyl)(6-oxo-6-undecyloxyhexyl)amino)octanoate (34, 19.8 mg, 55%) as a colorless oil.

¹H NMR (600 MHz, DMSO-*d*₆) δ 12.41 (s, 1H), 7.78 (d, *J* = 7.0 Hz, 1H), 7.67 (d, *J =* 1.2 Hz, 1H), 7.55 (s, 1H), 4.83-4.72 (m, 1H), 4.18-3.99 (m, 1H), 3.98 (t, *J* = 6.5 Hz, 2H), 3.43 (d, *J =* 7.4 Hz, 1H), 3.19 (q, *J =* 6.7 Hz, 2H), 2.41-2.10 (m, 10H), 1.76-1.04 (m, 68H), 0.84 (t, *J =* 7.0, 9H); ¹³C NMR (150 MHz, DMSO-*d*₆) δ 172.8, 172.5, 162.0, 136.5, 135.2, 118.5, 72.9, 67.6, 63.6, 61.0, 54.1, 54.0, 40.1, 38.2, 34.8, 33.8, 33.60, 33.56, 31.3, 31.2, 28.98, 28.95, 28.92, 28.87, 28.8, 28.7, 28.63, 28.60, 28.58, 28.4, 28.1, 26.7, 26.6, 26.4, 26.3, 25.4, 24.7, 24.6, 24.5, 22.8, 22.09, 22.07, 13.9; HRMS (ESI) m/z: [M+H]⁺ calcd for C₅₃H₉₉N₄O₆⁺ 875.7565, found 875.7544.

### Example 17. Production of heptadecan-9-yl 8-((2-hydroxy-6-(2-hydroxy-1H-imidazole-4-carboxamido)hexyl)(6-oxo-6-(undecyloxy)hexyl)amino)octanoate (compound 35)

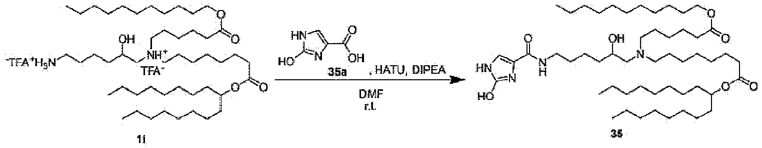

### Step 1: Synthesis of heptadecan-9-yl 8-((2-hydroxy-6-(2-hydroxy-1H-imidazole-4-carboxamido)hexyl)(6-oxo-6-(undecyloxy)hexyl)amino)octanoate (compound 35)

Heptadecan-9-yl 8-((6-amino-2-hydroxyhexyl)(6-oxo-6-undecyloxy)hexyl)octanoate trifluoroacetate salt (1i, 120.4 mg, 0.113 mmol), 2-hydroxy-1*H*-imidazole-4-carboxylic acid (35a, 24.9 mg, 0.194 mmol), and DIPEA (0.230 mL, 1.32 mmol) were added to an oven-dried flask and dissolved in anhydrous DMF (4.5 mL). The mixture was cooled in an ice bath (0 °C), followed by the addition of HATU (45.0 mg, 0.118 mmol). The reaction mixture was stirred at room temperature for 20 h. After completion, the reaction mixture was concentrated under reduced pressure and purified by silica gel chromatography (CH₂Cl₂/CH₃OH/NH₄OH (33%), 200:10:1), followed by LH-20 gel filtration chromatography (100% methanol), to afford heptadecan-9-yl 8-((2-hydroxy-6-(2-hydroxy-1*H*-imidazole-4-carboxamido)hexyl)(6-oxo-6-undecyloxyhexyl)amino)octanoate (35, 26.9 mg, 25%) as a colorless oil.

¹H NMR (600 MHz, DMSO*d*₆) δ 10.20 (s, 1H), 10.11 (t, *J* = 2.3 Hz, 1H), 7.71 (t, *J* = 5.6 Hz, 1H), 6.97 (t, *J* = 2.2 Hz, 1H), 4.95-4.60 (m, 1H), 3.98 (t, *J =* 6.5 Hz, 3H), 3.43 (d, *J* = 6.2 Hz, 1H), 3.13 (q, *J* = 6.2 Hz, 2H), 2.45-2.06 (m, 10H), 1.81-1.04 (m, 68H), 0.85 (t, J = 7.0, 9H); ¹³C NMR (150 MHz, DMSO-*d*₆) δ 172.8, 172.6, 158.6, 153.8, 117.8, 112.4, 72.9, 67.6, 63.6, 61.0, 54.1, 53.9, 40.1, 38.5, 34.7, 33.8, 33.600, 33.597, 31.30, 31.26, 29.5, 28.99, 28.96, 28.93, 28.88, 28.8, 28.7, 28.63, 28.61, 28.58, 28.5, 28.1, 26.7, 26.6, 26.34, 26.28, 25.4, 24.7, 24.6, 24.5, 22.8, 22.10, 22.08, 13.9; HRMS (ESI) m/z: [M+H]⁺ calcd for C₅₃H₉₉N₄O₇⁺ 891.7514, found 891.7487.

### Example 18. Production of heptadecan-9-yl 8-((2-hydroxy-6-(1-methyl-1H-imidazole-2-carboxamido)hexyl)(6-oxo-6-(undecyloxy)hexyl)amino)octanoate (compound 36)

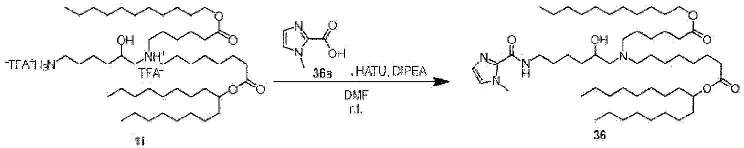

### Step 1: Production of heptadecan-9-yl 8-((2-hydroxy-6-(1-methyl-1H-imidazole-2-carboxamido)hexyl)(6-oxo-6-(undecyloxy)hexyl)amino)octanoate (compound 36)

Heptadecan-9-yl 8-((6-amino-2-hydroxyhexyl)(6-oxo-6-undecyloxy)hexyl)octanoate trifluoroacetate salt (1i, 100 mg, 0.0991 mmol), 1-methyl-1*H*-imidazole-2-carboxylic acid (36a, 16.8 mg, 0.133 mmol), and DIPEA (0.20 mL, 1.1 mmol) were added to an oven-dried flask and dissolved in anhydrous DMF (2.7 mL). HATU (41.7 mg, 0.110 mmol), dissolved in anhydrous DMF (1.0 mL), was added dropwise to the reaction mixture at 0 °C. The reaction was then stirred at room temperature for 100 min. The mixture was diluted with CH₂Cl₂, and the organic layer was washed with brine, dried over sodium sulfate, and concentrated under reduced pressure. The crude residue was purified by silica gel chromatography (CH₂Cl₂/CH₃OH/NH₄OH (33%), 200:10:1) to yield heptadecan-9-yl 8-((2-hydroxy-6-(1-methyl-1*H*-imidazole-2-carboxamido)hexyl)(6-oxo-6-undecyloxyhexyl)amino)octanoate (36, 71.4 mg, 81%) as a colorless oil.

¹H NMR (600 MHz, CDCl₃): δ 7.44 (s, 1H), 6.99 (s, 1H), 6.95 (s, 1H), 4.85 (qu, *J* = 6.2 Hz, 1H), 4.05 (t, *J =* 6.8 Hz, 2H), 4.04 (s, 3H), 3.94 (br, 1H), 3.44-3.35 (m, 2H), 3.03 (br, 6H), 2.32 (t, *J* = 7.2 Hz, 2H), 2.28 (t, *J* = 7.5 Hz, 2H), 1.78-1.19 (m, 68H), 0.88 (t, *J= 7.0* Hz, 9H); ¹³C NMR (150 MHz, DMSO-*d*₆): δ 172.7, 172.6, 158.8, 138.8, 126.8, 125.8, 73.0, 63.6, 38.2, 34.9, 34.6, 33.8, 33.5, 33.3, 31.3, 31.2, 29.2, 28.97, 28.96, 28.92, 28.84, 28.77, 28.70, 28.62, 28.56, 28.3, 28.1, 25.4, 24.7, 24.5, 24.1, 22.08, 22.06, 13.9 (2C, overlapped); HRMS (ESI) m/z: [M+H]⁺ calcd for C₅₃H₁₀₁N₄O₆⁺ 889.7716, found 889.7693.

### Example 19. Production of heptadecan-9-yl 8-((2-hydroxy-6-(1-methyl-1H-imidazole-4-carboxamido)hexyl)(6-oxo-6-(undecyloxy)hexyl)amino)octanoate (compound 37)

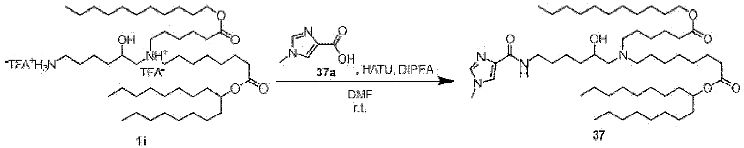

### Step 1: Production of heptadecan-9-yl 8-((2-hydroxy-6-(1-methyl-1H-imidazole-4-carboxamido)hexyl)(6-oxo-6-(undecyloxy)hexyl)amino)octanoate (compound 37)

1*H*-imidazole-4-carboxylic acid (34a, 16.5 mg, 0.147 mmol), HATU (31.2 mg, 0.0821 mmol), and DIPEA (0.120 mL, 0.689 mmol) were placed in an oven-dried flask and dissolved in anhydrous DMF (3.5 mL). The reaction mixture was stirred at room temperature for 30 min. A solution of heptadecan-9-yl 8-((6-amino-2-hydroxyhexyl)(6-oxo-6-undecyloxy)hexyl)octanoate trifluoroacetate (1i, 41.2 mg, 0.0408 mmol) in anhydrous DMF (1.0 mL) was then added at 0 °C, and the mixture was stirred at room temperature for 23 h. Upon completion, the reaction mixture was concentrated under reduced pressure. The residue was purified by silica gel column chromatography (CH₂Cl₂/CH₃OH/NH₄OH (33%), 200:10:1) to afford heptadecan-9-yl 8-((2-hydroxy-6-(1*H*-imidazole-4-carboxamido)hexyl)(6-oxo-6-(undecyloxy)hexyl)amino)octanoate (34, 19.8 mg, 55%) as a colorless oil.

¹H NMR (600 MHz, acetone-*d*₆): δ 7.50 (s, 1H), 7.48 (d, *J* = 1.3, 1H), 7.33 (s, 1H), 4.87 (qu, *J* = 6.2 Hz, 1H), 4.04 (t, *J* = 6.6 Hz, 2H), 3.77 (s, 3H), 3.58 (br, 1H), 3.45 (br, 1H), 3.35 (q, *J =* 6.6 Hz, 2H), 2.53 (qu, *J* = 6.5 Hz, 2H), 2.44-2.24 (m, 8H), 1.72-1.14 (m, 68H), 0.88 (t, *J* = 7.0 Hz, 9H); ¹³C NMR (150 MHz, acetone-*d*₆): δ 173.6, 173.5, 162.8, 138.7, 138.4, 123.2, 74.1, 67.9, 64.6, 62.2, 55.2, 55.0, 39.2, 35.6, 35.00, 34.97, 34.7, 33.7, 32.65, 32.61, 31.0, 30.35, 30.34, 30.30, 30.25, 30.24-29.4 (7C, overlapped with solvent residual), 28.0 (2C, overlapped), 27.8, 27.6, 26.7, 26.1, 25.9, 25.7, 24.0, 23.3 (2C, overlapped), 14.4 (2C, overlapped); HRMS (ESI) m/z: [M+H]⁺ calcd for C₅₃H₁₀₁N₄O₆⁺ 889.7716, found 889.7692.

### Example 20. Production of heptadecan-9-yl 8-((2-hydroxy-6-(1-methyl-1H-imidazole-5-carboxamido)hexyl)(6-oxo-6-(undecyloxy)hexyl)amino)octanoate (compound 38)

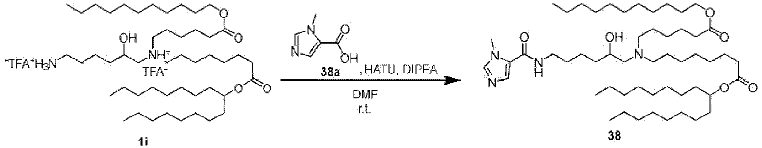

### Step 1: Production of heptadecan-9-yl 8-((2-hydroxy-6-(1-methyl-1H-imidazole-5-carboxamido)hexyl)(6-oxo-6-(undecyloxy)hexyl)amino)octanoate (compound 38)

1-Methyl-1*H*-imidazole-5-carboxylic acid (38a, 25.7 mg, 0.229 mmol) and DIPEA (0.250 mL, 1.44 mmol) were added to an oven-dried flask and dissolved in anhydrous DMF (1.5 mL). HATU (33.4 mg, 0.0879 mmol), dissolved in anhydrous DMF (0.5 mL), was added dropwise at 0 °C. The mixture was stirred at room temperature for 30 min. A solution of heptadecan-9-yl 8-((6-amino-2-hydroxyhexyl)(6-oxo-6-undecyloxy)hexyl)octanoate trifluoroacetate salt (1i, 79.1 mg, 0.0784 mmol) in anhydrous DMF (1.0 mL) was then added dropwise at 0 °C, and the reaction was stirred at room temperature for 20 h. The mixture was concentrated and purified using silica gel chromatography (CH₂Cl₂/CH₃OH/NH₄OH (33%), 200:10:1) and LH-20 column (methanol 100%) to give heptadecan-9-yl 8-((2-hydroxy-6-(1-methyl-1*H*-imidazole-5-carboxamido)hexyl)(6-oxo-6-(undecyloxy)hexyl)amino)octanoate (compound 38, 30.2 mg, 43%) as a colorless oil.

¹H NMR (400 MHz, DMSO-*d*₆) δ 8.18 (t, *J* = 5.7 Hz, 1H), 7.69 (s, 1H), 7.52 (d, *J* = 1.0 Hz, 1H), 4.77 (qu, *J =* 6.3 Hz, 1H), 4.02 (d, *J =* 3.8 Hz, 1H), 3.98 (t, *J =* 6.5 Hz, 2H), 3.79 (br, 3H), 3.44 (s, 1H), 3.16 (q, *J* = 6.5 Hz, 2H), 2.40-2.16 (m, 10H), 1.69-1.07 (m, 68H), 0.84 (t, *J =* 6.6 Hz, 9H); ¹³C NMR (150MHz, DMSO-*d*₆) δ 172.8, 172.9, 159.8, 141.6, 131.5, 126.0, 72.9, 67.6, 63.9, 61.0, 54.1, 54.0, 40.1, 38.5, 34.7, 33.8, 33.60, 33.56, 33.4, 31.3, 31.2, 29.5, 28.99, 28.96, 28.92, 28.9, 28.8, 28.7, 28.63, 28.61, 28.58, 28.4, 28.1, 26.7, 26.6, 26.4, 26.3, 25.4, 24.7, 24.6, 24.5, 22.8, 22.10, 22.08, 13.9; HRMS (ESI) m/z:[M+H]⁺ calcd for C₅₃H₁₀₁N₄O₆⁺ 889.7721, found, 889.7705.

### Example 21. Production of heptadecan-9-yl 8-((2-hydroxy-6-(2-hydroxy-1-methyl-1H-imidazole-4-carbozamido)hexyl)(6-oxo-6-(undecyloxy)hezyl)amino)octanoate (compound 39)

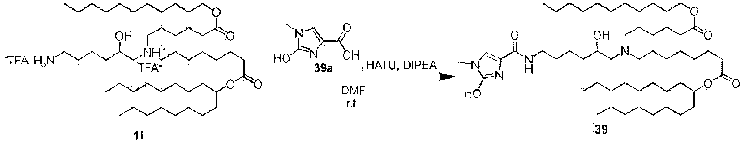

### Step 1: Synthesis of heptadecan-9-yl 8-((2-hydroxy-6-(2-hydroxy-1-methyl-1H-imidazole-4-carboxamido)hexyl)(6-oxo-6-(undecyloxy)hexyl)amino)octanoate (compound 39)

Heptadecan-9-yl 8-((6-amino-2-hydroxyhexyl)(6-oxo-6-undecyloxy)hexyl)octanoate trifluoroacetate (1i, 91.2 mg, 0.0903 mmol), 2-hydroxy-1-methyl-1*H*-imidazole-4-carboxylic acid (39a, 27.8 mg, 0.196 mmol), and DIPEA (0.160 mL, 0.919 mmol) were placed in an oven-dried flask and dissolved in anhydrous DMF (3.5 mL). HATU (43.3 mg, 0.114 mmol), dissolved in anhydrous DMF (0.5 mL), was added to the solution at 0 °C in an ice bath. The reaction mixture was stirred at room temperature for 18 h.The mixture was concentrated and purified using silica gel chromatography (CH₂Cl₂/CH₃OH/NH₄OH (33%), 200:10:1) and LH-20 column (methanol 100%) to give heptadecan-9-yl 8-((2-hydroxy-6-(2-hydroxy-1-methyl-1*H*-imidazole-4-carboxamido)hexyl)(6-oxo-6-(undecyloxy)hexyl)amino)octanoate (compound 39, 46.3 mg, 52%) as a colorless oil.

¹H NMR (500 MHz, DMSO-*d*₆) δ 10.30 (d, *J* = 2.1 Hz, 1H), 7.74 (t, *J* = 5.6 Hz, 1H), 7.09 (d, *J =* 2.1 Hz, 1H), 4.77 (qu, *J =* 6.3 Hz, 1H), 4.02 (s, 1H), 3.98 (t, *J =* 6.5 Hz, 2H), 3.43 (s, 1H), 3.11 (m, 5H), 2.43-2.13 (m, 10H), 1.67-1.02 (m, 68H), 0.85 (td, J = 6.9, 1.4 Hz, 9H); ¹³C NMR (150 MHz, DMSO-*d*₆) δ 172.8, 172.5, 158.5, 152.9, 116.3, 116.2, 73.0, 67.7, 63.71, 63.66, 61.1, 60.6, 54.2, 54.0, 40.2, 38.7, 34.8, 33.9, 33.7, 32.3, 31.43, 31.40, 31.38, 29.7, 29.6, 29.12, 29.08, 29.00, 28.9, 28.85, 28.81, 28.75, 28.71, 28.6, 28.2, 26.8, 26.7, 26.5, 26.4, 25.5, 25.1, 24.8, 24.7, 24.58, 24.55, 22.9, 22.21, 22.19, 14.04, 13.98; HRMS (ESI) m/z: [M+H]⁺ calcd for C₅₃H₁₀₁N₄O₇⁺ 905.7670, found 905.7646.

### Example 22. Production of heptadecan-9-yl 8-((2-hydroxy-6-(2-hydroxy-1-methyl-1H-imidazole-5-carbozamido)hexyl)(6-oxo-6-(undecyloxy)hezyl)amino)octanoate (compound 40)

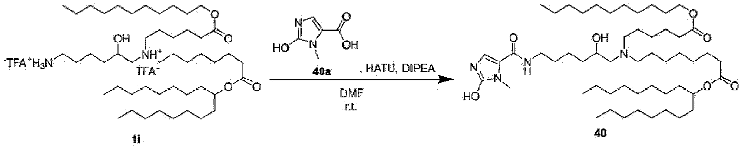

### Step 1: Synthesis of heptadecan-9-yl 8-((2-hydroxy-6-(2-hydroxy-1-methyl-1H-imidazole-5-carboxamido)hexyl)(6-oxo-6-(undecyloxy)hexyl)amino)octanoate (compound 40)

Heptadecan-9-yl 8-((6-amino-2-hydroxyhexyl)(6-oxo-6-undecyloxy)hexyl)octanoate trifluoroacetate (1i, 114 mg, 0.113 mmol), 2-hydroxy-1-methyl-1*H*-imidazole-5-carboxylic acid (40a, 41.0 mg, 0.288 mmol), and DIPEA (0.200 mL, 1.15 mmol) were placed in an oven-dried flask and dissolved in anhydrous DMF (4.5 mL). HATU (43.3 mg, 0.114 mmol), dissolved in anhydrous DMF (0.5 mL), was added to the reaction mixture at 0 °C in an ice bath. The reaction mixture was stirred at room temperature for 18 h. The mixture was concentrated and was purified using silica gel chromatography (CH₂Cl₂/CH₃OH/NH₄OH (33%), 200:10:1) and LH-20 column (methanol 100%) to give heptadecan-9-yl 8-((2-hydroxy-6-(2-hydroxy-1-methyl-1*H*-imidazole-5-carboxamido)hexyl)(6-oxo-6-(undecyloxy)hexyl)amino)octanoate (compound 40, 28.2 mg, 28%) as a colorless oil.

¹H NMR (600 MHz, DMSO-*d*₆) δ 10.44 (s, 1H), 7.91 (t, *J* = 5.7 Hz, 1H), 7.08 (s, 1H), 4.77 (qu, *J =* 6.2 Hz, 1H), 4.01 (d, *J =* 3.7 Hz, 1H), 3.98 (t, *J =* 6.5 Hz, 2H), 3.43 (s, 1H), 3.27 (s, 3H), 3.12 (q, *J =* 6.3 Hz, 2H), 2.39-2.17 (m, 10H), 1.60-1.06 (m, 68H), 1.00-0.73 (m, 9H); ¹³C NMR (150 MHz, DMSO-*d*₆) δ 172.8, 159.6, 153.6, 117.9, 111.9, 72.9, 67.6, 63.6, 54.1, 54.0, 40.1, 38.4, 34.7, 33.8, 33.5931, 33.5933, 31.30, 31.26, 29.5, 28.99, 28.96, 28.93, 28.88, 28.8, 28.7, 28.62, 28.59, 28.48, 28.46, 28.1, 26.7, 26.6, 26.35, 26.28, 25.4, 24.7, 24.6, 24.5, 22.8, 22.09, 22.08, 13.9; HRMS (ESI) m/z: [M+H]⁺ calcd for C₅₃H₁₀₁N₄O₇⁺ 905.7670, found 905.7657.

### Example 23. Production of heptadecan-9-yl 8-((6-(furan-2-carboxamido)-2-hydroxyhexyl)(6-oxo-6-(undecyloxy)hexyl)amino)octanoate (compound 50)

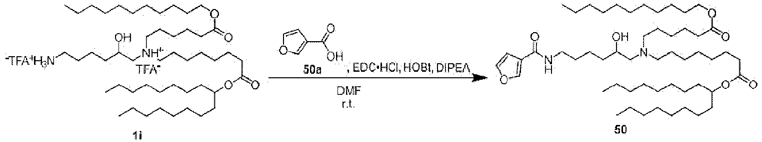

### Step 1: Synthesis of heptadecan-9-yl 8-((6-(furan-2-carboxamido)-2-hydroxyhexyl)(6-oxo-6-(undecyloxy)hexyl)amino)octanoate (compound 50)

Heptadecan-9-yl 8-((6-amino-3-hydroxyhexyl)(6-oxo-6-undecyloxy)hexyl)octanoate trifluoroacetate (1i, 19.4 mg, 0.0193 mmol), furan-3-carboxylic acid (50a, 9.3 mg, 0.083 mmol), and DIPEA (50 µL, 0.29 mmol) were placed in an oven-dried flask and dissolved in anhydrous DMF (2.0 mL). The solution was cooled to 0 °C in an ice bath, followed by the sequential addition of HOBt (9.6 mg, 0.071 mmol) and EDC·HCl (10.5 mg, 0.0547 mmol). The reaction mixture was stirred at 0 °C for 10 min, then allowed to warm to room temperature and stirred for 18 h. The mixture was concentrated and purified using silica gel chromatography (Hexane/EtOAc/CH₃OH/NH₄OH (33%), 70:40:10:1) to give heptadecan-9-yl 8-((6-(furan-2-carboxamido)-2-hydroxyhexyl)(6-oxo-6-(undecyloxy)hexyl)amino)octanoate (compound 50, 10.3 mg, 61%) as a colorless oil.

¹H NMR (500 MHz, acetone-*d*₆) δ 8.04 (s, 1H), 7.58 (s, 1H), 7.40 (s, 1H), 6.80 (s, 1H), 4.87 (q, *J* = 6.5 Hz, 1H), 4.04 (t, *J* = 6.7 Hz, 2H), 3.57 (s, 1H), 3.32 (d, *J* = 7.1 Hz, 2H), 2.70-2.18 (m, 10H), 1.71-1.13 (m, 68H), 0.88 (t, *J* = 6.6 Hz, 9H).

### Example 24. Production of heptadecan-9-yl 8-((2-hydroxy-6-(thiophene-3-carboxamido)hexyl)(6-oxo-6-(undecyloxy)hexyl)amino)octanoate (compound 51)

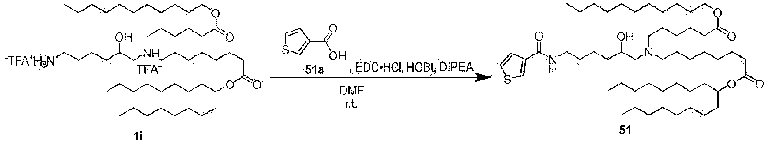

### Step 1: Synthesis of heptadecan-9-yl 8-((2-hydroxy-6-(thiophene-3-carboxamido)hexyl)(6- oxo-6-(undecyloxy)hexyl)amino)octanoate (compound 51)

Heptadecan-9-yl 8-((6-amino-2-hydroxyhexyl)(6-oxo-6-undecyloxy)hexyl)octanoate trifluoroacetate (1i, 20.1 mg, 0.0199 mmol), thiophene-3-carboxylic acid (51a, 10.5 mg, 0.0816 mmol), and DIPEA (50 µL, 0.28 mmol) were placed in an oven-dried flask and dissolved in anhydrous DMF (2.0 mL). The solution was cooled to 0 °C in an ice bath, followed by the sequential addition of HOBt (9.94 mg, 0.0736 mmol) and EDC·HCl (9.65 mg, 0.0503 mmol). The reaction mixture was stirred at 0 °C for 10 min, then allowed to warm to room temperature and stirred for 17 h. The mixture was concentrated and purified using silica gel chromatography (Hexane/EtOAc/CH₃OH/NH₄OH (33%), 140:80:10:1) to give heptadecan-9-yl 8-((2-hydroxy-6-(thiophene-3-carboxamido)hexyl)(6-oxo-6-(undecyloxy)hexyl)amino)octanoate (compound 51, 11.9 mg, 67%) as a colorless oil.

¹H NMR (400 MHz, acetone-*d*₆) δ 8.05 (dd, *J* = 2.9*,* 1.3 Hz, 1H), 7.58 (s, 1H), 7.54 (dd, *J* = 5.0, 1.3 Hz, 1H), 7.49 (dd, *J* = 5.1, 2.9 Hz, 1H), 4.89 (t, *J* = 6.3 Hz, 1H), 4.05 (t, *J* = 6.6 Hz, 2H), 3.59 (s, 1H), 3.45-3.30 (m, 2H), 2.64-2.25 (m, 10H), 1.77-1.09 (m,68H), 1.09-0.68 (m, 9H).

### Example 25. Production of heptadecan-9-yl 8-((6-(2-(2,4-dioxo-3,4-dihydropyrimidin-1(2H)-yl)acetamido)-2-hydroxyhexyl)(6-oxo-6-(undecyloxy)hexyl)amino)octanoate (compound 70)

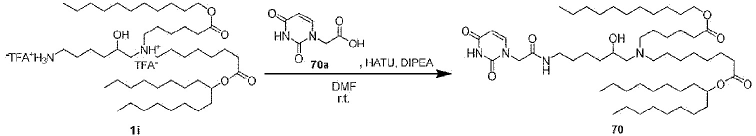

### Step 1: Synthesis of heptadecan-9-yl 8-((6-(2-(2,4-dioxo-3,4-dihydropyrimidin-1(2H)-yl)acetamido)-2-hydroxyhexyl)(6-oxo-6-(undecyloxy)hexyl)amino)octanoate (compound 70)

Heptadecan-9-yl 8-((6-amino-2-hydroxyhexyl)(6-oxo-6-undecyloxy)hexyl)octanoate trifluoroacetate (1i, 32.8 mg, 0.0325 mmol), 2-(2,4-dioxo-3,4-dihydropyrimidin-1(2*H*)-yl)acetic acid (70a, 12.0 mg, 0.065 mmol), and DIPEA (0.10 mL, 0.58 mmol) were placed in an oven-dried flask and dissolved in anhydrous DMF (3.2 mL). The solution was cooled to 0 °C in an ice bath, and HATU (18.5 mg, 0.0487 mmol) was added. The reaction mixture was stirred at 0 °C for 10 min, then allowed to warm to room temperature and stirred for 16 h. The mixture was concentrated and purified using silica gel chromatography (Hexane/EtOAc/CH₃OH/NH₄OH (33%), 70:40:10:1) to give heptadecan-9-yl 8-((6-(2-(2,4-dioxo-3,4-dihydropyrimidin-1(2*H*)-yl)acetamido)-2-hydroxyhexyl)(6-oxo-6-(undecyloxy)hexyl)amino)octanoate (compound 70, 22.4 mg, 74%) as a colorless oil.

¹H NMR (400 MHz, acetone-*d*₆) δ 9.96 (s, 1H), 7.50 (d, *J =* 7.8 Hz, 1H), 7.40 (s, 1H), 5.55 (d, *J =* 7.8 Hz, 1H), 4.87 (qu, *J =* 6.1 Hz, 1H), 4.41 (s, 2H), 4.04 (d, *J =* 6.5 Hz, 2H), 3.56 (br, 1H), 3.22 (q, *J =* 5.6 Hz, 2H), 2.60-2.22 (m, 10H), 1.72-1.07 (m, 68H), 0.88 (t, *J* = 6.1 Hz, 9H).

### Example 26. Production of heptadecan-9-yl 8-((6-((S)-2-acetamido-3-(1H-imidazol-5-yl)propanamido)-2-hydroxyhexyl)(6-oxo-6-(undecyloxy)hexyl)amino)octanoate (compound 76)

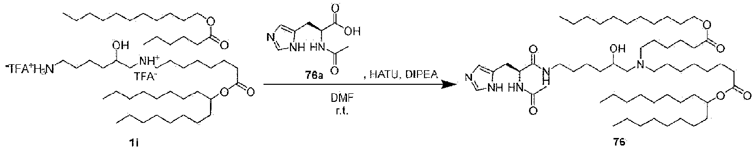

### Step 1: Synthesis of heptadecan-9-yl 8-((6-((S)-2-acetamido-3-(1H-imidazol-5-yl)propanamido)-2-hydroxyhexyl)(6-oxo-6-(undecyloxy)hexyl)amino)octanoate (compound 76)

Heptadecan-9-yl 8-((6-amino-2-hydroxyhexyl)(6-oxo-6-undecyloxy)hexyl)octanoate trifluoroacetate (1i, 27.6 mg, 0.0274 mmol), N-acetyl-L-histidine (76a, 11.0 mg, 0.055 mmol), and DIPEA (0.10 mL, 0.58 mmol) were placed in an oven-dried flask and dissolved in anhydrous DMF (9.1 mL). HATU (17.7 mg, 0.0466 mmol) was added to the reaction mixture at 0 °C in an ice bath. The reaction mixture was stirred at room temperature for 20 h. The mixture was concentrated and purified using silica gel chromatography (CH₂Cl₂/CH₃OH/NH₄OH (33%), 90:10:1) to give heptadecan-9-yl 8-((6-((S)-2-acetamido-3-(1*H*-imidazol-5-yl)propanamido)-2-hydroxyhexyl)(6-oxo-6-(undecyloxy)hexyl)amino)octanoate (compound 76, 12.2 mg, 51%) as a colorless oil.

¹H NMR (400 MHz, acetone-*d*₆) δ 7.67 (d, *J =* 7.8 Hz, 1H), 7.64 (s, 1H), 7.32 (s, 1H), 6.92 (s, 1H), 4.89 (br, 1H), 4.86 (qu, *J =* 6.3 Hz, 1H), 4.51 (s, 1H), 4.03 (t, *J* = 6.5 Hz, 2H), 4.01 (br, 1H), 3.17-2.87 (m, 4H), 2.40-1.72 (m, 10H), 1.72-1.05 (m, 68H), 0.87 (t, *J =* 6.0 Hz, 9H).

Compounds 122-178 listed in Table 1 below were synthesized by using aminoalkene hydrochlorides of varying chain lengths instead of 1-amino-5-hexyn hydrochlorides in Step 1 of Example 1, incorporating various alkyl tails in Steps 4 and 5, and using 1H-pyrrole-3-carboxylic acid in Step 6, as described in Step 1 of Example 13.

By applying the same method as in the Examples 2 and 3, compounds 4-6, 100-110, and 117-121 listed in Table 1 below were synthesized using aminoalkene hydrochlorides of varying chain lengths instead of 1-amino-5-hexin hydrochlorides in Step 1 of Example 3, varying amine or isocyanate derivatives instead of methylamine, and different alkyl tails in Steps 4 and 5.

By applying the same method as in the Example 4, compounds 12-99, 111-116, and 122 listed in Table 1 were synthesized using various carboxylic acid derivatives instead of cyclopentanecarboxylic acid in Step 1 of Example 4.

**[Table 1]**

| Compoun d No. | Structure | Generic name | MS (calcd.) / MS (found) |
|---|---|---|---|
| 1 | 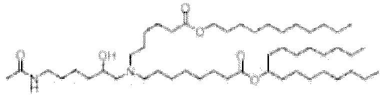 | Heptadecan-9-yl 8-((6-acetamido -2-hydroxyhexyl)(6-oxo-6-(undecyloxy)hexyl)amino)octanoate | [M + H]⁺ 823.75 / 823.75 |
| 2 | 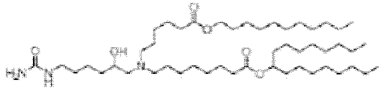 | Heptadecan-9-yl 8-((2-hydroxy-6-ureidohexyl)(6-oxo-6-(undecyloxy)hexyl)amino)octanoate | [M + H]⁺ 824.75 / 824.75 |
| 3 | 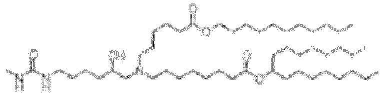 | Heptadecan-9-yl 8-((2- hydroxy-6-(3-methylureido)hexyl)(6-oxo-6-(undecyloxy)hexyl)amino)octanoate | [M + H]⁺ 838.76 / 838.76 |
| 4 | 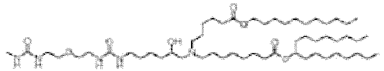 | Heptadecan-9-yl 17-hydroxy-3,11-dioxo-19-(6-oxo-6-(undecyloxy)hexyl-7-oxa-2,4,10,12,19-pentaazaheptacosan-27-oate | [M + H]⁺ 968.83 / 968.83 |
| 5 | 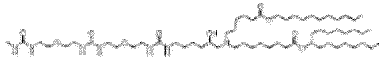 | Heptadecan-9-yl 25-hydroxy-3,11,19-trioxo-27-(6-oxo-6-(undecyloxy)hexyl)-7,15-dioxa-2,4,10,12,18,20,27-heptaazapentatriacontan-35-oate | [M + H]⁺ 1098.91 / 1098.91 |
| 6 | 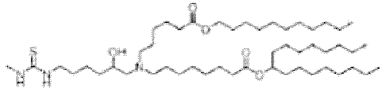 | Heptadecan-9-yl 8-((2-hydroxy-6-(3-methylthioureido)hexyl)(6-oxo-6-(undecyloxy)hexyl)amino)octanoate | [M + H]⁺ 854.74 / 854.74 |
| 7 | 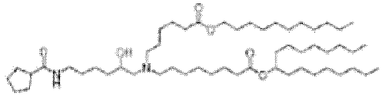 | Heptadecan-9-yl 8-((6-(cyclopentanecarboxamido)-2-hydroxyhexyl)(6-oxo-6-(undecyloxy)hexyl)amino)octanoate | [M + H]⁺ 877.80 / 877.80 |
| 8 | 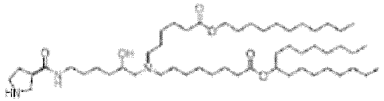 | Heptadecan-9-yl 8-((2-hydroxy-6-((R)-pyrrolidine-3-carboxamido)hexyl)(6-oxo-6-(undecyloxy)hexyl)amino)octanoate | [M + 2H]²⁺ 439.90 / 439.90 |
| 9 | 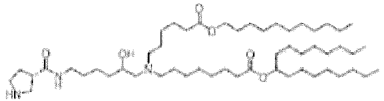 | Heptadecan-9-yl 8-((2-hydroxy-6-((S)-pyrrolidine-3-carboxamido)hexyl)(6-oxo-6-(undecyloxy)hexyl)amino)octanoate | [M + 2H]²⁺ 439.90 / 439.90 |
| 10 | 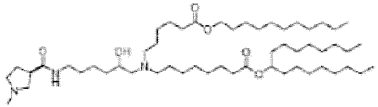 | Heptadecan-9-yl 8-((2-hydroxy-6-((R)-1-methylpyrrolidine-3-carboxamido)hexyl)(6-oxo-6-(undecyloxy)hexyl)amino)octanoate | [M + 2H]²⁺ 446.91 / 446.91 |
| 11 | 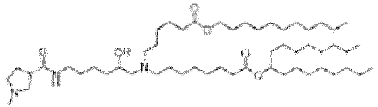 | Heptadecan-9-yl 8-((2-hydroxy-6-((S)-1-methylpyrrolidine-3-carboxamido)hexyl)(6-oxo-6-(undecyloxy)hexyl)amino)octanoate | [M + 2H]²⁺ 446.91 / 446.91 |
| 12 | 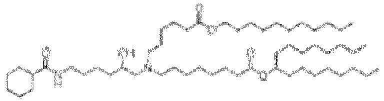 | Heptadecan-9-yl 8-((6-(cyclohexanecarboxamido)-2-hydroxyhexyl)(6-oxo-6-(undecyloxy)hexyl)amino)octanoate | [M + 2H]²⁺ 891.81 / 891.81 |
| 13 | 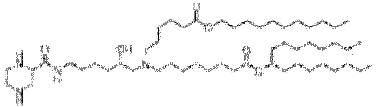 | Heptadecan-9-yl 8-((2-hydroxy-6-(piperazine-2-carboxamido)hexyl)(6-oxo-6-(undecyloxy)hexyl)amino)octanoate | [M + H]⁺ 893.80 / 893.80 |
| 14 | 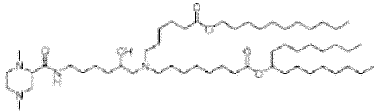 | Heptadecan-9-yl 8-((6-(1,4-dimethylpiperazine-2-carboxamido)-2-hydroxyhexyl)(6-oxo-6-(undecyloxy)hexyl)amino)octanoate | [M + H]⁺ 921.93 / 921.83 |
| 15 | 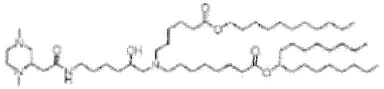 | Heptadecan-9-yl 8-((6-(2-(1,4-dimethylpiperazin-2-yl)acetamido)-2-hydroxyhexyl)(6-oxo-6-(undecyloxy)hexyl)amino)octanoate | [M + H]⁺ 935.85 / 935.85 |
| 16 | 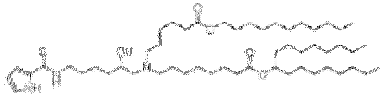 | Heptadecan-9-yl 8-((2-hydroxy-6-(1H-pyrrole-2-carboxamido)hexyl)(6-oxo-6-(undecyloxy)hexyl)amino)octanoate | [M + H]⁺ 874.76 / 874.76 |
| 17 | 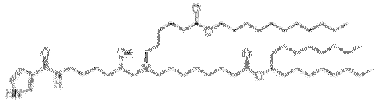 | Heptadecan-9-yl 8-((2-hydroxy-6-(1H-pyrrole-3-carboxamido)hexyl)(6-oxo-6-(undecyloxy)hexyl)amino)octanoate | [M + H]⁺ 874.76 / 874.76 |
| 18 | 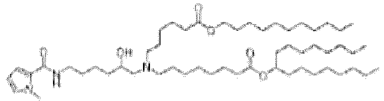 | Heptadecan-9-yl 8-((2-hydroxy-6-(1-methyl-1*H*-pyrrole-2-catboxamido)hexyl)(6-oxo-6-(undecyloxy)hexyl)amino)octanoate | [M + H]⁺ 888.78 / 888.78 |
| 19 | 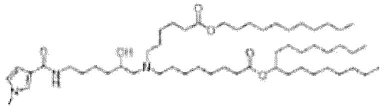 | Heptadecan-9-yl 8-((2-hydroxy-6-(1-methyl-1*H*-pyrrole-3-carboxamido)hexyl)(6-oxo-6-(undecyloxy)hexyl)amino)octanoate | [M + H]⁺ 888.78 / 888.78 |
| 20 | 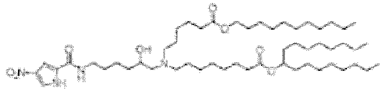 | Heptadecan-9-yl 8-((2-hydroxy-6-(4-nitro-1*H*-pyrrole-2-carboxamido)hexyl)(6-oxo-6-(undecyloxy)hexyl)amino)octanoate | [M + H]⁺ 919.75 / 919.75 |
| 21 | 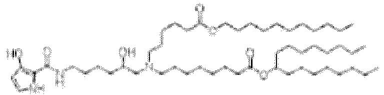 | Heptadecan-9-yl 8-((2-hydroxy-6-(3-hydroxy-1*H*-pyrrole-2-carboxamido)hexyl)(6-oxo-6-(undecyloxy)hexyl)amino)octanoate | [M + H]⁺ 890.76 / 890.76 |
| 22 | 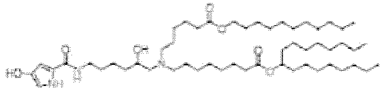 | Heptadecan-9-yl 8-((2-hydroxy-6-(4-hydroxy-1*H*-pyrrole -2-carboxamido)hexyl)(6-oxo-6-(undecyloxy)hexyl)amino)octanoate | [M + H]⁺ 890.76 / 890.76 |
| 23 | 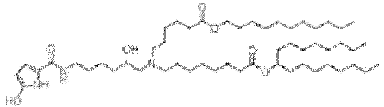 | Heptadecan-9-yl 8-((2-hydroxy-6-(5-hydroxy-1*H*-pyrrole-2-carboxamido)hexyl)(6-oxo-6-(undecyloxy)hexyl)amino)octanoate | [M + H]⁺ 890.76 / 890.76 |
| 24 | 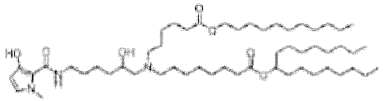 | Heptadecan-9-yl 8-((2-hydroxy-6-(3-hydroxy-1-methyl-1*H*-pyrrole-2-carboxamido)hexyl)(6-oxo-6-(undecyloxy)hexyl)amino)octanoate | [M + H]⁺ 904.77 / 904.77 |
| 25 | 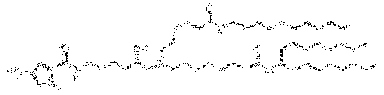 | Heptadecan-9-yl 8-((2-hydroxy-6-(4-hydroxy-1-methyl-1*H*-pyrrole-2-carboxamido)hexyl)(6-oxo-6-(undecyloxy)hexyl)amino)octanoate | [M + H]⁺ 904.77 / 904.77 |
| 26 | 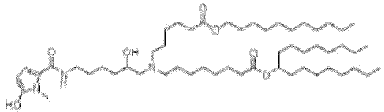 | Heptadecan-9-yl 8-((2-hydroxy-6-(5-hydroxy-1-methyl-1*H*-pyrrole-2-carboxamido)hexyl)(6-oxo-6-(undecyloxy)hexyl)amino)octanoate | [M + H]⁺ 904.77 / 904.77 |
| 27 | 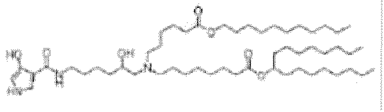 | Heptadecan-9-yl 8-((2-hydroxy-6-(4-hydroxy-1*H*-pyrrole-3-carboxamido)hexyl)(6-oxo-6-(undecyloxy)hexyl)amino)octanoate | [M + H]⁺ 890.76 / 890.76 |
| 28 | 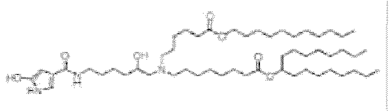 | Heptadecan-9-yl 8-((2- hydroxy-6-(5-hydroxy-1*H*-pyrrole-3-carboxamido)hexyl)(6-oxo-6-(undecyloxy)hexyl)amino)octanoate | [M + H]⁺ 890.76 / 890.76 |
| 29 | 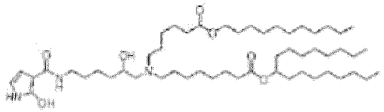 | Heptadecan-9-yl 8-((2-hydroxy-6-(2-hydroxy-1*H*-pyrrole-3-carboxamido)hexyl)(6-oxo-6-(undecyloxy)hexyl)amino)octanoate | [M + H]⁺ 890.76 / 890.76 |
| 30 | 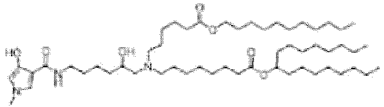 | Heptadecan-9-yl 8-((2-hydroxy-6-(4-hydroxy-1-methyl-1*H*-pyrrole-3-carboxamido)hexyl)(6-oxo-6-(undecyloxy)hexyl)amino)octanoate | [M + H]⁺ 904.77 / 904.77 |
| 31 | 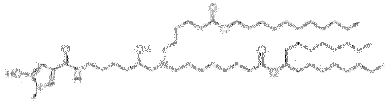 | Heptadecan-9-yl 8-((2-hydroxy-6-(5-hydroxy-1-methyl-1*H*-pyrrole-3-carboxamido)hexyl)(6-oxo-6-(undecyloxy)hexyl)amino)octanoate | [M + H]⁺ 904.77 / 904.77 |
| 32 | 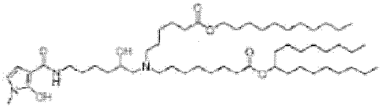 | Heptadecan-9-yl 8-((2-hydroxy-6-(2-hydroxy-1-methyl-1*H*-pyrrole-3-carboxamido)hexyl)(6-oxo-6-(undecyloxy)hexyl)amino)octanoate | [M + H]⁺ 904.77 / 904.77 |
| 33 | 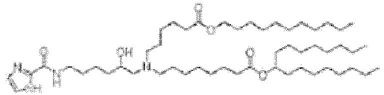 | Heptadecan-9-yl 8-((2-hydroxy-6-(1*H-*imidazole-2-carboxamido)hexyl)(6-oxo-6-(undecyloxy)hexyl)amino)octanoate | [M + H]⁺ 875.75 / 875.75 |
| 34 | 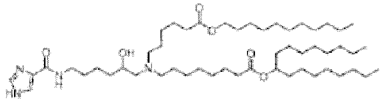 | Heptadecan-9-yl 8-((2-hydroxy-6-(1*H-*imidazole-4-carboxamido)hexyl)(6-oxo-6-(undecyloxy)hexyl)amino)octanoate | [M + H]⁺ 875.76 / 875.75 |
| 35 | 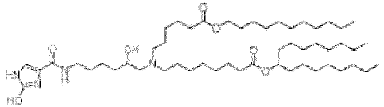 | Heptadecan-9-yl 8-((2-hydroxy-6-(2-hydroxy-1*H*-imidazole-4-carboxamido)hexyl)(6-oxo-6-(undecyloxy)hexyl)amino)octanoate | [M + H]⁺ 891.75 / 891.75 |
| 36 | 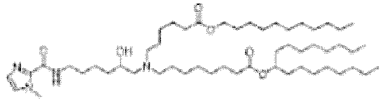 | Heptadecan-9-yl 8-((2-hydroxy-6-(1-methyl-1*H*-imidazole-2-carboxamido)hexyl)(6-oxo-6-(undecyloxy)hexyl)amino)octanoate | [M + H]⁺ 889.77 / 889.77 |
| 37 | 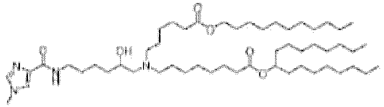 | Heptadecan-9-yl 8-((2-hydroxy-6-(1-methyl-1*H*-imidazole-4-carboxamido)hexyl)(6-oxo-6-(undecyloxy)hexyl)amino)octanoate | [M + H]⁺ 889.77 / 889,77 |
| 38 | 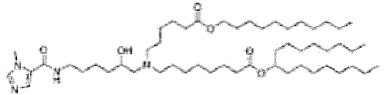 | Heptadecan-9-yl 8-((2-hydroxy-6-(1-methyl-1*H*-imidazole-5-carboxamido)hexyl)(6-oxo-6-(undecyloxy)hexyl)amino)octanoate | [M + H]⁺ 889.77 / 889.77 |
| 39 | 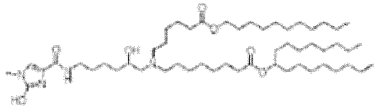 | Heptadecan-9-yl 8-((2-hydroxy-6-(2-hydroxy-1-methyl-1*H*-imidazole-4-carboxamido)hexyl)(6-oxo-6-(undecyloxy)hexyl)amino)octanoate | [M + H]⁺ 905.77 / 905.77 |
| 40 | 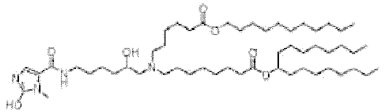 | Heptadecan-9-yl 8-((2-hydroxy-6-(2-hydroxy-1-methyl-1*H*-imidazole-5-carboxamido)hexyl)(6-oxo-6-(undecyloxy)hexyl)amino)octanoate | [M + H]⁺ 905.77 / 905.77 |
| 41 | 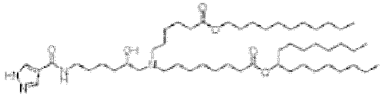 | Heptadecan-9-yl 8-((2-hydroxy-6-(1*H-*pyrazole-4-carboxamido)hexyl)(6-oxo-6-(undecyloxy)hexyl)amino)octanoate | [M + H]⁺ 875.76 / 875.76 |
| 42 | 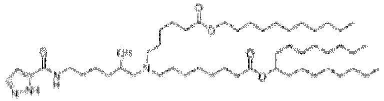 | Heptadecan-9-yl 8-((2-hydroxy-6-(1*H-*pyrazole-5-carboxamido)hexyl)(6-oxo-6-(undecyloxy)hexyl)amino)octanoate | [M + H]⁺ 875.76 / 875.76 |
| 43 | 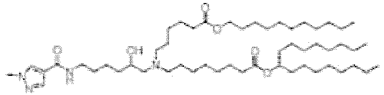 | Heptadecan-9-yl 8-((2-hydroxy-6-(1-methyl-1*H*-pyrazole-4-carboxamido)hexyl)(6-oxo-6-(undecyloxy)hexyl)amino)octanoate | [M + H]⁺ 889.77 / 889.77 |
| 44 | 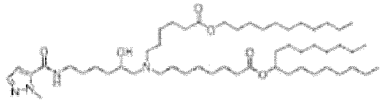 | Heptadecan-9-yl 8-((2-hydroxy-6-(1-methyl-1*H*-pyrazole-5-carboxamido)hexyl)(6-oxo-6-(undecyloxy)hexyl)amino)octanoate | [M + H]⁺ 889.77 / 889.77 |
| 45 | 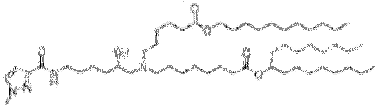 | Heptadecan-9-yl 8-((2-hydroxy-6-(1-methyl-1*H*-pyrazole-3-carboxamido)hexyl)(6-oxo-6-(undecyloxy)hexyl)amino)octanoate | [M + H]⁺ 889.77 / 889.77 |
| 46 | 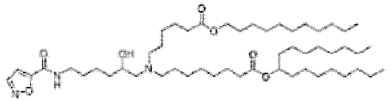 | Heptadecan-9-yl 8-((2-hydroxy-6-(isoxazole-5-carboxamido)hexyl)(6-oxo-6-(undecyloxy)hexyl)amino)octanoate | [M + H]⁺ 876.74 / 878.74 |
| 47 | 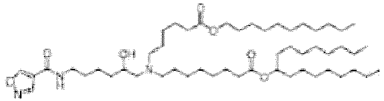 | Heptadecan-9-yl 8-((2-hydroxy-6-(isoxazole-4-carboxamido)hexyl)(6-oxo-6-(undecyloxy)hexyl)amino)octanoate | [M + H]⁺ 876.74 / 876.74 |
| 48 | 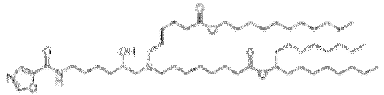 | Heptadecan-9-yl 8-((2-hydroxy-6-(oxazole-5-carboxamido)hexyl)(6-oxo-6-(undecyloxy)hexyl)amino)octanoate | [M + H]⁺ 876.74 / 876.74 |
| 49 | 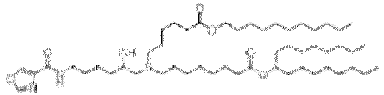 | Heptadecan-9-yl 8-((2-hydroxy-6-(oxazole-4-carboxamido)hexyl)(6-oxo-6-(undecyloxy)hexyl)amino)octanoate | [M + H]⁺ 876.74 / 876.74 |
| 50 | 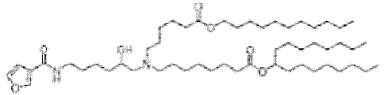 | Heptadecan-9-yl 8-((6-(furan-3-carboxamido)-2-hydroxyhexyl)(6-oxo-6-(undecyloxy)hexyl)amino)octanoate | [M + H]⁺ 875.75 / 875.75 |
| 51 | 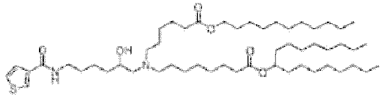 | Heptadecan-9-yl 8-((2-hydroxy-6-(thiophene-3-carboxamido)hexyl)(6-oxo-6-(undecyloxy)hexyl)amino)octanoate | [M + H]⁺ 891.72 / 891.72 |
| 52 | 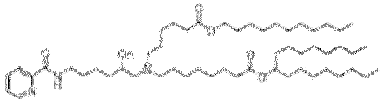 | Heptadecan-9-yl 8-((2-hydroxy-6-(picolinamido)hexyl)(6-oxo-6-(undecyloxy)hexyl)amino)octanoate | [M + H]⁺ 886.76 / 886.76 |
| 53 | 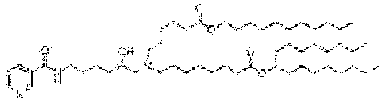 | Heptadecan-9-yl 8-((2-hydroxy-6-(nicotinamido)hexyl)(6-oxo-6-(undecyloxy)hexyl)amino)octanoate | [M + H]⁺ 886.76 / 886.76 |
| 54 | 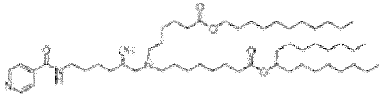 | Heptadecan-9-yl 8-((2-hydroxy-6-(isonicotinamido)hexyl)(6-oxo-6-(undecyloxy)hexyl)amino)octanoate | [M + H]⁺ 886.76 / 886.76 |
| 55 | 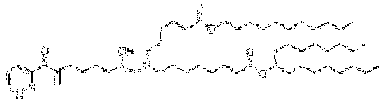 | Heptadecan-9-yl 8-((2-hydroxy-6-(pyridazine-3-carboxamido)heryl)(6-oxo-6-(undecyloxy)hexyl)amino)octanoate | [M + H]⁺ 887.76 / 887.76 |
| 56 | 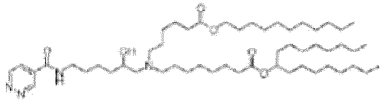 | Heptadecan-9-yl 8-((2-hydroxy-6-(pyridazine-4-carboxamido)heryl)(6-oxo-6-(undecyloxy)hexyl)amino)octanoate | [M + H]⁺ 887.76 / 887.76 |
| 57 | 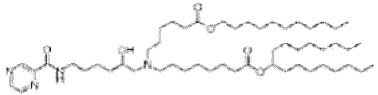 | Heptadecan-9-yl 8-((2-hydroxy-6-(pyrazine-2-carboxamido)hexyl)(6-oxo-6-(undecyloxy)hexyl)amino)octanoate | [M + H]⁺ 887.76 / 887.76 |
| 58 | 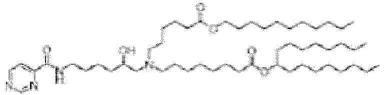 | Heptadecan-9-yl 8-((2-hydroxy-6-(pyrimidine-4-carboxamido)hexyl)(6-oxo-6-(undecyloxy)hexyl)amino)octanoate | [M + H]⁺ 887.76 / 887.76 |
| 59 | 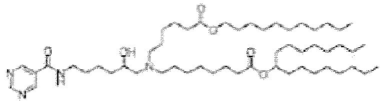 | Heptadecan-9-yl 8-((2-hydroxy-6-(pyrimidine-5-carboxamido)hexyl)(6-oxo-6-(undecyloxy)hexyl)amino)octanoate | [M + H]⁺ 887.76 / 887.76 |
| 60 | 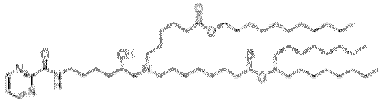 | Heptadecan-9-yl 8-((2-hydroxy-6-(pyrimidine-2-carboxamido)hexyl)(6-oxo-6-(undecyloxy)hexyl)amino)octanoate | [M + H]⁺ 887.76 / 887.76 |
| 61 | 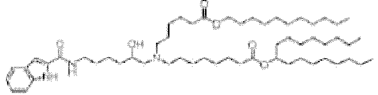 | Heptadecan-9-yl 8-((2-hydroxy-6-(1*H-*indole-2-carboxamido)hexyl)(6-oxo-6-(undecyloxy)hexyl)amino)octanoate | [M + H]⁺ 924.78 / 924.78 |
| 62 | 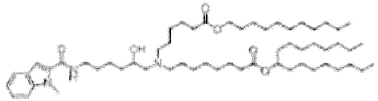 | Heptadecan-9-yl 8-((2-hydroxy-6-(1-methyl-1*H*-indole-2-carboxamido)hexyl)(6-oxo-6-(undecyloxy)hexyl)amino)octanoate | [M + H]⁺ 938.79 / 938.79 |
| 63 | 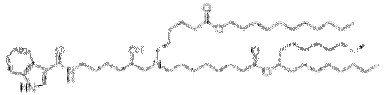 | Heptadecan-9-yl 8-((2-hydroxy-6-(1*H-*indole-3-carboxamido)hexyl)(6-oxo-6-(undecyloxy)hexyl)amino)octanoate | [M + H]⁺ 924.78 / 924.78 |
| 64 | 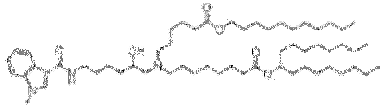 | Heptadecan-9-yl 8-((2-hydroxy-6-(1-methyl-1*H*-indole-3-carboxamido)hexyl)(6-oxo-6-(undecyloxy)hexyl)amino)octanoate | [M + H]⁺ 938.79 / 938.79 |
| 65 | 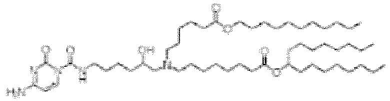 | Heptadecan-9-yl 8-((6-(4-amido-2-oxo-1,2-dihydropyrimidine-1-carboxamido)-2-hydroxyhexyl)(6-oxo-6-(undecyloxy)hexyl)amino)octanoate | [M + H]⁺ 918.76 / 918.76 |
| 66 | 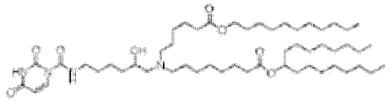 | Heptadecan-9-yl 8-((6-(2,4-dioxo-1,2,3,4-tetrahydropyrimidine-1-carboxamido)-2-hydroxyhexyl)(6-oxo-6-(undecyloxy)hexyl)amino)octanoate | [M + H]⁺ 919.75 / 919.75 |
| 67 | 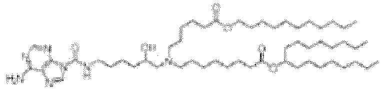 | Heptadecan-9-yl 8-((6-(6-amino-9*H-*purine-9-carboxamido)-2-hydroxyhexyl)(6-oxo-6-(undecyloxy)hexyl)amino)octanoate | [M + H]⁺ 942.77 / 942.77 |
| 68 | 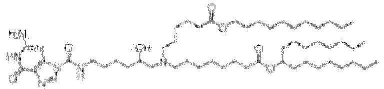 | Heptadecan-9-yl 8-((6-(2-amino-6-oxo-6,9-dihydro-1*H*-purine-9-carboxamido)-2-hydroxyhexyl)(6-oxo-6-(undecyloxy)hexyl)amino)octanoate | [M + H]⁺ 958.77 / 958.77 |
| 69 | 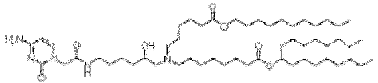 | Heptadecan-9-yl 8-((6-(2-(4-amino-2-oxopyrimidin-1(2*H*)-yl)acetamido)-2-hydroxyhexyl)(6-oxo-6-(undecyloxy)hexyl)amino)octanoate | [M + H]⁺ 932.78 / 932.78 |
| 70 | 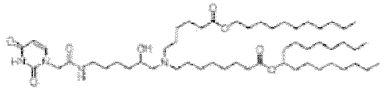 | Heptadecan-9-yl 8-((6-(2-(2,4-dioxo-3,4-dihydropyrimidin-1(2*H*)-yl)acetamido)-2-hydroxyhexyl)(6-oxo-6-(undecyloxy)hexyl)amino)octanoate | [M + H]⁺ 933.76 / 933.76 |
| 71 | 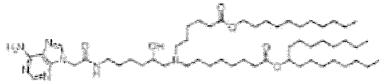 | Heptadecan-9-yl 8-((6-(2-(6-amino-9*H-*purin-9-yl)acetamido)-2-hydroxyhexyl)(6-oxo-6-(undecyloxy)hexyl)amino)octanoate | [M + H]⁺ 956.79 / 956.79 |
| 72 | 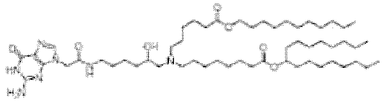 | Heptadecan-9-yl 8-((6-(2-(2-amino-6-oxo-1,6-dihydro-9*H*-purin-9-yl)acetamido)-2-hydroxyhexyl)(6-oxo-6-(undecyloxy)hexyl)amino)octanoate | [M + H]⁺ 972.78 / 972.78 |
| 73 | 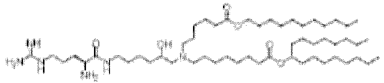 | Heptadecan-9-yl 8-((6-((*S*)-2-amino-5-guanidinopentanamido)-2-hydroxyhexyl)(6-oxo-6-(undecyloxy)hexyl)amino)octanoate | [M + H]⁺ 937.84 / 937.84 |
| 74 | 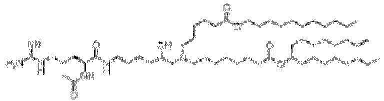 | Heptadecan-9-yl 8-((6-((*S*)-2-acetamido-5-guanidinopentanamido)-2-hydroxyhexyl)(6-oxo-6-(undecyloxy)hexyl)amino)octanoate | [M + H]⁺ 979.85 / 979.85 |
| 75 | 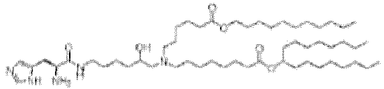 | Heptadecan-9-yl 8-((6-((S)-2-amino-3-(1*H*-imidazol-5-yl)propanamido)-2-hydroxyhexyl)(6-oxo-6-(undecyloxy)hexyl)amino)octanoate | [M + H]⁺ 918.80 / 918.80 |
| 76 | 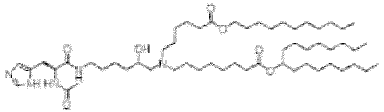 | Heptadecan-9-yl 8-((6-((S)-2-acetamido-3-(1*H*-imidazol-5-yl)propanamido)-2-hydroxyhexyl)(6-oxo-6-(undecyloxy)hexyl)amino)octanoate | [M + H]⁺ 960.81 / 960.81 |
| 77 | 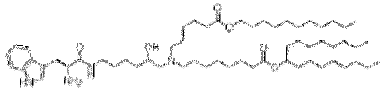 | Heptadecan-9-yl 8-((6-((*S*)-2-amino-3-(1*H*-indol-3-yl)propanamido)-2-hydroxyhexyl)(6-oxo-6-(undecyloxy)hexyl)amino)octanoate | [M + H]⁺ 967.82 / 967.82 |
| 78 | 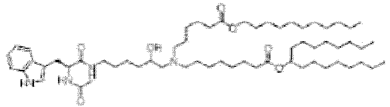 | Heptadecan-9-yl 8-((6-((*S*)-2-acetamido-3-(1*H*-indol-3-yl)propanamido)-2-hydroxyhexyl)(6-oxo-6-(undecyloxy)hexyl)amino)octanoate | [M + H]⁺ 1009.83 / 1009.83 |
| 79 | 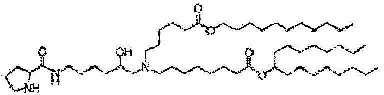 | Heptadecan-9-yl 8-((2-hydroxy-6-((*S*)-pyrrolidine-2-carboxamido)hexyl)(6-oxo-6-(undecyloxy)hexyl)amino)octanoate | [M + H]⁺ 878.79 / 878.79 |
| 80 | 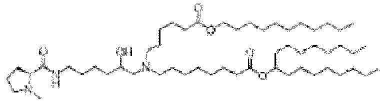 | Heptadecan-9-yl 8-((2-hydroxy-6-((*S*)-1-methylpyrrolidine-2-carboxamido)hexyl)(6-oxo-6-(undecyloxy)hexyl)amino)octanoate | [M + H]⁺ 892.81 / 892.81 |
| 81 | 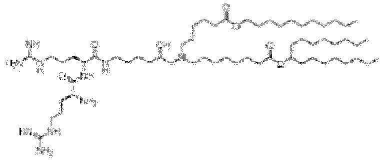 | Heptadecan-9-yl (6*S*,9*S*)-1,6-diamino-9-(3-guanidinopropyl)-16-hydroxy-1-imino-7,10-dioxo-18-(6-oxo-6-(undecyloxy)hexyl)-2,8,11,18-tetraazahexacosan-26-oate | [M + H]⁺ 1093.94 / 1093.94 |
| 82 | 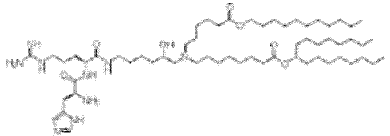 | Heptadecan-9-yl 8-((6-((*S*)-2-((*S*)-2-amino-3-(1*H*-imidazol-5-yl)propanamido)-5-guanidinopentanamido)-2-hydroxyhexyl)(6-oxo-6-(undecyloxy)hexyl)amino)octanoate | [M + H]⁺ 1074.90 / 1074.90 |
| 83 | 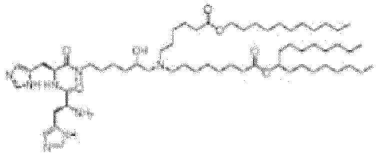 | Heptadecan-9-yl 8-((6-((*S*)-2-((*S*)-2-amino-3-(1*H*-imidazol-5-yl)propanamido)-3-(1H-imidazol-5-yl)propanamido)-2-hydroxyhexyl)(6-oxo-6-(undecyloxy)hexyl)amino)octanoate | [M + H]⁺ 1055.86 / 1055.86 |
| 84 | 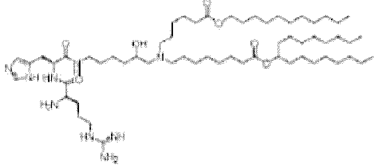 | Heptadecan-9-yl (6*R*,9*S*)-9-((1*H-*imidazol-5-yl)methyl)-1,6-diamino-16-hydroxy-1-imino-7,10-dioxo-18-(6-oxo-6-(undecyloxy)hexyl)-2,8,11,18-tetraazahexacosan-26-oate | [M + H]⁺ 1074.90 / 1074.90 |
| 85 | 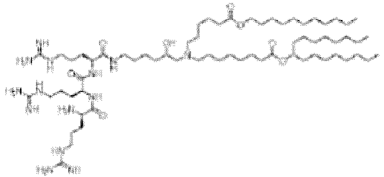 | Heptadecan-9-yl (6*R,*9*S,*12*S*)-1,6-diamino-9,12-bis(3-guanidinopropyl)-19-hydroxy-1-imino-7,10,13-trioxo-21-(6-oxo-6-(undecyloxy)hexyl)-2,8,11,14,21-pentaazanonacosan-29-oate | [M + H]⁺ 1250.04 / 1250.04 |
| 86 | 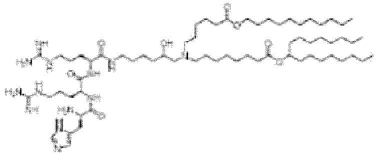 | Heptadecan-9-yl (6*S*,9*S*)-1-amino-6-((*R*)-2-amino-3-(1*H*-imidazol-5-yl)propanimdo)-9-(3-guanidinopropyl)-16-hydroxy-1-imino-7,10-dioxo-18-(6-oxo-6-(undecyloxy)hexyl)-2,8,11,18-tetraazahexacosan-26-oate | [M + H]⁺ 1231.00 / 1231.00 |
| 87 | 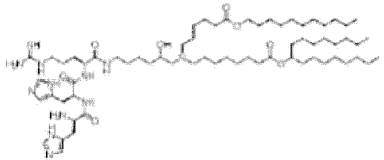 | Heptadecan-9-yl (2*R*,5*S,*8*S*)*-*5-((1*H-*imidazol-5-yl)methyl)-2-amino-8-(3-guanidinopropyl)-15-hydroxy-1-(1*H-*imidazol-5-yl)-3,6,9-trioxo-17-(6-oxo-6-(undecyloxy)hexyl)-4,7,10,17-tetraazapentacosan-25-oate | [M + H]⁺ 1211.96 / 1211.96 |
| 88 | 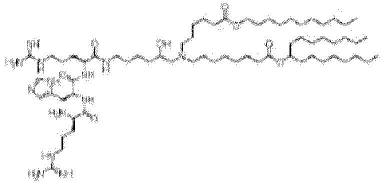 | Heptadecan-9-yl (6*R*,9*S*,12*S*)-9-((1*H-*imidazol-5-yl)methyl)-1,6-diamino-12-(3-guanidinopropyl)-19-hydroxy-1-imino-7,10,13-trioxo-21-(6-oxo-6-(undecyloxy)hexyl)-2,8,11,14,21-pentaazanonacosan-29-oate | [M + H]⁺ 1231.00 / 1231.00 |
| 89 | 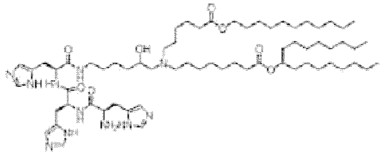 | Heptadecan-9-yl (2*R*,5*S*,8*S*)-5,8-bis-(1*H-*imidazol-5-yl)methyl)-2-amino-15-hydroxy-1-(1*H*-imidazol-5-yl)-3,6,9-trioxo-17-(6-oxo-6-(undecyloxy)hexyl)-4,7,10,17-tetraazapentacosan-25-oate | [M + H]⁺ 1192.92 / 1192.92 |
| 90 | 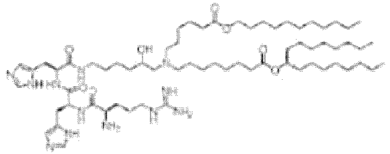 | Heptadecan-9-yl (6*R*,9*S*,12*S*)-9,12-bis(1*H*-imidazol-5-yl)methyl)-1,6-diamino-19-hydroxy-1-imino-7,10,13-trioxo-21-(6-oxo-6-(undecyloxy)hexyl)-2,8,11,14,21-pentaazanonacosan-29-oate | [M + H]⁺ 1211.96 / 1211.96 |
| 91 | 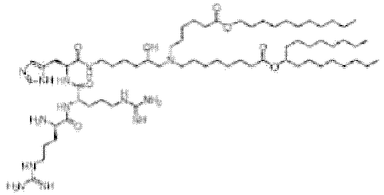 | Heptadecan-9-yl (6*R*,9*S*,12*S*)-12-((1*H-*imidazol-5-yl)methyl)-1,6-diamino-9-(3-guanidinopropyl)-19-hydroxy-1-imino-7,10,13-trioxo-21-(6-oxo-6-(undecyloxy)hexyl)-2,8,11,14,21-pentaazanonacosan-29-oate | [M + H]⁺ 1231.00 / 1231.00 |
| 92 | 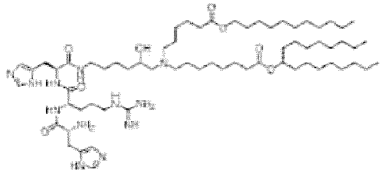 | Heptadecan-9-yl (6*S*,9*S*)-9-(1*H*-imidazol-5-yl)methyl)-1-amino-6((*R*)-2-amino-3-(1*H*-imidazol-5-yl)propanamido-16-hydroxy-1-imino-7,10-dioxo-18(6-oxo-6-(undecyloxy)hexyl)-2,8,11,18-tetraazahexacosan-26-oate | [M + H]⁺ 1211.96 / 1211.96 |
| 93 | 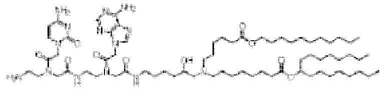 | Heptadecan-9-yl 1-(4-amino-2-oxopyrimidin-1(2*H*)-yl)-9-(2-6-amino-9*H*-purin-9-yl)acetyl)-3-(2-aminoethyl)17-hydroxy-2,5,11-trioxo-19-(6-oxo-6-(undecyloxy)hexyl)-3,6,9,12,19-pentaazaheptacosan-27-oate | [M + H]⁺ 1307.95 / 1307.95 |
| 94 | 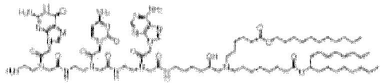 | Heptadecan-9-yl 9-(2-(4-amino-2-oxopyrimidin-1(2*H*)-yl)acetyl)-1-(2-amino-6-oxo-1,.6-dihydro-9*H*-purin-9-yl)-15-(2-(6-amino-9*H*-purin-9-yl)acetyl)-3-(2-aminoethyl)-23-hydroxy-2,5,11,17-tetraoxo-25-(6-oxo-6-(undecyloxy)hexyl)-3,6,9,12,15,18,25-heptaazatritriacontan-23-oate | [M + H]⁺ 1599.06 / 1599.06 |
| 95 | 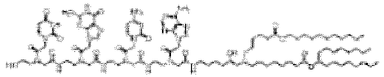 | Heptadecan-9-yl 15-(2-(4-amino-2-oxopyrimidin-1(2*H*)-yl)acetyl)-9-(2-(2-amino-6-oxo-1,6-dihydro-9H-purin-9-yl)acetyl)-21-(2-(6-amino-9*H*-purin-9-yl)acetyl)-3-(2-aminoethyl)-1-(2,4-dioxo-3,4-dihydropyrimidin-1(2*H*)-yl)-29-hydroxy-2,5,11,17,23-pentaoxo-31-(6-oxo-6-(undecyloxy)hexyl)-3,6,9,12,15,18,21,24,31-nanaazanonatriacontann-39-oate | [M + H]⁺ 1851.15 / 1851.15 |
| 96 | 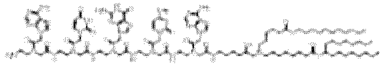 | Heptadecan-9-yl 21-(2-(4-amino-2-oxopyrimidin-1(2*H*)-yl)acetyl)-15-(2-(2-amino-6-oxo-1,6-dihydro-9H-purin-9-yl)acetyl)-1-(6-amino-9*H*-purin-9-yl)acetyl)-27-(2-(6-amino-9*H*-purin-9-yl)acetyl-3-(2-aminoethyl)-9-(2-(2,4-dioxo-3,4-dihydropyrimidin-1(2*H*)-yl)acetyl)-35-hydroxy-2,5,11,17,23,29-hexaoxo-37-(6-oxo-6-(undecyloxy)hexyl)-3,6,9,12,15,18,21,24,27,30,37-undecaazapentatetracontan-45-oate | [M + H]⁺ 2126.26 / 2126.26 |
| 97 | 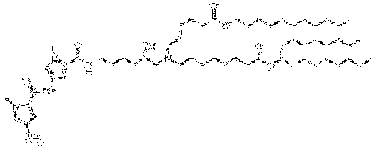 | Heptadecan-9-yl 8-((6-(4-(4-amino-1-methyl-1*H*-pyrrole-2-carboxamido)-1-methyl-1*H*-pyrrole-2-carboxamido)-2-hydoroxyhexyl)(6-oxo-6-(undecyloxy)hexyl)amino)octanoate | [M + H]⁺ 1025.84 / 1025.84 |
| 98 | 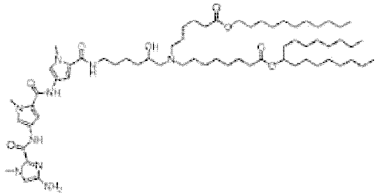 | Heptadecan-9-yl 8-((6-(4-(4-(4-amino-1-methyl-1*H*-imidazole-2-carboxamido)-1-methyl-1*H*-pyrrole-2-carboxamido)-1-methyl-1*H*-pyrrole-2-carboxamido)-2-hydoroxyhexyl)(6-oxo-6-(undecyloxy)hexyl)amino)octanoate | [M + H]⁺ 1148.88 / 1148.88 |
| 99 | 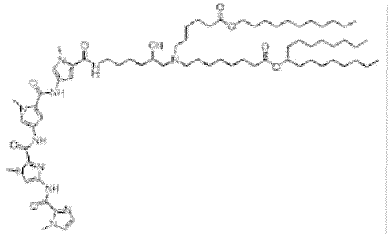 | Heptadecan-9-yl 8-((2-hydroxy-6-(1-methyl-4-(1-methyl-4-(1-methyl-4-(1-methyl-1*H*-imidazole-2-carboxamido)-1H-imidazole-2- carboxamido)-1H-pyrrole-2-carboxamido)-1*H*-pyrrole-2-carboxamido)hexyl)(6-oxo-6-(undecyloxy)hexyl)amino)octanoate | [M + H]⁺ 1256.91 / 1256.91 |
| 100 | 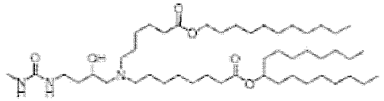 | Heptadecan-9-yl 8-((2-hydroxy-4-(3-methylureido)butyl)(6-oxo-6-(undecyloxy)hexyl)amino)octanoate | [M + H]⁺ 810.73 / 810.73 |
| 101 | 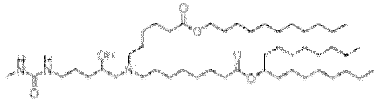 | Heptadecan-9-yl 8-((2-hydroxy-5-(3-methylureido)pentyl)(6-oxo-6-(undecyloxy)hexyl)amino)octanoate | [M + H]⁺ 824.75 / 824.75 |
| 102 | 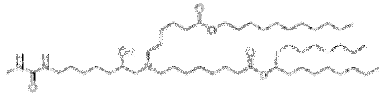 | Heptadecan-9-yl 8-((2-hydroxy-7-(3-methylureido)heptyl)(6-oxo-6-(undecyloxy)hexyl)amino)octanoate | [M + H]⁺ 852.78 / 852.78 |
| 103 | 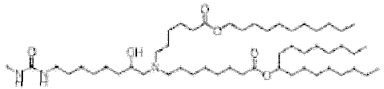 | Heptadecan-9-yl 8-((2-hydroxy-8-(3-methylureido)octyl)(6-oxo-6-(undecyloxy)hexyl)amino)octanoate | [M + H]⁺ 866.79 / 866.79 |
| 104 | 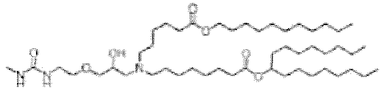 | Heptadecan-9-yl 9-hydroxy-3-oxo-11-(6-oxo-6-(undecyloxy)hexyl)-7-oxa-2,4,11-triazanonadecan-19-oate | [M + H]⁺ 840.74 / 840.74 |
| 105 | 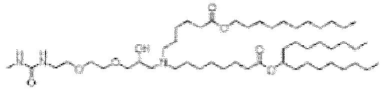 | Heptadecan-9-yl 12-hydroxy-3-oxo-14-(6-oxo-6-(undecyloxy)hexyl)-7,10-dioxa-2,4,14-triazadocosan-22-oate | [M + H]⁺ 884.77 / 884.77 |
| 106 | 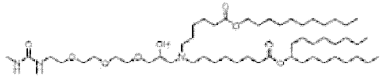 | Heptadecan-9-yl 15-hydroxy-3-oxo-17-(6-oxo-6-(undecyloxy)hexyl)-7,10,13-trioxa-2,4,17-triazapentacosan-25-oate | [M + H]⁺ 928.79 / 928.79 |
| 107 | 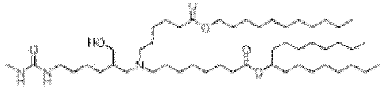 | Heptadecan-9-yl 8-((2-(hydroxymethyl)-6-(3-methylureido)hexyl)(6-oxo-6-(undecyloxy)hexyl)amino)octanoate | [M + H]⁺ 852.78 / 852.78 |
| 108 | 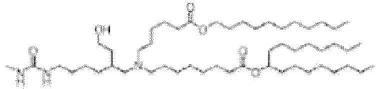 | Heptadecan-9-yl 8-((2-(2-hydroxyethyl)-6-(3-methylureido)hexyl)(6-oxo-6-(undecyloxy)hexyl)amino)octanoate | [M + H]⁺ 866.79 / 866.79 |
| 109 | 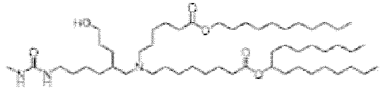 | Heptadecan-9-yl 8-((2-(3-hydroxypropyl)-6-(3-methylureido)hexyl)(6-oxo-6-(undecyloxy)hexyl)amino)octanoate | [M + H]⁺ 880.81 / 880.81 |
| 110 | 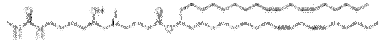 | (6Z,9Z,28Z,31Z)-heptatriaconta-6,9,28,31-tetraen-19-yl 4-((2-hydroxy-6-(3-methylureido)hexyl)(methyl)amino)butan oate | [M + H]⁺ 800.72 / 800.72 |
| 111 | 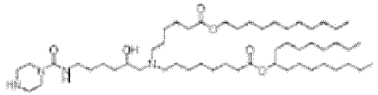 | Heptadecan-9-yl 8-((2-hydroxy-6-(piperazine-1-carboxamido)hexyl)(6-oxo-6-(undecyloxy)hexyl)amino)octanoate | [M + H]⁺ 893.80 / 893.80 |
| 112 | 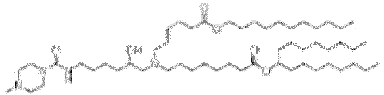 | Heptadecan-9-yl 8-((2-hydroxy-6-(4-methylpiperazine-1-carboxamido)hexyl)(6-oxo-6-(undecyloxy)hexyl)amino)octanoate | [M + H]⁺ 907.82 / 907.82 |
| 113 | 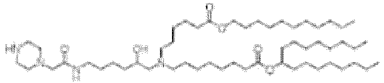 | Heptadecan-9-yl 8-((2-hydroxy-6-(2-piperazin-1-yl)acetamido)hexyl)(6-oxo-6-(undecyloxy)hexyl)amino)octanoate | [M + H]⁺ 907.82 / 907.82 |
| 114 | 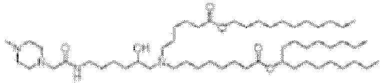 | Heptadecan-9-yl 8-((2-hydroxy-6-(2-(4-methylpiperazin-1-yl)acetamido)hexyl)(6-oxo-6-(undecyloxy)hexyl)amino)octanoate | [M + H]⁺ 921.83 / 921.83 |
| 115 | 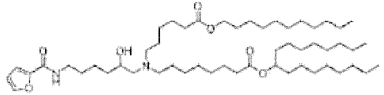 | Heptadecan-9-yl 8-((6-(furan-2-carboxamido)-2-hydroxyhexyl)(6-oxo-6-(undecyloxy)hexyl)amino)octanoate | [M + H]⁺ 875.74 / 875.74 |
| 116 | 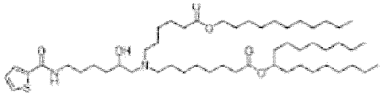 | Heptadecan-9-yl 8-((2-hydroxy-6-(thiophene-2-carboxamido)hexyl)(6-oxo-6-(undecyloxy)hexyl)amino)octanoate | [M + H]⁺ 891.72 / 891.72 |
| 117 | 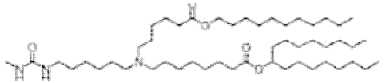 | Heptadecan-9-yl 8-((6-(3-methylureido)hexyl)(6-oxo-6-(undecyloxy)hexyl)amino)octanoate | [M + H]⁺ 822.77 / 822.77 |
| 118 | 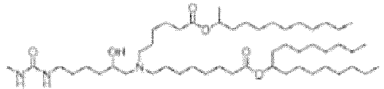 | Heptadecan-9-yl 8-((6-(dodecan-2-yloxy)-6-oxohexyl)(2-hydroxy-6-(3-methylureido)hexyl)amino)octanoate | [M + H]⁺ 852.78 / 852.78 |
| 119 | 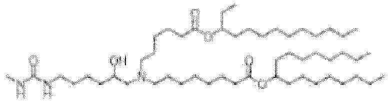 | Heptadecan-9-yl 8-((2-hydroxy)-6-(3-methylureido)hexyl)(6-oxo-6-(tridecan-3-yloxy)hexyl)amino)octanoate | [M + H]⁺ 883.79 / 866.79 |
| 120 | 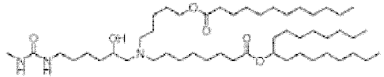 | 5-((8-(Heptadecan-9-yloxy) 8-oxooctyl)(2-hydroxy-6-(3-methylureido)hexyl)amino)pentyl dodecanoate | [M + H]⁺ 838.76 / 838.76 |
| 121 | 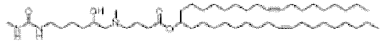 | (9Z,28Z)-heptatriaconta-9,28-dien-19-yl 4-((2-hydroxy-6-(3-methylureido)hexyl)(methyl)amino)butan oate | [M + H]⁺ 804.76 / 804.76 |
| 122 | 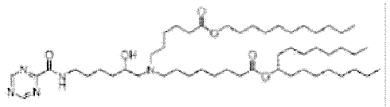 | Heptadecan-9-yl 8-((2-hydroxy-6-(1,3,5-triazine-2-carboxamido)hexyl(6-oxo-6-(undecyloxy)hexyl)amino)octanoate | [M + H]⁺ 888.75 / 888.75 |
| 123 | 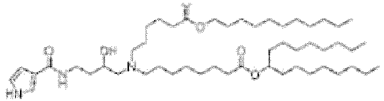 | Heptadecan-9-yl 8-((2-hydroxy-4-(1*H-*pyrrole-3-carboxamido)butyl)(6-oxo-6-(undecyloxy)hexyl)amino)octanoate | [M + H]⁺ 846.73 / 846.73 |
| 124 | 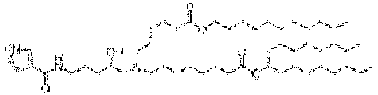 | Heptadecan-9-yl 8-((2-hydroxy-5-(1*H-*pyrrole-3-carboxamido)pentyl)(6-oxo-6-(undecyloxy)hexyl)amino)octanoate | [M + H]⁺ 860.75 / 860.74 |
| 125 | 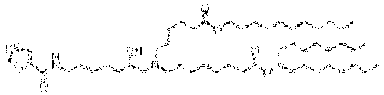 | Heptadecan-9-yl 8-((2-hydroxy-7-(1*H-*pyrrole-3-carboxamido)heptyl)(6-oxo-6-(undecyloxy)hexyl)amino)octanoate | [M + H]⁺ 888.78 / 888.77 |
| 126 | 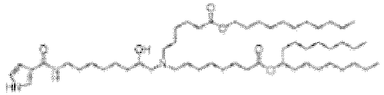 | Heptadecan-9-yl 8-((2-hydroxy-8-(1*H-*pyrrole-3-carboxamido)octyl)(6-oxo-6-(undecyloxy)hexyl)amino)octanoate | [M + H]⁺ 902.79 / 902.79 |
| 127 | 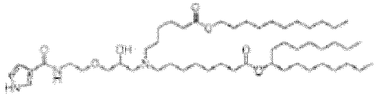 | Heptadecan-9-yl 8-((3-(2-(1*H*-pyrrole-3-carboxamido)ethoxy)-2-hydroxypropyl)(6-oxo-6-(undecyloxy)hexyl)amino)octanoate | [M + H]⁺ 876.74 / 876.74 |
| 128 | 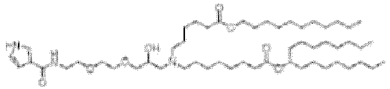 | Heptadecan-9-yl 10-hydroxy-1-oxo-12-(6-oxo-6-(undecyloxy)hexyl)-1-(1*H*-pyrrol-3-yl)-5,8-dioxa-2,12-diazacosan-20-oate | [M + H]⁺ 920.77 / 920.76 |
| 129 | 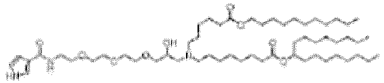 | Heptadecan-9-yl 13-hydroxy-1-oxo-15-(6-oxo-6-(undecyloxy)hexyl)-1-(1*H*-pyrrol-3-yl)-5,8,11-trioxa-2,15-diazatricosan-23-oate | [M + H]⁺ 964.79 / 964.79 |
| 130 | 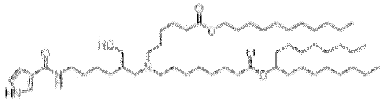 | Heptadecan-9-yl 8-((2-hydroxymethyl)-6-(1*H*-pyrrole-3-carboxamido)hexyl)(6-oxo-6-(undecyloxy)hexyl)amino)octanoate | [M + H]⁺ 888.78 / 888.77 |
| 131 | 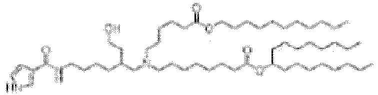 | Heptadecan-9-yl 8-((2-(2-hydroxyethyl)-6-(1*H*-pyrrole-3-carboxamido)hexyl)(6-oxo-6-(undecyloxy)hexyl)amino)octanoate | [M + H]⁺ 902.79 / 902.79 |
| 132 | 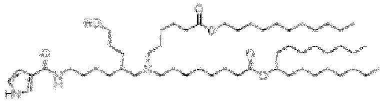 | Heptadecan-9-yl 8-((2-(3-hydroxypropyl)-6-(1*H*-pyrrole-3-carboxamido)hexyl)(6-oxo-6-(undecyloxy)hexyl)amino)octanoate | [M + H]⁺ 916.81 / 916.81 |
| 133 | 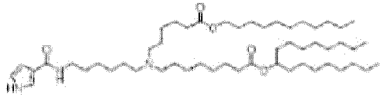 | Heptadecan-9-yl 8-((6-(1*H*-pyrrole-3-carboxamido)hexyl)(6-oxo-6-(undecyloxy)hexyl)amino)octanoate | [M + H]⁺ 858.77 / 858.76 |
| 134 | 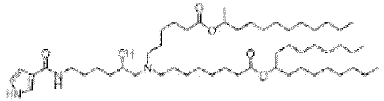 | Heptadecan-9-yl 8-((6-(dodecan-2-yloxy)-6-oxohexyl)(2-hydroxy-6-(1*H*-pyrrole-3-carboxamido)hexyl)amino)octanoate | [M + H]⁺ 888.78 / 888.77 |
| 135 | 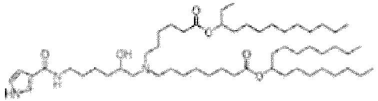 | Heptadecan-9-yl 8-((2-hydroxy-6-(1*H-*pyrrole-3-carboxamido)hexyl)(6-oxo-6-(tridecan-3-yloxy)hexyl)amino)octanoate | [M + H]⁺ 902.79 / 902.79 |
| 136 | 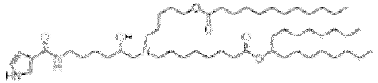 | 5-((8-Heptadecan-9-yloxy)-8-oxooctyl)(2-hydroxy-6-(1*H*-pyrrole-3-carboxamido)hexyl)amino)pentyl dodecanoate | [M + H]⁺ 874.76 / 874.76 |
| 137 | 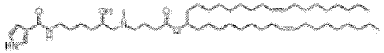 | (9Z,28Z)-heptatriaconta-9,28-dien-19-yl 4-((2-(hydroxy-6-(1*H*-pyrrole-3-carboxamido)hexyl)(methyl)amino)butan oate | [M + H]⁺ 840.76 / 840.75 |
| 138 | 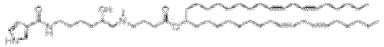 | (6Z,9Z,28Z,31Z)-heptatriaconta-6,9,28,31-tetraen-19-yl 4-((2-hydroxy-6-(1*H*-pyrrole-3-carboxamido)hexyl)(methyl)amino)butan oate | [M + H]⁺ 836.72 / 836.72 |
| 139 | 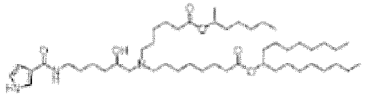 | Heptadecan-9-yl 8-((6-(heptan-2-yloxy)-6-oxohexyl)(2-hydroxy-6-(1*H*-pyrrole-3-carboxamido)hexyl)amino)octanoate | [M + H]⁺ 818.70 / 818.69 |
| 140 | 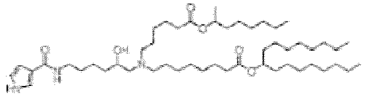 | Heptadecan-9-yl 8-((2-hydroxy-6-(1*H-*pyrrole-3-carboxamido)hexyl)(6-(octan-2-yloxy)-6-oxohexyl)amino)octanoate | [M + H]⁺ 832.71 / 832.71 |
| 141 | 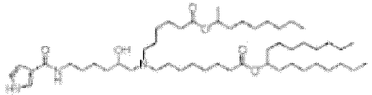 | Heptadecan-9-yl 8-((2-hydroxy-6-(1*H-*pyrrole-3-carboxamido)hexyl)(6-(nonan-2-yloxy)-6-oxohexyl)amino)octanoate | [M + H]⁺ 846.73 / 846.73 |
| 142 | 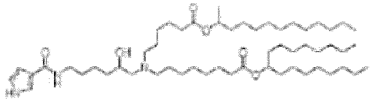 | Heptadecan-9-yl 8-((2-hydroxy-6-(1*H-*pyrrole-3-carboxamido)hexyl)(6-oxo-6-(tridecan-2-yloxy)hexyl)amino)octanoate | [M + H]⁺ 902.79 / 902.79 |
| 143 | 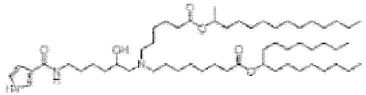 | Heptadecan-9-yl 8-((2-hydroxy-6-(1*H-*pyrrole-3-carboxamido)hexyl)(6-oxo-6-(tetradecan-2-yloxy)hexyl)amino)octanoate | [M + H]⁺ 916.81 / 916.80 |
| 144 | 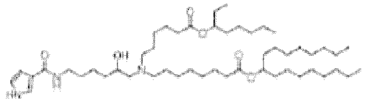 | Heptadecan-9-yl 8-((2-hydroxy-6-(1*H-*pyrrole-3-carboxamido)hexyl)(6-(octan-3-yloxy)-6-oxohexyl)amino)octanoate | [M + H]⁺ 832.71 / 832.71 |
| 145 | 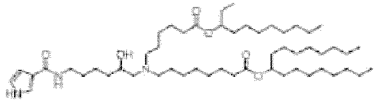 | Heptadecan-9-yl 8-((2-hydroxy-6-(1*H-*pyrrole-3-carboxamido)hexyl)(6-oxo-6-(undecan-3-yloxy)hexyl)amino)octanoate | [M + H]⁺ 874.76 / 874.76 |
| 146 | 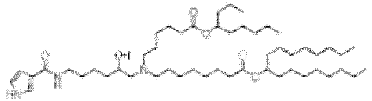 | Heptadecan-9-yl 8-((2-hydroxy-6-(1*H-*pyrrole-3-carboxamido)hexyl)(6-(nonan-4-yloxy)-6-oxohexyl)amino)octanoate | [M + H]⁺ 846.73 / 846.73 |
| 147 | 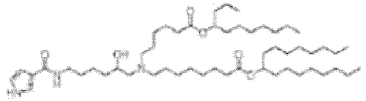 | Heptadecan-9-yl 8-((2-hydroxy-6-(1*H-*pyrrole-3-carboxamido)hexyl)(6-oxo-6-(undecan-4-yloxy)hexyl)amino)octanoate | [M + H]⁺ 874.76 / 874.76 |
| 148 | 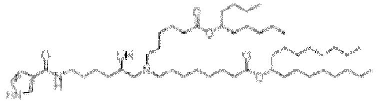 | Heptadecan-9-yl 8-((6-(decan-5-yloxy)-6-oxohexyl)(2-hydroxy-6-(1*H*-pyrrole-3-carboxamido)hexyl)amino)octanoate | [M + H]⁺ 860.75 / 860.74 |
| 149 | 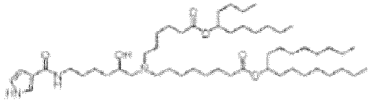 | Heptadecan-9-yl 8-((2-hydroxy-6-(1*H-*pyrrole-3-carboxamido)hexyl)(6-oxo-6-(undecan-5-yloxy)hexyl)amino)octanoate | [M + H]⁺ 874.76 / 874.76 |
| 150 | 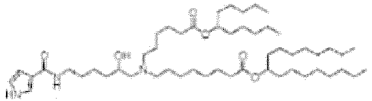 | Heptadecan-9-yl 8-((2-hydroxy-6-(1*H-*pyrrole-3-carboxamido)hexyl)(6-oxo-6-(undecan-6-yloxy)hexyl)amino)octanoate | [M + H]⁺ 874.76 / 874.76 |
| 151 | 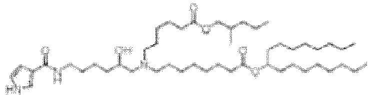 | Heptadecan-9-yl 8-((2-hydroxy-6-(1*H-*pyrrole-3-carboxamido)hexyl)(6-((2-methylpentyl)oxy)-6-oxohexyl)amino)octanoate | [M + H]⁺ 804.68 / 804.68 |
| 152 | 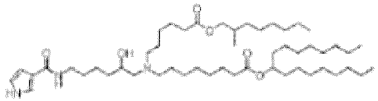 | Heptadecan-9-yl 8-((2-hydroxy-6-(1*H-*pyrrole-3-carboxamido)hexyl)(6-((2-methyloctyl)oxy)-6-oxohexyl)amino)octanoate | [M + H]⁺ 846.73 / 846.73 |
| 153 | 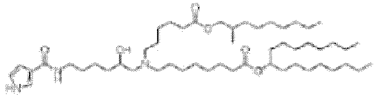 | Heptadecan-9-yl 8-((2-hydroxy-6-(1*H-*pyrrole-3-carboxamido)hexyl)(6-((2-methylnonyl)oxy)-6-oxohexyl)amino)octanoate | [M + H]⁺ 860.75 / 860.74 |
| 154 | 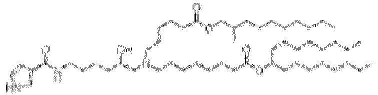 | Heptadecan-9-yl 8-((2-hydroxy-6-(1*H-*pyrrole-3-carboxamido)hexyl)(6-((2-methyldecyl)oxy)-6-oxohexyl)amino)octanoate | [M + H]⁺ 874.76 / 874.76 |
| 155 | 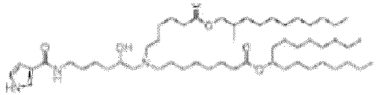 | Heptadecan-9-yl 8-((2-hydroxy-6-(1*H-*pyrrole-3-carboxamido)hexyl)(6-((2-methylundecyl)oxy)-6-oxohexyl)amino)octanoate | [M + H]⁺ 888.78 / 888.77 |
| 156 | 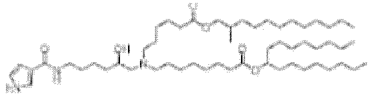 | Heptadecan-9-yl 8-((2-hydroxy-6-(1*H-*pyrrole-3-carboxamido)hexyl)(6-((2-methyldodecyl)oxy)-6-oxohexyl)amino)octanoate | [M + H]⁺ 902.79 / 902.79 |
| 157 | 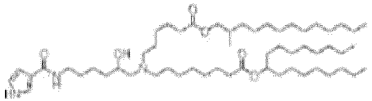 | Heptadecan-9-yl 8-((2-hydroxy-6-(1*H-*pyrrole-3-carboxamido)hexyl)(6-((2-methyltridecyl)oxy)-6-oxohexyl)amino)octanoate | [M + H]⁺ 916.81 / 916.80 |
| 158 | 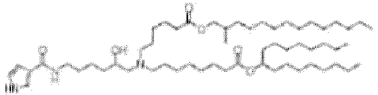 | Heptadecan-9-yl 8-((2-hydroxy-6-(1*H-*pyrrole-3-carboxamido)hexyl)(6-((2-methyltetradecyl)oxy)-6-oxohexyl)amino)octanoate | [M + H]⁺ 930.82 / 930.83 |
| 159 | 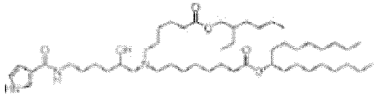 | Heptadecan-9-yl 8-((6-((2-ethylhexyl)oxy)-6-oxohexyl)(2-hydroxy-6-(1*H*-pyrrole-3-carboxamido)hexyl)amino)octanoate | [M + H]⁺ 832.71 / 832.72 |
| 160 | 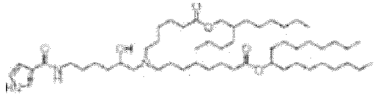 | Heptadecan-9-yl 8-((6-((2-butyloctyl)oxy)-6-oxohexyl)(2-hydroxy-6-(1*H*-pyrrole-3-carboxamido)hexyl)(amino)octanoate | [M + H]⁺ 888.78 / 868.77 |
| 161 | 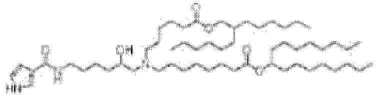 | Heptadecan-9-yl 8-((6-((2-hexyloctyl)oxy)-6-oxohexyl)(2-hydroxy-6-(1*H*-pyrrole-3-carboxamido)hexyl)amino)octanoate | [M + H]⁺ 916.81 / 916.80 |
| 162 | 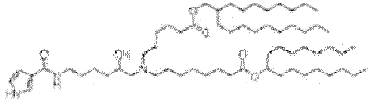 | Heptadecan-9-yl 8-((2-hydroxy-6-(1*H-*pyrrole-3-carboxamido)hexyl)(6-((2-octyldodecyl)oxy)-6-oxohexyl)amino)octanoate | [M + H]⁺ 1000.90 / 1000.90 |
| 163 | 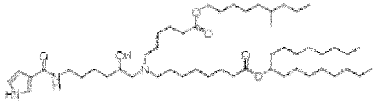 | Heptadecan-9-yl 8-((2-hydroxy-6-(1*H-*pyrrole-3-carboxamido)hexyl)(6-((6-methylnonyl)oxy)-6-oxohexyl)amino)octanoate | [M + H]⁺ 860.75 / 860.74 |
| 164 | 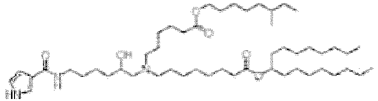 | Heptadecan-9-yl 8-((2-hydroxy-6-(1*H-*pyrrole-3-carboxamido)hexyl)(6-((6-methyloctyl)oxy)-6-oxohexyl)amino)octanoate | [M + H]⁺ 846.73 / 846.73 |
| 165 | 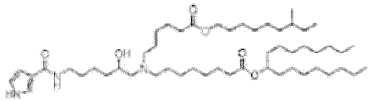 | Heptadecan-9-yl 8-((2-hydroxy-6-(1*H-*pyrrole-3-carboxamido)hexyl)(6-((7-methylnonyl)oxy)-6-oxohexyl)amino)octanoate | [M + H]⁺ 860.75 / 860.74 |
| 166 | 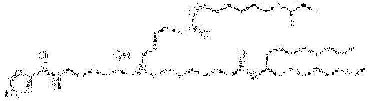 | Heptadecan-9-yl 8-((2-hydroxy-6-(1*H-*pyrrole-3-carboxamido)hexyl)(6-((8-methyldecyl)oxy)-6-oxohexyl)amino)octanoate | [M + H]⁺ 874.76 / 874.76 |
| 167 | 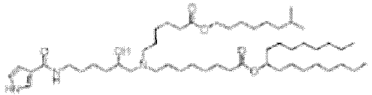 | Heptadecan-9-yl 8-((2-hydroxy-6-(1*H-*pyrrole-3-carboxamido)hexyl)(6-((7-methyloctyl)oxy)-6-oxohexyl)amino)octanoate | [M + H]⁺ 846.73 / 846.73 |
| 168 | 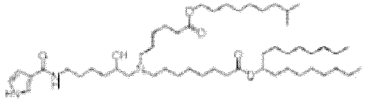 | Heptadecan-9-yl 8-((2-hydroxy-6-(1*H-*pyrrole-3-carboxamido)hexyl)(6-((8-methylnonyl)oxy)-6-oxohexyl)amino)octanoate | [M + H]⁺ 860.75 / 860.74 |
| 169 | 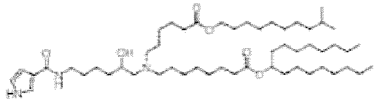 | Heptadecan-9-yl 8-((2-hydroxy-6-(1*H-*pyrrole-3-carboxamido)hexyl)(6-((9-methyldecyl)oxy)-6-oxohexyl)amino)octanoate | [M + H]⁺ 874.76 / 874.76 |
| 170 | 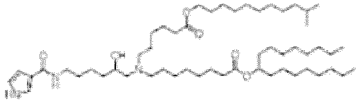 | Heptadecan-9-yl 8-((2-hydroxy-6-(1*H-*pyrrole-3-carboxamido)hexyl)(6-((10-methylundecyl)oxy)-6-oxohexyl)amino)octanoate | [M + H]⁺ 888.78 / 888.77 |
| 171 | 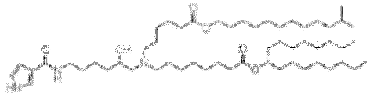 | Heptadecan-9-yl 8-((2-hydroxy-6-(1*H-*pyrrole-3-carboxamido)hexyl)(6-((11-methyldodecyl)oxy)-6-oxohexyl)amino)octanoate | [M + H]⁺ 902.79 / 902.79 |
| 172 | 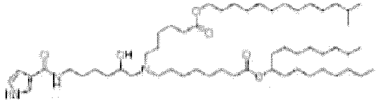 | Heptadecan-9-yl 8-((2-hydroxy-6-(1*H-*pyrrole-3-carboxamido)hexyl)(6-((12-methyltridecyl)oxy)-6-oxohexyl)amino)octanoate | [M + H]⁺ 916.81 / 916.80 |
| 173 | 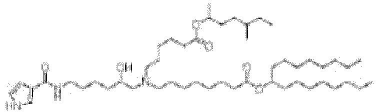 | Heptadecan-9-yl 8-((2-hydroxy-6-(1*H-*pyrrole-3-carboxamido)hexyl)(6-((5-methylheptan-2-yl)oxy)-6-oxohexyl)amino)octanoate | [M + H]⁺ 832.71 / 832.71 |
| 174 | 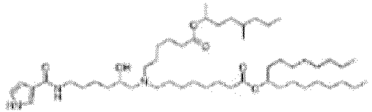 | Heptadecan-9-yl 8-((2-hydroxy-6-(1*H-*pyrrole-3-carboxamido)hexyl)(6-((5-methyloctan-2-yl)oxy)-6-oxohexyl)amino)octanoate | [M + H]⁺ 846.73 / 846.73 |
| 175 | 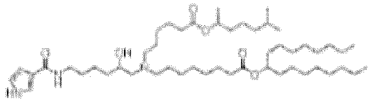 | Heptadecan-9-yl 8-((2-hydroxy-6-(1*H-*pyrrole-3-carboxamido)hexyl)(6-((6-methylheptan-2-yl)oxy)-6-oxohexyl)amino)octanoate | [M + H]⁺ 832.71 / 832.71 |
| 176 | 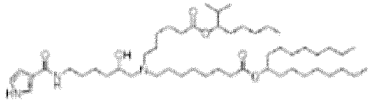 | Heptadecan-9-yl 8-((2-hydroxy-6-(1*H-*pyrrole-3-carboxamido)hexyl)(6-((2-methyloctan-3-yl)oxy)-6-oxohexyl)amino)octanoate | [M + H]⁺ 846.73 / 846.73 |
| 177 | 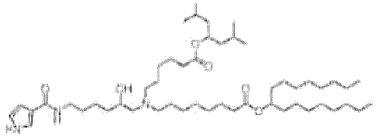 | Heptadecan-9-yl 8-((6-((2,6-dimethylheptan-4-yl)oxy)-6-oxohexyl)(2-hydroxy-6-(1*H*-pyrrole-3-carboxamido)hexyl)amino)octanoate | [M + H]⁺ 846.73 / 846.73 |
| 178 | 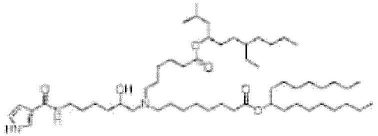 | Heptadecan-9-yl 8-((6-((7-ethyl-2-methylundecan-4-yl)oxy)-6-oxohexyl)(2-hydroxy-6-(1*H*-pyrrole-3-carboxamido)hexyl)amino)octanoate | [M + H]⁺ 916.81 / 916.80 |

### Example 27. Production of mRNA-LNP

Compounds 1-178, either manufactured according to Examples 1 to 29 or purchased commercially (e.g., SM-102 (Broadpharm, USA; patent document 3 [PCT/US2016/052352 (BENENARO, K. E.; KUMARASINGHE, E. S.; CORNEBISE, M.), 2016.09.16]), ALC-0315 (Broadpharm, USA; patent document 4 [PCT/US2016/029572 (TAM, Y.; HOPE, M. J.; WEISSMAN, D.; PARDI, N.) 2016.04.27]), DLin-MC3-DMA (MC3; Broadpharm, USA; patent document 5[US2010/0324120 A1 (JIANXIN, C. et al. 2010.12.23.]) etc.), phospholipids(e.g., DSPC; Sigma-Aldrich, USA), cholesterol (e.g., cholesterol; Sigma-Aldrich, USA), and PEGylated ligids (e.g., ALC-0159 (Broadpharm, USA; patent document 6[PCT/US2015/034496 (ANSELL, S. M.; DU, X) 2015.06.05]), DMG-PEG (DMG- PEG2000; Sigma-Aldrich, USA) etc.) was dissolved in organic layer (ethanol). Dilute mRNA (e.g., firefly luciferase mRNA (fLuc mRNA), enhanced green fluorescent protein mRNA (EGFP mRNA), SARS-CoV-2 spike mRNA, TriLink Biotechnologies, USA, etc.) 5-100 µg in 5-50 mM aqueous sodium acetate solution or 5-50 mM aqueous sodium citrate solution 0.2-2.0 mL toprepare the aqueous phase.

mRNA- LNPs were produced by mixing an organic phase (ethanol), containing ionizable lipids, phospholipids, cholesterol, and PEGylated lipids, with an aqueous phase (5-50 mM sodium acetate or sodium citrate aqueous solution) containing mRNA. The mixing was performed at an average flow rate of 25-250 µL/10 s using a microfluidic mixer (NanoAssemblr^{®} Spark, Precision Nanosystems, Canada). The constituent lipid compounds were dissolved in ethanol at a molar ratio of (20-60):(0-25):(30-60):(0-5) for ionizable lipids: phospholipids: cholesterol: pegylated lipids, with the total molar ratio summing to 100. LNPs were then prepared by mixing the organic phase and the aqueous phase such that the weight ratio of ionizable lipids to mRNA was (4-30):1. To remove ethanol from the mRNA-LNPs and to adjust the pH of the LNP aqueous solution to a level similar to the physiological pH (~7.4), dialysis was performed using a dialysis cassette (Slide-A-Lyzer^{™} Dialysis Cassette, 10K MWCO, ThermoFisher, USA) against phosphate-buffered saline (PBS, pH 7.4, Invitrogen, USA) for 12-18 hours with multiple buffer exchanges.

Hereinafter, the LNP composition will be referred to according to the type of ionizable lipid used in the production of mRNA-LNP.

The numbers indicated in the drawings or tables correspond to the compound numbers described in the Examples used in the production of the mRNA-LNP. For example, the number '16' shown in fig, 3 refers to an mRNA-LNP produced using compound 16, which will be referred to as 'composition 16'.

### [Experimental Example]

### Experimental Example 1. Measuring pKₐ of mRNA-LNP

The apparent pKₐ of the mRNA-LNPs prepared according to Example 27 was determined by analysis using 6-(p-toluidino)-2-naphthalenesulfonic acid sodium salt (TNS; Sigma-Aldrich, USA).

TNS, an anionic compound, generally does not exhibit fluorescence in aqueous solution. However, as the pH decreases, the proportion of positively charged ionizable lipids increases, allowing electrostatic interactions with TNS. As a result, TNS migrates into the hydrophobic domain (lipophilic) and exhibits progressively stronger fluorescence. When the pH of the solution increases and the ionizable lipids become neutralized, TNS can no longer engage in electrostatic interactions. Consequently, TNS loses its lipophilic character, exits the hydrophobic domain, and its fluorescence is quenched. Based on this principle, the pKₐ of LNPs can be measured by plotting the change in TNS fluorescence intensity in response to variations in the surrounding pH as an S-shaped curve, and calculating the logarithmic value of the inflection point. LNPs with a pKₐ typically ranging from 6.0-7.0 exhibit near-neutral surface chargein plasma (pH ~ 7.4) upon intravenous injection, thereby reducing nonspecific interactions with body tissues and increasing delivery efficiency to hepatocytes. Once internalized into cells, the acidic environment of the endosome (pH 5.5) causes the LNPs to become highly positive charged, which facilitates endosome escape of the encapsulated active substances, such as nucleic acids, proteins, or synthetic drugs, thereby enhancing in vivo delivery efficiency. (Non-patent literature 1 [Sabnis, S. et al. Mol. Ther. 2018, 26, 1509], non-patent document 2[Jayaraman, M.et al. Angew. Chem. Int. Ed. 2012, 51, 8529]).

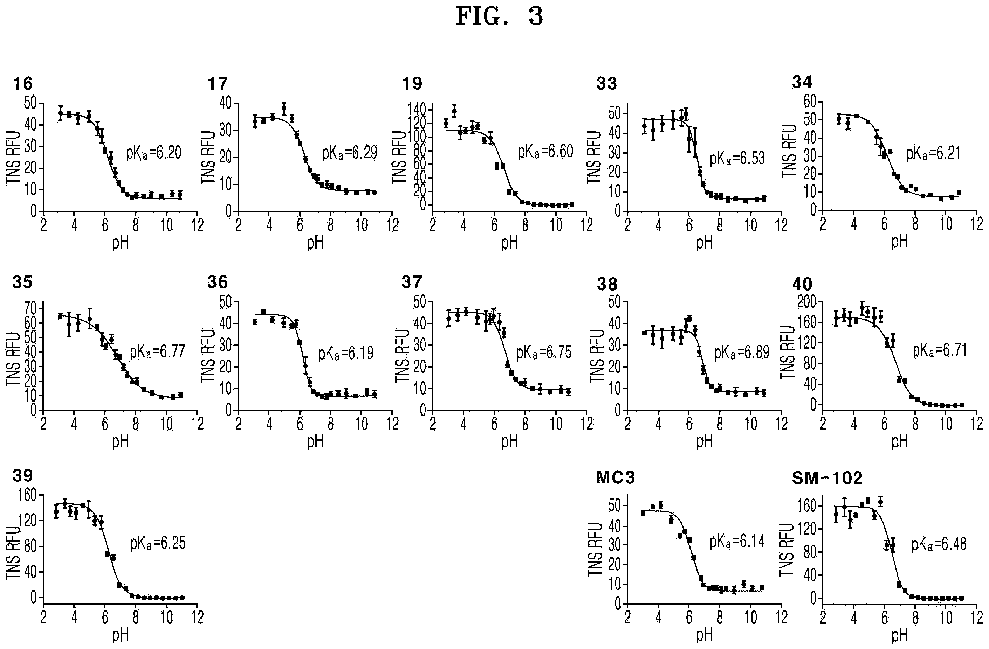

Specifically, a master buffer was prepared containing 10 mM sodium phosphate, 10 mM sodium borate, 10 mM sodium citrate, and 150 mM sodium chloride (NaCl). The pH of this aqueous solution was then adjusted over a range from pH 2.5 to 12 using 1 M sodium hydroxide (NaOH) and 1 M hydrochloric acid (HCl). Then, TNS standard stock solutions at concentrations of 150 µM or 300 µM were prepared by dissolving TNS in DMSO. LNPs were prepared at mRNA concentrations of 0.02-0.08 mg/mL in either 1× PBS buffer or 20 mM Tris (tris(hydroxymethyl)aminomethane) buffer containing 8% sucrose. Solutions adjusted to each pH were dispensed into three wells (n = 3) of a black-bottomed 96-well plate, with 90-94 µL added per well. Subsequently, 3.26-4.00 µL of LNPs and 2 µL of the TNS solution were added to each well. After gently mixing the contents of each well, the fluorescence intensity was measured using a multiplate reader (Cary Eclipse, Agilent Technologies, USA) at an excitation wavelength of λₑₓ 330 nm and an emission wavelength of λₑₘ 435 nm. The measured results are shown in FIG. 3.

FIG. 3 is a graph illustrating the results of measuring the apparent pKₐ values of mRNA-LNPs produced according to Example 27 using a TNS binding assay.

Specifically, composition 16, composition 17, composition 19, and composition 33 to 40 were selected from among the mRNA-LNPs prepared in Example 27, and their pKₐ values were measured using TNS binding assay. Composition 16, 17, 19, and 33 to 40 were prepared under their respective optimal formulation conditions, which were based on known conditions disclosed in technical reports provided bythe institutions that developed the corresponding ionizable lipids.

The pKₐ values of the mRNA-LNPs, along with their corresponding formulation conditions, are shown in table 2 below.

Referring to FIG. 3 and Table 2, the pKₐ values of the mRNA-LNPs produced according to the Example were all found to be within the range of 6.0 to 7.0, suggesting that the in vivo drug delivery efficiency is suitable.

In addition, it was confirmed that these values were similar to the pKₐ values of mRNA-LNPs prepared using commercially available ionizable lipids such as SM-102 and MC3

**[Table 2]**

| Ionizable lipid | N/P ratio | Ionizable/helper/choL./ PEG | size (nm) | | Zeta ( mV) | EE (%) | P*K*ₐ |
|---|---|---|---|---|---|---|---|
| | | | Number | PDI | | | |
| SM-102 | *5.67:1 | *50:10:38.5:1.5 | 101 | 0.140 | -2.99 | 94 | 6.48 |
| MC3 | *4.68:1 | *50:10:38.5:1.5 | 93 | 0.174 | -2.65 | 95 | 6.14 |
| 16 | 3.5:1 | 50:10:38.5:1.5 | 132 | 0.101 | -7.87 | 91 | 6.20 |
| 17 | 3.5:1 | 50:10:38.5:1.5 | 151 | 0.100 | -6.80 | 94 | 6.29 |
| 18 | 3.5:1 | 50:9.5:37.5:3 | 125 | 0.145 | -1.04 | 88 | - |
| 19 | 3.5:1 | 50:9.5:37.5:3 | 185 | 0.193 | -5.68 | 93 | 6.60 |
| 21 | 7:1 | 50:10:38.5:1.5 | 112 | 0.155 | -4.55 | 92 | - |
| 24 | 3.5:1 | 37.5:15.5:45.5:1.5 | 137 | 0.114 | -7.89 | 90 | - |
| 33 | 7:1 | 37.5:15:44.5:3 | 113 | 0.145 | -8.94 | 90 | 6.53 |
| 34 | 7:1 | 37.5:15:44.5:3 | 117 | 0.143 | -1.50 | 94 | 6.21 |
| 35 | 11:1 | 50:10:38.5:1.5 | 123 | 0.040 | -3.90 | 90 | 6.77 |
| 36 | 11:1 | 37.5:15:44.5:3 | 112 | 0.073 | -3.19 | 87 | 6.19 |
| 37 | 11:1 | 37.5:15:44.5:3 | 115 | 0.154 | -3.30 | 89 | 6.75 |
| 38 | 11:1 | 37.5:15:44.5:3 | 142 | 0.132 | -4.28 | 88 | 6.89 |
| 39 | 7:1 | 37.5:15:44.5:3 | 183 | 0.106 | -2.36 | 90 | 6.25 |
| 40 | 11:1 | 50:9.5:37.5:3 | 107 | 0.154 | -1.73 | 92 | 6.71 |

### Experimental Example 2. Measurement of the size, polydispersity, and surface charge of mRNA-LNP

Each mRNA-LNPs produced in Example 27 was diluted with PBS to a final mRNA concentration of 1 µg/mL. The size, polydispersity index (PDI), and surface charge of the mRNA-LNPs were then measured using a Malvern Zetasizer Nano ZS90 (Malvern Instruments, UK).

For particles intended for systemic administration, such as via blood vessels, the particle size must be less than 200 nm in order to pass through the endothelial capillaries of the liver. In addition, the size distribution is considered uniform only when the PDI is 0.3 or less, which enables consistent and efficient therapeutic effects (Non-patent document 3 [Danaei, M.et al). Pharmaceutics 2018, 10, 57]).

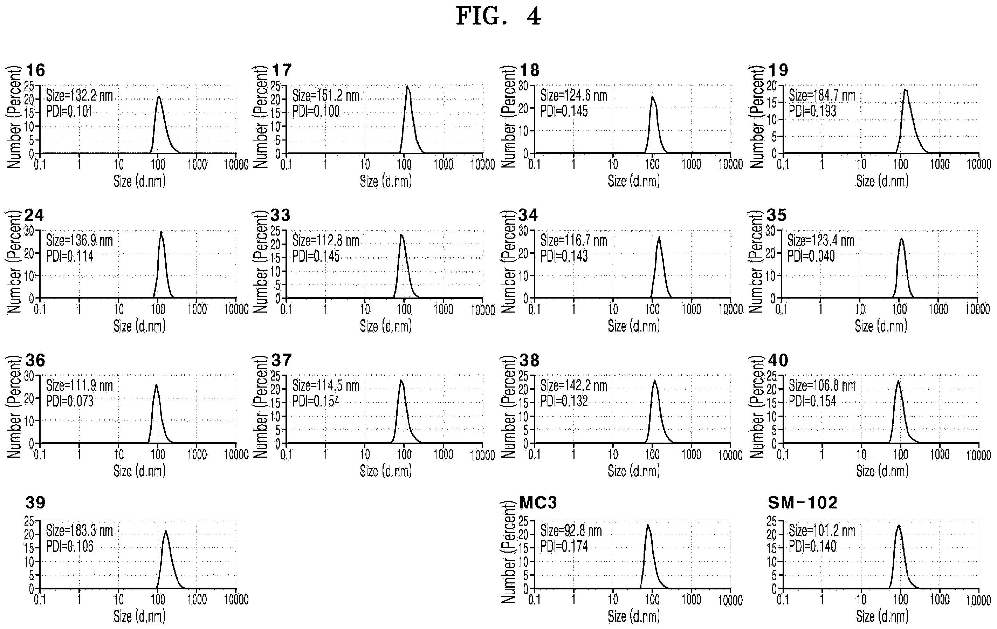

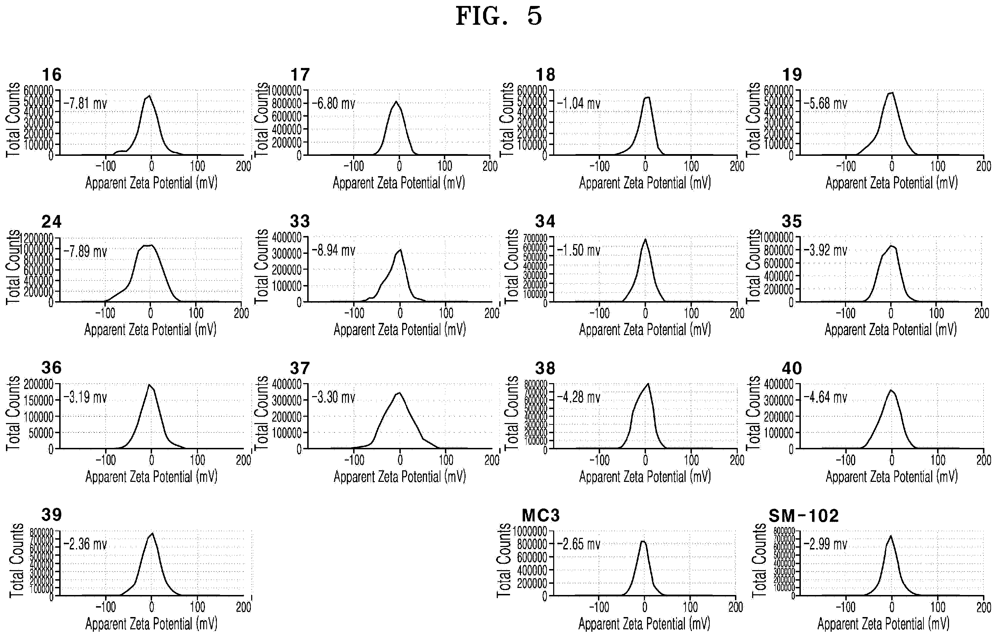

FIG. 4 is a graph illustrating the measured hydrodynamic diameter and PDI of the mRNA-LNPs prepared according to Example 27. FIG. 5 is a graph illustrating the measured surface charge of the mRNA-LNPs prepared according to Example 27. The measurement results are summarized in Table 2 above.

Referring to FIGS. 4, 5, and Table 2, the particle sizes of all measured mRNA-LNPs were within the appropriate range of 200 nm or less, and the PDI values were 0.2 or less, indicating h high uniformity.

### Experimental Example 3. Measurement of mRNA encapsulation efficiency

The mRNA encapsulation efficiency (EE, %) of LNPs containing mRNA was measured using the Quant-IT^{™} RiboGreens RNA assay kit (Invitrogen, USA).

LNPs preparedin Example 27 were diluted to a final volume of 50 µL using either 1X TE (Tris-EDTA) buffer or 1X TE buffer containing 2% Triton-X 100, such that the final concentration of mRNA was 20-30 ng/mL. The diluted samples were added to each well of a 96-well plate. Group A (without Triton X-100) was supplemented with 50 µL of 1×TE buffer, while Group B (with Triton X-100) was supplemented with 50 µL of 1×TE buffer containg 2% Triton-X 100. To release the encapsulated mRNA, the LNPs were treated with Triton-X 100 and incubated at 37°C for 10 minutes. Subsequently, 100 µL of ribogreen reagent was added to each well. The fluorescence intensities of Group A and Group B were measured using a multi-plate reader (λₑₓ 480 nm, λₑₘ 520 nm), and mRNA encapsulation efficiency (%) was calculated using Equation 1 below. The encapsulation efficiency for each LNP was determined as the average of two independent measurements. The results are summarized in Table 2 above.

As shown in Table 2, the mRNA encapsulation efficiency of the LNPs was approximately 90%, confirming that mRNA was encapsulated with relatively high efficiency. mRNA encapsulation efficiency (%) = [(Group B fluorescence intensity - Group A fluorescence intensity)/(Group B fluorescence intensity)] X 100

### Experimental Example 4. Evaluation of protein expression efficiency of EGFP mRNA-LNP administered to cells

LNPs encapsulating EGFP mRNA were prepared using various ionizable lipids according to the method described in Example 27, administered to HeLa cells (ATCC, USA) to compare *in vitro* protein expression efficiency over time. This evaluation was intended to reflect factors such as translation efficiency, endosomal escape, and mRNA stability. As a controls, EGFP mRNA-LNPs formulated with the ionizable lipid MC3 or SM-102 were used.

In the LNP formulations, cholesterol was used as the structural lipid, DMG-PEG was used as the PEG-lipid, and DSPC was used as the phospholipid. Additionally, for fluorescence microscopic tracking of the LNPs, 1 mol% of the total phospholipid content was substituted with sulfo-cyanine 5.5-DSPE.

Specifically, 18 hours prior to mRNA-LNPs administration, HeLa cells were seeded at a density of 1×10⁵ cells per well in an 8-well plate and cultured at 37°C in a humidified atmosphere containing 5% CO₂ using DMEM (Gibco, USA) supplemented with FBS and antibiotics. The concentration of the administered mRNA-LNPs was adjusted to 100 ng per well based on mRNA contend and applied to the cells. At 5, 10, 15, and 30 minutes, and at 1, 3 , 6 , 24 , and 48 hours after treatment, the culture medium was removed, and the cells were fixed with 4% paraformaldehyde(PFA) for 15 minutes. Fluorescence images were then acquired using a fluorescence microscope.

Experimental Example 5. Evaluation of protein expression efficiency of fLuc mRNA-LNPs administered via intramuscular (I.M.) injection and intravenous (I.V.) injection.

All animal experiments described below were conducted with prior approval from the Institutional Animal Care and Use Committee (IACUC) of the Korea Resecarch Institute of Bioscience and Biotechnology (KRIBB), following ethical review.

fLuc mRNA-LNPs formulated using various ionizable lipids were prepared according to the method described in Example 27 and administered in vivo via intramuscular (I.M.) or intravenous (I.V.) injection to compare the resulting protein expression efficiency in vivo. As a control, EGFP mRNA-LNPs formulated with commercially available ionizable lipids MC3 or SM-102 were used. All mRNA-LNPs were administered at equal mRNA doses per body weight across different concentration groups (n≥4).

Specifically, the dose of mRNA-LNPs was adjusted to 0.1-0.5 mg/kg of body weight, and the formulation was administered via I.M. or I.V. injection to female C57BL/6 mice (6-10 weeksold,Orient Bio, Korea) Subsequently, at 1, 3, 6, 24, 48, and 72 hours after administration, whole-body luminescence images were acquired using an in vivo imaging system (IVIS Lumina III, PerkinElmer, Walzam, MA, USA). To assess protein expression levels, 200 µL of D-luciferin (15 mg/mL) was administered via intraperitoneal (I.P.) injection 10 minutes prior to imaging. During image acquisition, the animals were anesthized by inhalation of 3% isoflurane in 97% medicalgrade oxygen. In addition, the acquired luminescence images were quantitatively analyzed using imaging software (Living Image Software Version 4.4; PerkinElmer, USA).

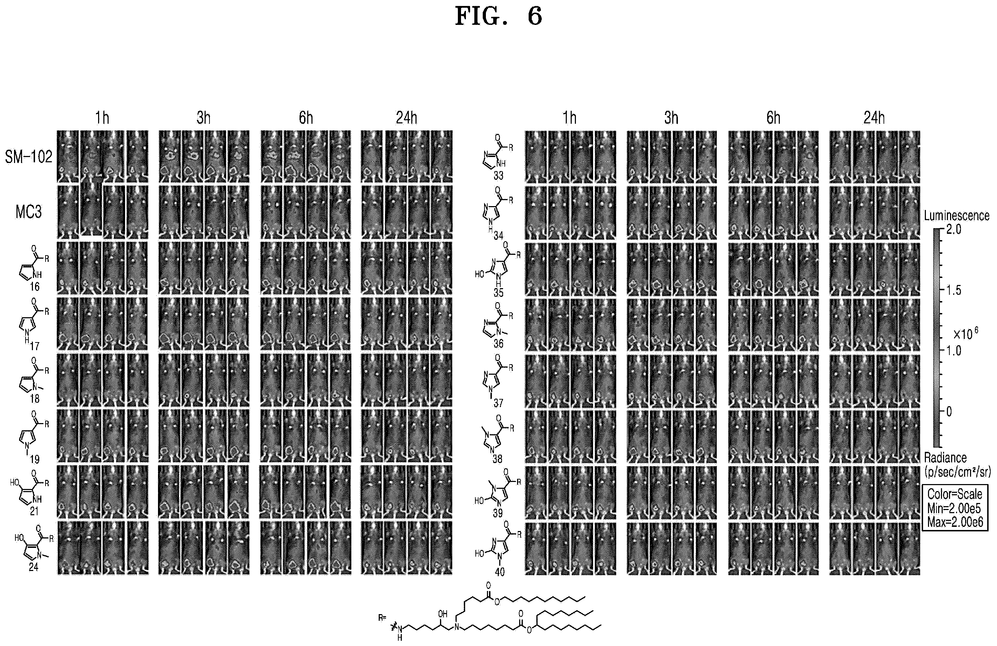

FIG. 6 shows whole-body images of a mouse intramuscularly injected with fLuc mRNA-LNP produced according to Example 27, captured at each time point after administration using an IVIS Lumina III imaging system for small animals.

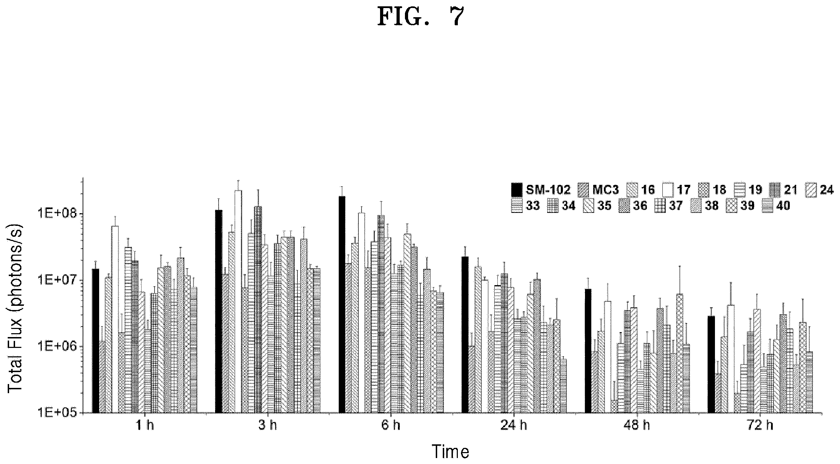

FIG. 7 is a graph presenting the results of quantitative analysis of the luminescence image shown in FIG. 6. After intramuscular injection of the fLuc mRNA-LNPs, the luminescence intensity at the injection site was measured over time. The measured values are summarized in Table 3 below.

**[Table 3]**

| Ionizable lipid | Total flux | | | | | |
|---|---|---|---|---|---|---|
| | 1 hour | 3 hours | 6 hours | 12 hours | 48 hours | 72 hours |
| SM-102 | 1.48E+07 | 1.12E+08 | 1.82E+08 | 2.27E+07 | 7.43E+06 | 2.86E+06 |
| MC3 | 1.20E+06 | 1.24E+07 | 1.78E+07 | 1.03E+06 | 8.41E+05 | 3.89E+05 |
| 16 | 1.11E+07 | 5.26E+07 | 3.64E+07 | 1.61E+07 | 1.71E+06 | 1.40E+06 |
| 17 | 6.53E+07 | 2.23E+08 | 1.02E+08 | 1.00E+07 | 4.80E+06 | 4.21E+06 |
| 18 | 1.63E+06 | 7.75E+06 | 1.56E+07 | 1.70E+06 | 1.54E+05 | 1.96E+05 |
| 19 | 3.16E+07 | 5.15E+07 | 3.82E+07 | 8.29E+06 | 1. 12E+06 | 5.38E+05 |
| 21 | 1.98E+07 | 1.29E+08 | 9.56E+07 | 1.25E+07 | 3.51E+06 | 1.66E+06 |
| 24 | 6.70E+06 | 3.49E+07 | 4.45E+07 | 7.85E+06 | 3.86E+06 | 3.66E+06 |
| 33 | 1.80E+06 | 1.17E+07 | 1.26E+07 | 2.62E+06 | 4.56E+05 | 4.96E+05 |
| 34 | 6.37E+06 | 3.60E+07 | 1.70E+07 | 2.79E+06 | 1.11E+06 | 7.64E+05 |
| 35 | 1.54E+07 | 4.49E+07 | 5.04E+07 | 6.12E+06 | 7.96E+05 | 1.27E+06 |
| 36 | 1.62E+07 | 4.51E+07 | 3.18E+07 | 1.03E+07 | 3.79E+06 | 3.09E+06 |
| 37 | 7.40E+06 | 8.89E+06 | 5.97E+06 | 2.32E+06 | 2.13E+06 | 1.85E+06 |
| 38 | 2.19E+07 | 4.19E+07 | 1.47E+07 | 2.13E+06 | 7.89E+05 | 5.32E+05 |
| 39 | 1.19E+07 | 1.50E+07 | 6.90E+06 | 2.55E+06 | 6.11E+06 | 2.33E+06 |
| 40 | 7.92E+06 | 1.50E+07 | 6.60E+06 | 6.46E+05 | 1.10E+06 | 8.29E+05 |

Referring to FIGS. 6, 7, and Table 3, it was confirmed that all mRNA-LNPs produced according to Example 27 successfully induced in vivo protein expression.

Furthermore, the luminescence intensity at the injection site 3 hours after intramuscular injection was measured, and the relative protein expression efficiency was compared to that of control LNPs formulatedwith MC3 or SM-102. The results of the protein expression analysis are summarized in Table 4 below.

**[Table 4]**

| Ionizable lipid | Fold increase in total flux at 3 h | |
|---|---|---|
| | MC3 | SM-102 |
| 16 | 4.26 | 0.47 |
| 17 | 18.09 | 1.99 |
| 18 | 0.63 | 0.07 |
| 19 | 4.17 | 0.46 |
| 21 | 10.42 | 1.14 |
| 24 | 2.82 | 0.31 |
| 33 | 0.94 | 0.10 |
| 34 | 2.91 | 0.32 |
| 35 | 3.64 | 0.40 |
| 36 | 3.65 | 0.40 |
| 37 | 0.72 | 0.08 |
| 38 | 3.39 | 0.37 |
| 39 | 1.21 | 0.13 |
| 40 | 1.22 | 0.13 |

As shown in Table 4, with the exception of compositions 18, 33, 37, and 40,most of the testedmRNA-LNPs exhibited higher protein expression levels than the control group (MC3) at 3hours post-injection. Notably, compositions 17 and 21 demonstrated significantly higher protein expression compared to the control group (SM-102) at the same time point.

### Experimental Example 6. Formulation optimization of mRNA-LNP

The composition ratio between mRNAs and lipid compound constituting fLuc mRNA-LNPs produced using various ionizable lipids (compounds 1-178) in accordance with the method described in Example 27 wasoptimized.

Specifically, formulation optimization was carried out by adjusting the molar ratios of the four constituent lipids in the LNPs, namely ionizable lipids:phospholipids:cholesterol:PEGylated lipids, within the range of (20-60):(0-25):(30-60):(0-5). Additionally, the N/P ratio-defined as the ratio of the number of ionizable nitrogen atoms in the ionizable lipid to the number of phosphorus atoms in the phosphate backbone of the mRNA- was evaluated in the range of 1.5-15. In practice, more than 15 different formulations were prepared for each ionizable lipid using a standardized procedure. The resulting fLuc mRNA-LNPs were intramuscularly injected into mice in accordance with Experimental Example 5.Luminescence images were acquired at various time points post-injection using an IVIS, and the protein expression levels were quantitatively analyzed. Based on the results, the optimal formulation conditions-i.e., the composition ratio and N/P ratio- yielding the highest protein expression for each ionizable lipid were determined. The optimal composition ratio and N/P ratios for each ionizable lipid are summaried in Table 2 above.

### Experimental Example 7. Evaluation of endosomal escape efficiency of EGFP mRNA-LNP administered to cells

To evaluate the efficiency of endosomal escape of mRNA delivered into the cytoplasm after cellular uptake, EGFP mRNA-LNPs were prepared using various ionizable lipids in accordance with the method described in Example 27 and administered to cells. As a control, EGFP mRNA-LNPs formulated with the commercially available ionizable lipids MC3 or SM-102 were used.

The remaining lipid components constituting the LNPs included cholesterol, DMG-PEG as the PEG-lipids, and DSPC as the phospholipid. For fluorescence microscopy tracking, 0.1 mol% of the total lipid content (i.e., 1 mol% of total phospholipids, assuming phospholipids make up 10 mol% of total lipids) was composed of sulfo-cyanine 5.5-DSPE or ATTO 647N DOPE (Sigma-Aldrich, USA).

Specifically, 18 hours prior to mRNA-LNPs administration, HeLa cells (ATCC, USA) were seeded at a density of 1×10⁵ cells per well in an 8-well plate and cultured in DMEM medium (Gibco, USA) supplemented with FBS and antibiotics at 37°C in a 5% CO₂incubator. As an additional control, EGFP mRNA not formulated into LNPs was directly introduced into the cytoplasm of HeLa cells by electroporation, and these cells were seeded in separate wells for comparative analysis. The dose of administered mRNA-LNPs was adjusted to 100 ng/well (based on mRNA content) and applied to the HeLa cells. Four hours post-injection, the cells were fixed with 4% PFA and imaged using fluorescence microscopy.

To quantitatively measure the number of mRNA molecules present in the cytoplasm, single-molecule fluorescence in situ hybridization (smFISH) was performed. After appropriate incubation, fluorescence imaging was conducted, and the endosome escape efficiency was analyzed by calculating the ratio (R/L) of the number of mRNA molecules detected in the cytoplasm (R) to the number of intracellular LNPs (L). This ratio was compared across different ionizable lipid compositions. To validate fluorescence -based analysis, co-staining with endocytic markers EEA1 (early endosomes) and Lamp1 (lysosomes)- was also performed.

### Experimental Example 8. In vitro evaluation of immunogenicity induced by SARS-CoV-2 Spike mRNA-LNPs

SARS-CoV-2 spike mRNA-LNPs were prepared using various ionizable lipids according to the method described in Example 27, and their immunogenicity was evaluated in vitro by analyzing the expression levels of inflammation-related factors (CCL2, CCL3, IL-1β, and IL-6) at the mRNA level in cells. Specifically, HEK-293T cells were treated with themRNA-LNPs at a dose of 1 µg per well. After 24 hours, total RNA was extracted using Trizol reagent (Thermo Fisher, #15596026). Chloroform was added to the lysate and incubated for 2-3 minutes, followed by centrifugation at 12,000 x g for 15 minutes at 4°C to separate the aqueous phase containing RNA. The aqueous phase was transferred to a new tube, and isopropanol was added. The mixture was incubated at 4°C for 10 minutes and centrifuged at 12,000 x g for 15 minutes at 4°C. The supernatant was discarded, and the RNA pellet was washed with 75% ethanol. After vortexing the sample, it was centrifuged at 7,500 x g, for 5 minutes at 4°C. The supernatant was removed, and the RNA pellet was resuspended in 2-50 µL of RNase-free water. The extracted RNA was reverse-transcribed into cDNA using a reverse transcription kit (Toyobo, #FSK-101). Quantitative real-time PCR (RT-qPCR) was then performed using a Dice TP800 thermal real-timePCR system and SFC green qPCR master mix (SFC, #pgm1005). All measurements were normalized to the housekeeping gene glyceraldehyde-3-phosphate dehydrogenase (GAPDH) . Each experiment was performed in triplicate per condition, and each measurement was conducted in duplicate (n=6). The gene-specific primer sequence used are listed below.
Homo sapiens C-C motif chemokine ligand 2 (CCL2), mRNA
NCBI Reference Sequence: NM_002982.4
F: CAATCAATGCCCCAGTCACC
R: TCGGAGTTTGGGTTTGCTTG
Homo sapiens C-C motif chemokine ligand 3 (CCL3), transcript variant 1, mRNA
NCBI Reference Sequence: NM_002983.3
F: TGTCCTCCTCTGCACCATG
R: TTAGGAAGATGACACCGGGC
Homo sapiens interleukin 1 beta (IL1B), mRNA
NCBI Reference Sequence: NM_000576.3
F: GGAGAATGACCTGAGCACCT
R: GGAGGTGGAGAGCTTTCAGT
Homo sapiens interleukin 6 (IL6), transcript variant 3, mRNA
NCBI Reference Sequence: NM_001371096.1
F: AGTCCTGATCCAGTTCCTGC
R: CTACATTTGCCGAAGAGCCC

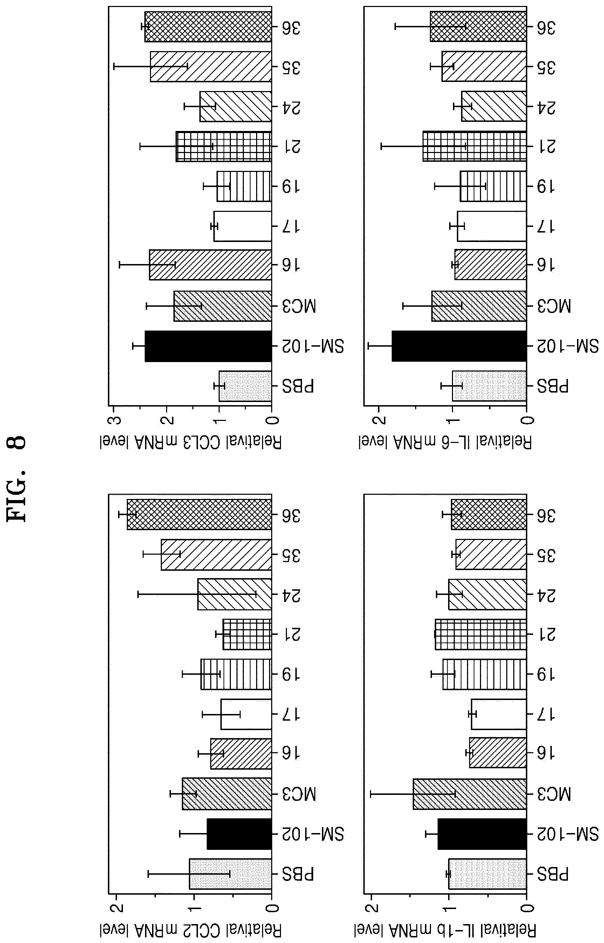

The measurement results are shown in FIG. 8 and Table 5 below.

FIG. 8 illustrates the mRNA levels of four inflammation- related markers induced by LNP treatment in cells administered with mRNA-LNPs produced according to Example 27.

Table 5 summarizes the in vitro immunogenicity levels (relative to PBS control) of the spike mRNA-LNPs produced according to one embodiment.

**[Table 5]**

| Ionizable lipid | CCL2 | | CCL3 | | IL-1β | | IL-6 | |
|---|---|---|---|---|---|---|---|---|
| | Aver age | Standard Deviation | Aver age | Standard Deviation | Aver age | Standard Deviation | Aver age | Standard Deviation |
| PBS | 1.07 | 0.53 | 1.00 | 0.10 | 1.00 | 0.02 | 1.01 | 0.14 |
| SM-102 | 0.83 | 0.36 | 2.41 | 0.24 | 1.14 | 0.17 | 1.82 | 0.33 |
| MC3 | 1.15 | 0.17 | 1.86 | 0.53 | 1.46 | 0.55 | 1.27 | 0.39 |
| 16 | 0.79 | 0.16 | 2.31 | 0.59 | 0.74 | 0.04 | 0.97 | 0.03 |
| 17 | 0.65 | 0.24 | 1.10 | 0.05 | 0.70 | 0.05 | 0.94 | 0.10 |
| 19 | 0.91 | 0.24 | 1.04 | 0.24 | 1.07 | 0.15 | 0.89 | 0.34 |
| 21 | 0.63 | 0.09 | 1.82 | 0.68 | 1.18 | 0.01 | 1.39 | 0.58 |
| 24 | 0.96 | 0.76 | 1.36 | 0.30 | 0.99 | 0.16 | 0.87 | 0.12 |
| 35 | 1.42 | 0.24 | 2.30 | 0.69 | 0.91 | 0.05 | 1.14 | 0.16 |
| 36 | 1.86 | 0.11 | 2.40 | 0.07 | 0.96 | 0.13 | 1.30 | 0.47 |

### Experimental Example 9. In vivo toxicity evaluations

Toxicity induced by fLuc mRNA-LNPs prepared using various ionizable lipids according to the method of Example 27 was evaluated through serum biochemical analysis following I.M. injection into mice.

Specifically, 50-100 µL of serum was collected each mouse at 24 and 48 hours after intravenous injection using the retro-orbital bleeding method. The serum biochemical analysis was performed using Hitachi 7150 Chemistry Analyzer, measuring indicators of liver function- aspartate aminotransferase (AST), alanine aminotransferase (ALT), alkaline phosphatase (ALP)- and indicators of kidney fuction- blood ureanitrogen (BUN)and creatinine.

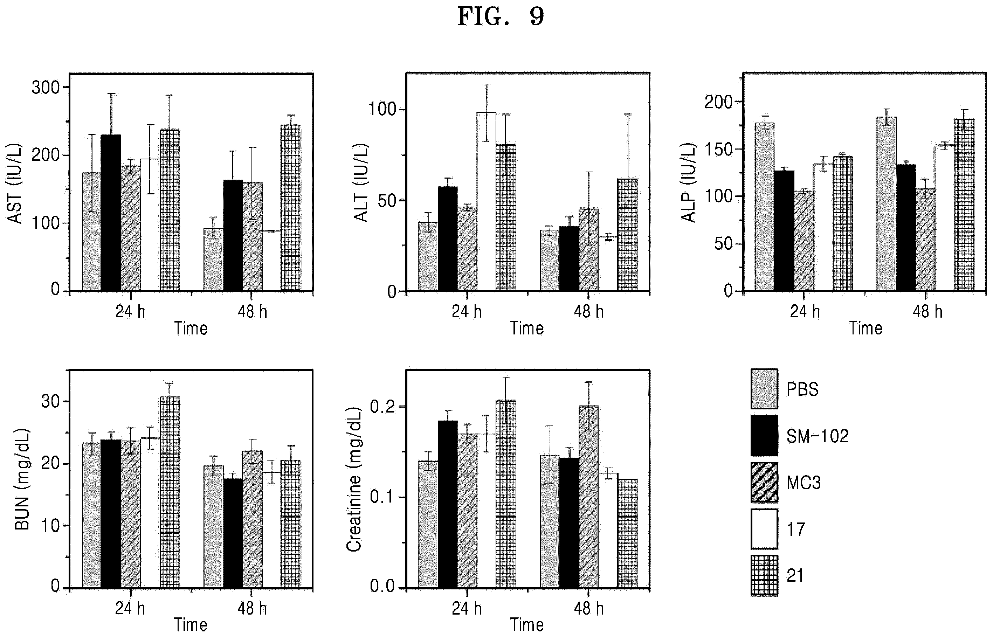

FIG. 9 shows the results of toxicity maker analysis (AST, ALT, ALP, BUN, and creatinine) after administration of mRNA-LNPs produced according to Example 27,based on blood collected at designated time points.

As shown in FIG. 9, Composition 17 exhibited similar ALP and BUN levels compared to control groups (PBS and SM-102) and lower AST levels than both controls, indicating superior in vivo safety.

### Experimental Example 10. Stability evaluation of mRNA-LNPs according to storage temperature and duration

Stability of fLuc mRNA-LNPs produced using various ionizable lipids according to the method of Example 27, was assessed by storing the LNPs at 4°C and 25°C.At 1, 7, and 30 days post-manufacture, the size, PDI, and encapsulation efficiency (EE) of each mRNA-LNPs samples were measured using the methods described in Experimental Examples 2 and 3.

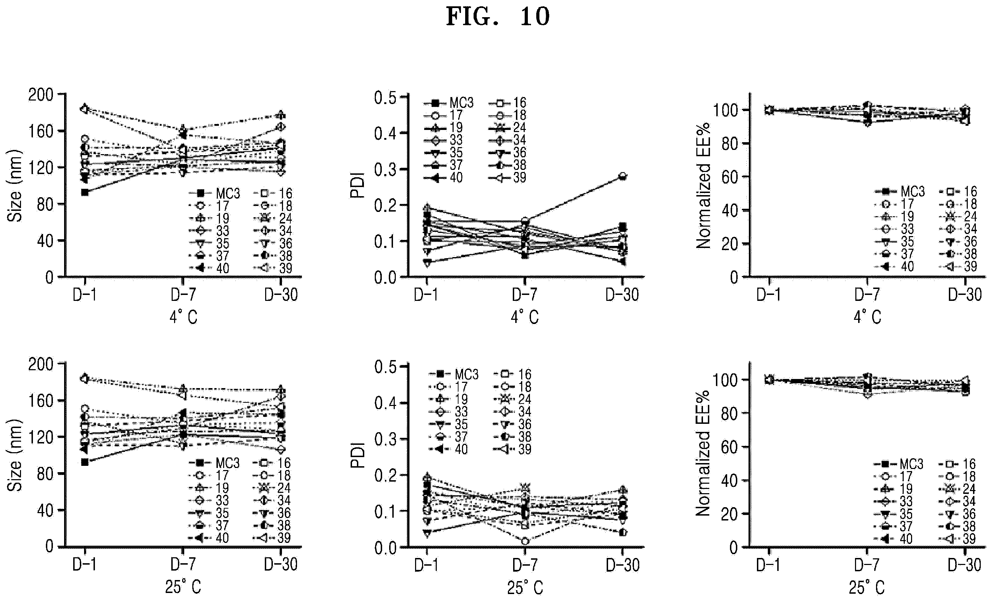

FIG. 10 presents graphs of the size, PDI, and encapsulation efficiency of the mRNA-LNPs over time and under different storage conditions.. The results of this analysis are summarized in Table 6 below.

Referring to FIG. 10 and Table 6, all mRNA-LNPs maintained stable size, PDI, and encapsulation efficiency with no significant changes at either 4°C or 25°C up to 30 days, confirming the high storage stability of the LNP formulations.

**[Table 6]**

| Ionizable lipid | | Size (nm) | | | PDI | | | Normalized EE% | | |
|---|---|---|---|---|---|---|---|---|---|---|
| | | D-1 | D-7 | D-30 | D-1 | D-7 | D-30 | D-1 | D-7 | D-30 |
| MC3 | 4°C | 92.8 | 128.7 | 125.6 | 0.174 | 0.060 | 0.142 | 95.0 | 94.0 | 90.4 |
| | 25°C | 92.8 | 123.0 | 119.3 | 0.174 | 0.110 | 0.123 | 95.0 | 90.8 | 88.3 |
| 16 | 4°C | 132.2 | 137.1 | 141.5 | 0.101 | 0.138 | 0.069 | 90.9 | 91.5 | 86.9 |
| | 25°C | 132.2 | 137.7 | 145.1 | 0.101 | 0.059 | 0.093 | 90.9 | 89.1 | 84.4 |
| 17 | 4°C | 151.2 | 133.8 | 130.7 | 0.100 | 0.098 | 0.081 | 94.0 | 91.3 | 89.8 |
| | 25°C | 151.2 | 134.2 | 128.1 | 0.100 | 0.114 | 0.128 | 94.0 | 90.2 | 88.6 |
| 18 | 4°C | 124.6 | 121.4 | 126.6 | 0.145 | 0.064 | 0.106 | 88.0 | 85.3 | 87.6 |
| | 25°C | 124.6 | 126.1 | 127.5 | 0.145 | 0.016 | 0.125 | 88.0 | 85.6 | 87.0 |
| 19 | 4°C | 184.7 | 161.2 | 177.4 | 0.193 | 0.122 | 0.071 | 93.1 | 90.6 | 87.2 |
| | 25°C | 184.7 | 172.5 | 171.7 | 0.193 | 0.107 | 0.159 | 93.1 | 88.1 | 88.3 |
| 24 | 4°C | 136.9 | 123.7 | - | 0.114 | 0.114 | - | 89.7 | 90.5 | - |
| | 25°C | 136.9 | 113.5 | - | 0.114 | 0.163 | - | 89.7 | 89.6 | - |
| 33 | 4°C | 112.8 | 120.3 | 115.6 | 0.145 | 0.079 | 0.113 | 90.0 | 83.3 | 89.3 |
| | 25°C | 112.8 | 122.9 | 106.4 | 0.145 | 0.133 | 0.113 | 90.0 | 82.1 | 87.2 |
| 34 | 4°C | 116.7 | 121.7 | 164.4 | 0.143 | 0.126 | 0.079 | 94.0 | 92.9 | 91.4 |
| | 25°C | 116.7 | 129.6 | 164.4 | 0.143 | 0.139 | 0.132 | 94.0 | 90.9 | 89.3 |
| 35 | 4°C | 123.4 | 130.3 | 141.3 | 0.040 | 0.089 | 0.127 | 90.2 | 83.7 | 88.6 |
| | 25°C | 123.4 | 133.3 | 123.8 | 0.040 | 0.098 | 0.076 | 90.2 | 85.3 | 88.3 |
| 36 | 4°C | 111.9 | 114.4 | 120.9 | 0.073 | 0.146 | 0.076 | 87.2 | 89.1 | 86.6 |
| | 25°C | 111.9 | 110.6 | 119.2 | 0.073 | 0.123 | 0.092 | 87.2 | 89.0 | 83.8 |
| 37 | 4°C | 114.5 | 123.7 | 138.7 | 0.154 | 0.155 | 0.280 | 89.5 | 89.9 | 90.0 |
| | 25°C | 114.5 | 133.6 | 135.7 | 0.154 | 0.087 | 0.126 | 89.5 | 88.0 | 83.8 |
| 38 | 4°C | 142.2 | 141.1 | 147.3 | 0.132 | 0.086 | 0.086 | 88.5 | 91.1 | 87.6 |
| | 25°C | 142.2 | 140.4 | 152.0 | 0.132 | 0.093 | 0.041 | 89.5 | 88.0 | 86.1 |
| 39 | 4°C | 183.3 | 139.1 | 144.4 | 0.106 | 0.076 | 0.101 | 89.6 | 89.9 | 83.7 |
| | 25°C | 183.3 | 165.8 | 153.3 | 0.106 | 0.067 | 0.107 | 89.6 | 89.3 | 89.3 |
| 40 | 4°C | 106.8 | 155.6 | 146.2 | 0.154 | 0.106 | 0.044 | 92.3 | 89.0 | 87.5 |
| | 25°C | 106.8 | 146.8 | 144.8 | 0.154 | 0.112 | 0.091 | 92.3 | 90.9 | 89.3 |

The above description of the present disclosure is merely an example of the technical ideas of the disclosure, and various modifications and variations will be apparent to those skilled in the art to which the disclosure belongs, without departing from the technical ideas or essential features of the disclosure. Therefore, the Examples disclosed in the present disclosure shall be understood as exemplary and not limited in all aspects.

## Claims

1. A lipid compound of the following Formula 1, an isomer thereof, or a salt thereof: 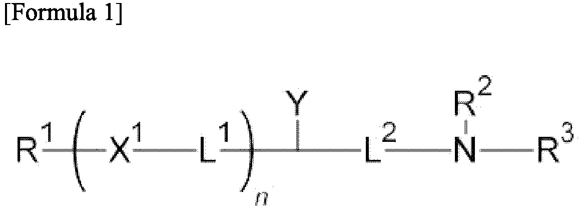 wherein,
R¹ is hydrogen, hydroxy, C₁₋₆ alkyl, C₂₋₆ alkenyl, C₁₋₆ alkoxy, C₂₋₆ alkenyloxy, - CN, -NO₂, -N(R)₂, -C(=O)R, -C(=O)OR, -OC(=O)R, -C(=O)N(R)₂, - NRC(=O)R, - NRC(=O)N(R)₂, -NRC(=S)N(R)₂, C₃₋₁₄ cycloalkyl, 5- to 10-membered heteroaryl, 3- to 14-membered heterocyclyl, -O-5- to 10-membered heteroaryl, or -O-3- to 14-membered heterocyclyl, wherein C₁₋₆ alkyl, C₂₋₆ alkenyl, C₁₋₆ alkoxy, C₂₋₆ alkenyloxy, C₃₋₁₄ cycloalkyl, 5- to 10-membered heteroaryl, 3- to 14-membered heterocyclyl, -O-5- to 10-membered heteroaryl or -O-3- to 14-membered heterocyclyl are each independently unsubstituted or substituted with 1 to 3 halogens or C₁₋₆ alkyl;
X¹ is a simple bond, -CO-C₁₋₆ alkyl, -CO-C₂₋₆ alkenyl, -C(=O)NR-, - NRC(=O)-, - NRC(=O)NR-, -NRC(=S)NR-, C₆₋₁₀ aryl, C₃₋₁₄ cycloalkyl, 5- to 10-membered heteroaryl, 3- to 14- membered heterocyclyl, nucleobases, an amino acid monomer, or an amino acid oligomer, wherein -CO-C₁₋₆ alkyl, - CO-C₂₋₆ alkenyl, C₆₋₁₀ aryl, C₃₋₁₄ cycloalkyl, 5- to 10-membered heteroaryl, 3-to 14-membered heterocyclyl, a nucleobase, an amino acid monomer, and an amino acid oligomer are each independently unsubstituted or substituted with 1 to 3 halogens or C₁₋₆ alkyl;
L¹ is C₁₋₁₄ alkylene, C₂₋₁₄ alkenylene, M, R^{a}M, MR^{a}, MR^{a}M¹, or R^{a}MR^{b}; n is an integer from 1 to 5, and in case n being from 2 to 5, X¹ and L¹ are each independently selected;
L² is C₁₋₁₄ alkylene, C₂₋₁₄ alkenylene, M, R^{a}M, MR^{a}, MR^{a}M¹, or R^{a}MR^{b}, and L² is the same as or different from L¹;
Y is hydrogen, -(CH₂)ₘ OH, -(CH₂)ₘ SH or -(CH₂)ₘ SeH, wherein m is an integer from 0 to 5;
R² and R³ are each independently C₁₋₃₀ alkyl, C₂₋₃₀ alkenyl, or R^{c}MR^{d}, wherein C₁₋₃₀ alkyl and C₂₋₃₀ alkenyl are each independently unsubstituted or substituted with 1 to 3 C₁₋₁₆ alkyl or C₂₋₁₆ alkenyl;
M and M¹ are each independently -NHC(=O)-, -C(=O)NH-, -C(=O)O-, - OC(=O)-, -C(=O)-, -NH-, -S-, -SS-, -O-, -S(=O)₂-, -C(=O)S-, -SC(=O)-, - NHC(=O)NH-, - NHC(=O)O-, or -OC(=O)NH-;
R is each independently hydrogen, C₁₋₆ alkyl, C₂₋₆ alkenyl, C₃₋₁₄ cycloalkyl, 5-to 10-membered heteroaryl, or 3- to 14-membered heterocyclyl;
R^{a} and R^{b} are independently -(CH₂)₁-, -C₃₋₂₀ cycloalkyl-(CH₂)₁-, -(CH₂)₁ - C₃₋₂₀ cycloalkyl-, -C₆₋₂₀ aryl-(CH₂)₁-, -(CH₂)₁-C₆₋₂₀ aryl-, -NH-(CH2)1 -, or -(CH₂)-, wherein 1 is an integer from 0 to 10; R^{c} is C₁₋₁₄ alkylene or C₂₋₁₄ alkenylene;
R^{d} is C₁₋₂₀ alkyl, C₂₋₂₀ alkenyl, or hydrogen, wherein C₁₋₂₀ alkyl and C₂₋₂₀ alkenyl are each independently unsubstituted or substituted with 1 to 3 C₁₋₂₀ alkyl or C₂₋₂₀ alkenyl;
the heteroaryl is an aromatic heterocycle compromising 1 to 6 heteroatoms selected from N, O, and S, and the heterocycle is an aliphatic heterocycle compromising 1 to 6 heteroatoms selected from N, O, and S.

2. The lipid compound, an isomer thereof, or a salt thereof of claim 1, wherein the compound of the Formula 1 is a compound of Formula 2: 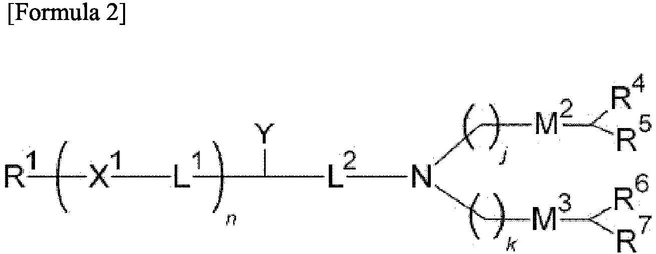 wherein,
*j* and *k* are independently an integer of 1 to 12;
M² and M³ are each independently -NHC(=O)-, -C(=O)NH-, -C(=O)O-, - OC(=O)-, -C(=O)-, -NH-, -S-, -SS-, -O-, -S(O)₂-, -C(=O)S-, -SC(=O)-, - NHC(=O)NH-, - NHC(=O)O-, or -OC(=O)NH; and
R⁴, R⁵, R⁶, and R⁷ are each independently hydrogen, C₁₋₂₄ alkyl, or C₂₋₂₄ alkenyl, wherein the C₁₋₂₄ alkyl and C₂₋₂₄ alkenyl are each independently unsubstituted or substituted with 1 to 3 C₁₋₂₀ alkyl or C₂₋₂₀ alkenyl.

3. The lipid compound, an isomer thereof, or a salt thereof of claim 1 or 2, wherein
R¹ is hydrogen, hydroxy, C₁₋₆ alkyl, C₁₋₆ alkoxy, -CN, -NO₂, -NH₂, -C(=O)-C₁₋₃ alkyl, -C(=O)NH₂, -NHC(=O)-C₁₋₃ alkyl, -NHC(=O)NH₂, or -NHC(=S)NH₂;
X¹ is a simple bond, -CO-C₁₋₆ alkyl-, -CO-C₂₋₆ alkenyl-, -C(=O)NH-, - C(=O)N(CH₃)-, -NHC(=O)-, -N(CH₃)C(=O)-, -NHC(=O)NH-, - N(CH₃)C(=O)NH-, -NHC(=S)NH-, or -N(CH₃)C(=S)NH-, pyrrolyl, furanyl, thiophenyl, pyrazolyl, imidazolyl, isoxazolyl, oxazolyl, isothiazolyl, thiazolyl, 1,2,3-triazolyl, 1,3,4-triazolyl, 1-oxa-2,3-diazolyl, 1-oxa-2,4-diazolyl, 1-oxa-2,5-diazolyl, 1-oxa-3,4-diazolyl, 1-thia-2,3-dia zolyl, 1-thia-2,4-diazolyl, 1-thia-2,5-diazolyl, 1-thia-3,4-diazolyl, tetrazolyl, pyridinyl, pyridazinyl, pyrimidinyl, pyrazinyl, indolyl, isoindolyl, benzofuranyl, benzothiophenyl, indazolyl, benzimidazolyl, benzotriazolyl, pyrrolo[2,3-b]pyridinyl, pyrrolo[2,3-c]pyridinyl, pyrrolo[3,2- c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-c]pyridinyl, pyra zolo[3,4-c]pyridinyl, pyrazolo[3,4-b]pyridinyl, purinyl, indolizinyl, imidazo[1,2-a]pyridinyl, imidazo[1,5-a]pyridinyl, pyrazolo[1,5-a]pyridinyl, pyrrolo[1,2-b]pyridazinyl, imidazo[1,2-c]pyrimidinyl, quinolinyl, isoquinolinyl, cinnolinyl, azaquinazolinyl, quinoxalinyl, phthalazinyl, 1,6-naphthyridinyl, 1,7-naphthyridinyl, 1,8-naphthyridinyl, 1,5-naphthyridinyl, 2,6-naphthyridinyl, 2,7-naphthyridinyl, pyrido[3,2-d]pyrimidinyl, pyrido[4,3-d]pyri midinyl, pyrido[3,4-d]pyrimidinyl, pyrido[2,3-b]pyrimidinyl, pyrido[2,3- b]pyrazinyl, pyrido[3,4-b]pyrazinyl, pyrimido[5,4-d]pyrimidinyl, pyrazino[2,3-b]pyrazinyl, pyrimido[4,5-d]pyrimidinyl, nucleobase, amino acid monomer or amino acid oligomer.

4. The lipid compound, an isomer thereof, or a salt thereof of one of claim 1 to 3, wherein
the lipid compound is selected from the group consisting of below:
(1) Heptadecan-9-yl 8-((6-acetamido-2-hydroxyhexyl)(6-oxo-6-(undecyloxy)hexyl)amino)octanoate;
(2) Heptadecan-9-yl 8-((2-hydroxy-6-ureidohexyl)(6-oxo-6-(undecyloxy)hexyl)amino)octanoate;
(3) Heptadecan-9-yl 8-((2-hydroxy-6-(3-methylureido)hexyl)(6-oxo-6-(undecyloxy)hexyl)amino)octanoate;
(4) Heptadecan-9-yl 17-hydroxy-3,11-dioxo-19-(6-oxo-6-(undecyloxy)hexyl)-7-oxa-2,4,10,12,19-pentaazaheptacosan-27-oate;
(5) Heptadecan-9-yl 25-hydroxy-3,11,19-trioxo-27-(6-oxo-6-(undecyloxy)hexyl)-7,15-dioxa-2,4,10,12,18,20,27-heptaazapentatriacontan-35-oate;
(6) Heptadecan-9-yl 8-((2-hydroxy-6-(3-methylthioureido)hexyl)(6-oxo-6-(undecyloxy)hexyl)amino)octanoate;
(7) Heptadecan-9-yl 8-((6-(cyclopentanecarboxamido)-2-hydroxyhexyl)(6-oxo-6-(undecyloxy)hexyl)amino)octanoate;
(8) Heptadecan-9-yl 8-((2-hydroxy-6-((*R*)-pyrrolidine-3-carboxamido)hexyl)(6-oxo-6-(undecyloxy)hexyl)amino)octanoate;
(9) Heptadecan-9-yl 8-((2-hydroxy-6-((*S*)-pyrrolidine-3-carboxamido)hexyl)(6-oxo-6-(undecyloxy)hexyl)amino)octanoate;
(10) Heptadecan-9-yl 8-((2-hydroxy-6-((*R*)-1-methylpyrrolidine-3-carboxamido)hexyl)(6-oxo-6-(undecyloxy)hexyl)amino)octanoate;
(11) Heptadecan-9-yl 8-((2-hydroxy-6-((*S*)-1-methylpyrrolidine-3-carboxamido)hexyl)(6-oxo-6-(undecyloxy)hexyl)amino)octanoate;
(12) Heptadecan-9-yl 8-((6-(cyclohexanecarboxamido)-2-hydroxyhexyl)(6-oxo-6-(undecyloxy)hexyl)amino)octanoate;
(13) Heptadecan-9-yl 8-((2-hydroxy-6-(piperazine-2-carboxamido)hexyl)(6-oxo-6-(undecyloxy)hexyl)amino)octanoate;
(14) Heptadecan-9-yl 8-((6-(1,4-dimethylpiperazine-2-carboxamido)-2-hydroxyhexyl)(6-oxo-6-(undecyloxy)hexyl)amino)octanoate;
(15) Heptadecan-9-yl 8-((6-(2-(1,4-dimethylpiperazine-2-yl)acetamido)-2-hydroxyhexyl)(6-oxo-6-(undecyloxy)hexyl)amino)octanoate;
(16) Heptadecan-9-yl 8-((2-hydroxy-6-(1*H*-pyrrole-2-carboxamido)hexyl)(6-oxo-6-(undecyloxy)hexyl)amino)octanoate;
(17) Heptadecan-9-yl 8-((2-hydroxy-6-(1*H*-pyrrole-3-carboxamido)hexyl)(6-oxo-6-(undecyloxy)hexyl)amino)octanoate;
(18) Heptadecan-9-yl 8-((2-hydroxy-6-(1-methyl-1*H*-pyrrole-2-carboxamido)hexyl)(6-oxo-6-(undecyloxy)hexyl)amino)octanoate;
(19) Heptadecan-9-yl 8-((2-hydroxy-6-(1-methyl-1*H*-pyrrole-3-carboxamido)hexyl)(6-oxo-6-(undecyloxy)hexyl)amino)octanoate;
(20) Heptadecan-9-yl 8-((2-hydroxy-6-(4-nitro-1*H*-pyrrole-2-carboxamido)hexyl)(6-oxo-6-(undecyloxy)hexyl)amino)octanoate;
(21) Heptadecan-9-yl 8-((2-hydroxy-6-(3-hydroxy-1*H*-pyrrole-2-carboxamido)hexyl)(6-oxo-6-(undecyloxy)hexyl)amino)octanoate;
(22) Heptadecan-9-yl 8-((2-hydroxy-6-(4-hydroxy-1*H*-pyrrole-2-carboxamido)hexyl)(6-oxo-6-(undecyloxy)hexyl)amino)octanoate;
(23) Heptadecan-9-yl 8-((2-hydroxy-6-(5-hydroxy-1*H*-pyrrole-2-carboxamido)hexyl)(6-oxo-6-(undecyloxy)hexyl)amino)octanoate;
(24) Heptadecan-9-yl 8-((2-hydroxy-6-(3-hydroxy-1-methyl-1*H*-pyrrole-2-carboxamido)hexyl)(6-oxo-6-(undecyloxy)hexyl)amino)octanoate;
(25) Heptadecan-9-yl 8-((2-hydroxy-6-(4-hydroxy-1-methyl-1*H*-pyrrole-2-carboxamido)hexyl)(6-oxo-6-(undecyloxy)hexyl)amino)octanoate;
(26) Heptadecan-9-yl 8-((2-hydroxy-6-(5-hydroxy-1-methyl-1*H*-pyrrole-2-carboxamido)hexyl)(6-oxo-6-(undecyloxy)hexyl)amino)octanoate;
(27) Heptadecan-9-yl 8-((2-hydroxy-6-(4-hydroxy-1*H*-pyrrole-3-carboxamido)hexyl)(6-oxo-6-(undecyloxy)hexyl)amino)octanoate;
(28) Heptadecan-9-yl 8-((2-hydroxy-6-(5-hydroxy-1*H*-pyrrole-3-carboxamido)hexyl)(6-oxo-6-(undecyloxy)hexyl)amino)octanoate;
(29) Heptadecan-9-yl 8-((2-hydroxy-6-(2-hydroxy-1*H*-pyrrole-3-carboxamido)hexyl)(6-oxo-6-(undecyloxy)hexyl)amino)octanoate;
(30) Heptadecan-9-yl 8-((2-hydroxy-6-(4-hydroxy-1-methyl-1*H*-pyrrole-3-carboxamido)hexyl)(6-oxo-6-(undecyloxy)hexyl)amino)octanoate;
(31) Heptadecan-9-yl 8-((2-hydroxy-6-(5-hydroxy-1-methyl-1*H*-pyrrole-3-carboxamido)hexyl)(6-oxo-6-(undecyloxy)hexyl)amino)octanoate;
(32) Heptadecan-9-yl 8-((2-hydroxy-6-(2-hydroxy-1-methyl-1*H*-pyrrole-3-carboxamido)hexyl)(6-oxo-6-(undecyloxy)hexyl)amino)octanoate;
(33) Heptadecan-9-yl 8-((2-hydroxy-6-(1*H*-imidazole-2-carboxamido)hexyl)(6-oxo-6-(undecyloxy)hexyl)amino)octanoate;
(34) Heptadecan-9-yl 8-((2-hydroxy-6-(1*H*-imidazole-4-carboxamido)hexyl)(6-oxo-6-(undecyloxy)hexyl)amino)octanoate;
(35) Heptadecan-9-yl 8-((2-hydroxy-6-(2-hydroxy-1*H*-imidazole-4-carboxamido)hexyl)(6-oxo-6-(undecyloxy)hexyl)amino)octanoate;
(36) Heptadecan-9-yl 8-((2-hydroxy-6-(1-methyl-1*H*-imidazole-2-carboxamido)hexyl)(6-oxo-6-(undecyloxy)hexyl)amino)octanoate;
(37) Heptadecan-9-yl 8-((2-hydroxy-6-(1-methyl-1*H*-imidazole-4-carboxamido)hexyl)(6-oxo-6-(undecyloxy)hexyl)amino)octanoate;
(38) Heptadecan-9-yl 8-((2-hydroxy-6-(1-methyl-1*H*-imidazole-5-carboxamido)hexyl)(6-oxo-6-(undecyloxy)hexyl)amino)octanoate;
(39) Heptadecan-9-yl 8-((2-hydroxy-6-(2-hydroxy-1-methyl-1*H*-imidazole-4-carboxamido)hexyl)(6-oxo-6-(undecyloxy)hexyl)amino)octanoate;
(40) Heptadecan-9-yl 8-((2-hydroxy-6-(2-hydroxy-1-methyl-1*H*-imidazole-5-carboxamido)hexyl)(6-oxo-6-(undecyloxy)hexyl)amino)octanoate;
(41) Heptadecan-9-yl 8-((2-hydroxy-6-(1*H*-pyrazole-4-carboxamido)hexyl)(6-oxo-6-(undecyloxy)hexyl)amino)octanoate;
(42) Heptadecan-9-yl 8-((2-hydroxy-6-(1*H*-pyrazole-5-carboxamido)hexyl)(6-oxo-6-(undecyloxy)hexyl)amino)octanoate;
(43) Heptadecan-9-yl 8-((2-hydroxy-6-(1-methyl-1*H*-pyrazole-4-carboxamido)hexyl)(6-oxo-6-(undecyloxy)hexyl)amino)octanoate;
(44) Heptadecan-9-yl 8-((2-hydroxy-6-(1-methyl-1*H*-pyrazole-5-carboxamido)hexyl)(6-oxo-6-(undecyloxy)hexyl)amino)octanoate;
(45) Heptadecan-9-yl 8-((2-hydroxy-6-(1-methyl-1*H*-pyrazole-3-carboxamido)hexyl)(6-oxo-6-(undecyloxy)hexyl)amino)octanoate;
(46) Heptadecan-9-yl 8-((2-hydroxy-6-(isoxazole-5-carboxamido)hexyl)(6-oxo-6-(undecyloxy)hexyl) amino)octanoate;
(47) Heptadecan-9-yl 8-((2-hydroxy-6-(isoxazole-4-carboxamido)hexyl)(6-oxo-6-(undecyloxy)hexyl)amino)octanoate;
(48) Heptadecan-9-yl 8-((2-hydroxy-6-(oxazole-5-carboxamido)hexyl)(6-oxo-6-(undecyloxy)hexyl)amino)octanoate;
(49) Heptadecan-9-yl 8-((2-hydroxy-6-(oxazole-4-carboxamido)hexyl)(6-oxo-6-(undecyloxy)hexyl)amino)octanoate;
(50) Heptadecan-9-yl 8-((6-(furan-3-carboxamido)-2-hydroxyhexyl)(6-oxo-6-(undecyloxy)hexyl)amino)octanoate;
(51) Heptadecan-9-yl 8-((2-hydroxy-6-(thiophene-3-carboxamido)hexyl)(6-oxo-6-(undecyloxy)hexyl)amino)octanoate;
(52) Heptadecan-9-yl 8-((2-hydroxy-6-(picolinamido)hexyl)(6-oxo-6-(undecyloxy)hexyl)amino)octanoate;
(53) Heptadecan-9-yl 8-((2-hydroxy-6-(nicotinamido)hexyl)(6-oxo-6-(undecyloxy)hexyl)amino)octanoate;
(54) Heptadecan-9-yl 8-((2-hydroxy-6-(isonicotinamido)hexyl)(6-oxo-6-(undecyloxy)hexyl)amino)octanoate;
(55) Heptadecan-9-yl 8-((2-hydroxy-6-(pyridazine-3-carboxamido)hexyl)(6-oxo-6-(undecyloxy)hexyl)amino)octanoate;
(56) Heptadecan-9-yl 8-((2-hydroxy-6-(pyridazine-4-carboxamido)hexyl)(6-oxo-6-(undecyloxy)hexyl)amino)octanoate;
(57) Heptadecan-9-yl 8-((2-hydroxy-6-(pyrazine-2-carboxamido)hexyl)(6-oxo-6-(undecyloxy)hexyl)amino)octanoate;
(58) Heptadecan-9-yl 8-((2-hydroxy-6-(pyrimidine-4-carboxamido)hexyl)(6-oxo-6-(undecyloxy)hexyl)amino)octanoate;
(59) Heptadecan-9-yl 8-((2-hydroxy-6-(pyrimidine-5-carboxamido)hexyl)(6-oxo-6-(undecyloxy)hexyl)amino)octanoate;
(60) Heptadecan-9-yl 8-((2-hydroxy-6-(pyrimidine-2-carboxamido)hexyl)(6-oxo-6-(undecyloxy)hexyl)amino)octanoate;
(61) Heptadecan-9-yl 8-((2-hydroxy-6-(1*H*-indole-2-carboxamido)hexyl)(6-oxo-6-(undecyloxy)hexyl)amino)octanoate;
(62) Heptadecan-9-yl 8-((2-hydroxy-6-(1-methyl-1*H*-indole-2-carboxamido)hexyl)(6-oxo-6-(undecyloxy)hexyl)amino)octanoate;
(63) Heptadecan-9-yl 8-((2-hydroxy-6-(1*H*-indole-3-carboxamido)hexyl)(6-oxo-6-(undecyloxy)hexyl)amino)octanoate;
(64) Heptadecan-9-yl 8-((2-hydroxy-6-(1-methyl-1*H*-indole-3-carboxamido)hexyl)(6-oxo-6-(undecyloxy)hexyl)amino)octanoate;
(65) Heptadecan-9-yl 8-((6-(4-amino-2-oxo-1,2-dihydropyrimidine-1-carboxamido)-2-hydroxyhexyl)(6-oxo-6-(undecyloxy)hexyl)amino)octanoate;
(66) Heptadecan-9-yl 8-((6-(2,4-dioxo-1,2,3,4-tetrahydropyrimidine-1-carboxamido)-2-hydroxyhexyl)(6-oxo-6-(undecyloxy)hexyl)amino)octanoate;
(67) Heptadecan-9-yl 8-((6-(6-amino-9*H*-purine-9-carboxamido)-2-hydroxyhexyl)(6-oxo-6-(undecyloxy)hexyl)amino)octanoate;
(68) Heptadecan-9-yl 8-((6-(2-amino-6-oxo-6,9-dihydro-1*H*-purine-9-carboxamido)-2-hydroxyhexyl)(6-oxo-6-(undecyloxy)hexyl)amino)octanoate;
(69) Heptadecan-9-yl 8-((6-(2-(4-amino-2-oxopyrimidin-1(2*H*)-yl)acetamido)-2-hydroxyhexyl)(6-oxo-6-(undecyloxy)hexyl)amino)octanoate;
(70) Heptadecan-9-yl 8-((6-(2-(2,4-dioxo-3,4-dihydropyrimidine-1(2*H*)-yl)acetamido)-2-hydroxyhexyl)(6-oxo-6-(undecyloxy)hexyl)amino)octanoate;
(71) Heptadecan-9-yl 8-((6-(2-(6-amino-9*H*-purin-9-yl)acetamido)-2-hydroxyhexyl)(6-oxo-6-(undecyloxy)hexyl)amino)octanoate;
(72) Heptadecan-9-yl 8-((6-(2-(2-amino-6-oxo-1,6-dihydro-9*H*-purin-9-yl)acetamido)-2-hydroxyhexyl)(6-oxo-6-(undecyloxy)hexyl) amino)octanoate;
(73) Heptadecan-9-yl 8-((6-((*S*)-2-amino-5-guanidinopentanamido)-2-hydroxyhexyl)(6-oxo-6-(undecyloxy)hexyl)amino)octanoate;
(74) Heptadecan-9-yl 8-((6-((*S*)-2-acetamido-5-guanidinopentanamido)-2-hydroxyhexyl)(6-oxo-6-(undecyloxy)hexyl)amino)octanoate;
(75) Heptadecan-9-yl 8-((6-((*S*)-2-amino-3-(1*H*-imidazol-5-yl)propanamido)-2-hydroxyhexyl)(6-oxo-6-(undecyloxy)hexyl)amino)octanoate;
(76) Heptadecan-9-yl 8-((6-((*S*)-2-acetamido-3-(1*H*-imidazol-5-yl)propanamido)-2-hydroxyhexyl)(6-oxo-6-(undecyloxy)hexyl)amino)octanoate;
(77) Heptadecan-9-yl 8-((6-((*S*)-2-amino-3-(1*H*-indole-3-yl)propanamido)-2-hydroxyhexyl)(6-oxo-6-(undecyloxy)hexyl)amino)octanoate;
(78) Heptadecan-9-yl 8-((6-((*S*)-2-acetamido-3-(1*H*-indole-3-yl)propanamido)-2-hydroxyhexyl)(6-oxo-6-(undecyloxy)hexyl)amino)octanoate;
(79) Heptadecan-9-yl 8-((2-hydroxy-6-((*S*)-pyrrolidine-2-carboxamido)hexyl)(6-oxo-6-(undecyloxy)hexyl)amino)octanoate;
(80) Heptadecan-9-yl 8-((2-hydroxy-6-((*S*)-1-methylpyrrolidine-2-carboxamido)hexyl)(6-oxo-6-(undecyloxy)hexyl)amino)octanoate;
(81) Heptadecan-9-yl (6*S*,9*S*)-1,6-diamino-9-(3-guanidinopropyl)-16-hydroxy-1-imino-7,10-dioxo-18-(6-oxo-6-(undecyloxy)hexyl)-2,8,11,18-tetraazahexacosan-26-oate;
(82) Heptadecan-9-yl 8-((6-((*S*)-2-((*S*)-2-amino-3-(1*H*-imidazol-5-yl)propanamido)-5-guanidinopentanamido)-2-hydroxyhexyl)(6-oxo-6-(undecyloxy)hexyl)amino)octanoate;
(83) Heptadecan-9-yl 8-((6-((*S*)-2-((*S*)-2-amino-3-(1*H*-imidazol-5-yl)propanamido)-3-(1*H*-imidazol-5-yl)propanamido)-2-hydroxyhexyl)(6-oxo-6-(undecyloxy)hexyl)amino)octanoate;
(84) Heptadecan-9-yl (6*R*,9*S*)-9-((1*H*-imidazol-5-yl)methyl)-1,6-diamino-16-hydroxy-1-imino-7,10-dioxo-18-(6-oxo-6-(undecyloxy)hexyl)-2,8,11,18-tetraazahexacosan-26-oate;
(85) Heptadecan-9-yl (6*R*,9*S*,12*5*)-1,6-diamino-9,12-bis(3-guanidinopropyl)-19-hydroxy-1-imino-7,10,13-trioxo-21-(6-oxo-6-(undecyloxy)hexyl)-2,8,11,14,21-pentaazanonacosan-29-oate;
(86) Heptadecan-9-yl (6*S*,9*S*)-1-amino-6-((*R*)-2-amino-3-(1*H*-imidazol-5-yl)propanamido)-9-(3-guanidinopropyl)-16-hydroxy-1-imino-7,10-dioxo-18-(6-oxo-6-(undecyloxy)hexyl)-2,8,11,18-tetraazahexacosan-26-oate;
(87) Heptadecan-9-yl (2*R*,5*S*,8*S*)-5-((1*H*-imidazol-5-yl)methyl)-2-amino-8-(3-guanidinopropyl)-15-hydroxy-1-(1*H*-imidazol-5-yl)-3,6,9-trioxo-17-(6-oxo-6-(undecyloxy)hexyl)-4,7,10,17-tetraazapentacosan-25-oate;
(88) Heptadecan-9-yl (6*R*,9*S*,12*S*)-9-((1*H-*imidazol-5-yl)methyl)-1,6-diamino-12-(3-guanidinopropyl)-19-hydroxy-1-imino-7,10,13-trioxo-21-(6-oxo-6-(undecyloxy)hexyl)-2,8,11,14,21-pentaazanonacosan-29-oate;
(89) Heptadecan-9-yl (2*R*,5*S*,8*S*)-5,8-bis((1*H*-imidazol-5-yl)methyl)-2-amino-15-hydroxy-1-(1*H*-imidazol-5-yl)-3,6,9-trioxo-17-(6-oxo-6-(undecyloxy)hexyl)-4,7,10,17-tetraazapentacosan-25-oate;
(90) Heptadecan-9-yl (6*R*,9*S*,12*S*)-9,12-bis((1*H*-imidazol-5-yl)methyl)-1,6-diamino-19-hydroxy-1-imino-7,10,13-trioxo-21-(6-oxo-6-(undecyloxy)hexyl)-2,8,11,14,21-pentaazanonacosan-29-oate;
(91) Heptadecan-9-yl (6*R*,9*S*,12*S*)-12-((1*H*-imidazol-5-yl)methyl)-1,6-diamino-9-(3-guanidinopropyl)-19-hydroxy-1-imino-7,10,13-trioxo-21-(6-oxo-6-(undecyloxy)hexyl)-2,8,11, 14,21-pentaazanonacosan-29-oate;
(92) Heptadecan-9-yl (6*S*,9*S*)-9-((1*H*-imidazol-5-yl)methyl)-1-amino-6-((*R*)-2-amino-3-(1*H*-imidazol-5-yl)propanamido)-16-hydroxy-1-imino-7,10-dioxo-18-(6-oxo-6-(undecyloxy)hexyl)-2,8,11,18-tetraazahexacosan-26-oate;
(93) Heptadecan-9-yl 1-(4-amino-2-oxopyrimidin-1(2*H*)-yl)-9-(2-(6-amino-9*H-*purin-9-yl)acetyl)-3-(2-aminoethyl)-17-hydroxy-2,5,11-trioxo-19-(6-oxo-6-(undecyloxy)hexyl)-3,6,9,12,19-pentaazaheptacosan-27-oate;
(94) Heptadecan-9-yl 9-(2-(4-amino-2-oxopyrimidin-1(2*H*)-yl)acetyl)-1-(2-amino-6-oxo-1,6-dihydro-9*H*-purin-9-yl)-15-(2-(6-amino-9*H*-purin-9-yl)acetyl)-3-(2-aminoethyl)-23-hydroxy-2,5,11,17-tetraoxo-25-(6-oxo-6-(undecyloxy)hexyl)-3,6,9,12,15,18,25-heptaazatritriacontan-23-oate;
(95) Heptadecan-9-yl 15-(2-(4-amino-2-oxopyrimidin-1(2*H*)-yl)acetyl)-9-(2-(2-amino-6-oxo-1,6-dihydro-9H-purin-9-yl)acetyl)-21-(2-(6-amino-9*H*-purin-9-yl)acetyl)-3-(2-aminoethyl)-1-(2,4-dioxo-3,4-dihydropyrimidin-1(2*H*)-yl)-29-hydroxy-2,5,11,17,23-pentaoxo-31-(6-oxo-6-(undecyloxy)hexyl)-3,6,9,12,15,18,21,24,31-nonaazanonatriacontan-39-oate;
(96) Heptadecan-9-yl 21-(2-(4-amino-2-oxopyrimidin-1(2*H*)-yl)acetyl)-15-(2-(2-amino-6-oxo-1,6-dihydro-9*H*-purin-9-yl)acetyl)-1-(2-(6-amino-9*H*-purin-9-yl)-27-(2-(6-amino-9*H*-purin-9-yl)acetyl)-3-(2-aminoethyl)-9-(2-(2,4-dioxo-3,4-dihydropyrimidin-1(2*H*)-yl)acetyl)-35-hydroxy-2,5,11,17,23,29-hexaoxo-37-(6-oxo-6-(undecyloxy)hexyl)-3,6,9,12,15,18,21,24,,27,30,37-undecaazapentatetracontan-45-oate;
(97) Heptadecan-9-yl 8-((6-(4-(4-amino-1-methyl-1*H*-pyrrole-2-carboxamido)-1-methyl-1*H*-pyrrole-2-carboxamido)-2-hydroxyhexyl)(6-oxo-6-(undecyloxy) hexyl)amino)octanoate;
(98) Heptadecan-9-yl 8-((6-(4-(4-(4-amino-1-methyl-1*H*-imidazole-2-carboxamido)-1-methyl-1*H*-pyrrole-2-carboxamido)-1-methyl-1*H*-pyrrole-2-carboxamido)-2-hydroxyhexyl)(6-oxo-6-(undecyloxy)hexyl)amino)octanoate;
(99) Heptadecan-9-yl 8-((2-hydroxy-6-(1-methyl-4-(1-methyl-4-(1-methyl-4-(1-methyl-1*H*-imidazole-2-carboxamido)-1*H*-imidazole-2-carboxamido)-1*H-*pyrrole-2-carboxamido)-1*H*-pyrrole-2-carboxamido)hexyl)(6-oxo-6-(undecyloxy)hexyl)amino)octanoate;
(100) Heptadecan-9-yl 8-((2-hydroxy-4-(3-methylureido)butyl)(6-oxo-6-(undecyloxy)hexyl)amino)octanoate;
(101) Heptadecan-9-yl 8-((2-hydroxy-5-(3-methylureido)pentyl)(6-oxo-6-(undecyloxy)hexyl)amino)octanoate;
(102) Heptadecan-9-yl 8-((2-hydroxy-7-(3-methylureido)heptyl)(6-oxo-6-(undecyloxy)hexyl)amino)octanoate;
(103) Heptadecan-9-yl 8-((2-hydroxy-8-(3-methylureido)octyl)(6-oxo-6-(undecyloxy)hexyl)amino)octanoate;
(104) Heptadecan-9-yl 9-hydroxy-3-oxo-11-(6-oxo-6-(undecyloxy)hexyl)-7-oxa-2,4,11-triazanonadecan-19-oate;
(105) Heptadecan-9-yl 12-hydroxy-3-oxo-14-(6-oxo-6-(undecyloxy)hexyl)-7,10-dioxa-2,4,14-triazadocosan-22-oate;
(106) Heptadecan-9-yl 15-hydroxy-3-oxo-17-(6-oxo-6-(undecyloxy)hexyl)-7,10,13-trioxa-2,4,17-triazapentacosan-25-oate;
(107) Heptadecan-9-yl 8-((2-(hydroxymethyl)-6-(3-methylureido)hexyl)(6-oxo-6-(undecyloxy)hexyl)amino)octanoate;
(108) Heptadecan-9-yl 8-((2-(2-hydroxyethyl)-6-(3-methylureido)hexyl)(6-oxo-6-(undecyloxy)hexyl)amino)octanoate;
(109) Heptadecan-9-yl 8-((2-(3-hydroxypropyl)-6-(3-methylureido)hexyl)(6-oxo-6-(undecyloxy)hexyl)amino)octanoate;
(110)(6Z,9Z,28Z,31Z)-heptatriaconta-6,9,28,31-tetraen-19-yl 4-((2-hydroxy-6-(3-methylureido)hexyl)(methyl)amino)butanoate;
(111) Heptadecan-9-yl 8-((2-hydroxy-6-(piperazine-1-carboxamido)hexyl)(6-oxo-6-(undecyloxy)hexyl)amino)octanoate;
(112) Heptadecan-9-yl 8-((2-hydroxy-6-(4-methylpiperazine-1-carboxamido)hexyl)(6-oxo-6-(undecyloxy)hexyl)amino)octanoate;
(113) Heptadecan-9-yl 8-((2-hydroxy-6-(2-(piperazin-1-yl)acetamido)hexyl)(6-oxo-6-(undecyloxy)hexyl)amino)octanoate;
(114) Heptadecan-9-yl 8-((2-hydroxy-6-(2-(4-methylpiperazin-1-yl)acetamido)hexyl)(6-oxo-6-(undecyloxy)hexyl)amino)octanoate;
(115) Heptadecan-9-yl 8-((6-(furan-2-carboxamido)-2-hydroxyhexyl)(6-oxo-6-(undecyloxy)hexyl)amino)octanoate;
(116) Heptadecan-9-yl8-((2-hydroxy-6-(thiophene-2-carboxamido)hexyl)(6-oxo-6-(undecyloxy)hexyl)amino)octanoate;
(117) Heptadecan-9-yl 8-((6-(3-methylureido)hexyl)(6-oxo-6-(undecyloxy) hexyl)amino)octanoate;
(118) Heptadecan-9-yl 8-((6-(dodecan-2-yloxy)-6-oxohexyl)(2-hydroxy-6- (3-methylureido)hexyl)amino)octanoate;
(119) Heptadecan-9-yl 8-((2-hydroxy-6-(3-methylureido)hexyl)(6-oxo-6-(tridecan-3-yloxy)hexyl) amino)octanoate;
(120) 5-((8-(Heptadecan-9-yloxy)-8-oxooctyl)(2-hydroxy-6-(3-methylureido)hexyl)amino)pentyl dodecanoate;
(121) (9Z,28Z)-heptatriaconta-9,28-dien-19-yl 4-((2-hydroxy-6-(3-methylureido)hexyl)(methyl)amino)butanoate;
(122) Heptadecan-9-yl 8-((2-hydroxy-6-(1,3,5-triazine-2-carboxamido)hexyl)(6-oxo-6-(undecyloxy)hexyl)amino)octanoate;
(123) Heptadecan-9-yl 8-((2-hydroxy-4-(1*H*-pyrrole-3-carboxamido)butyl)(6-oxo-6-(undecyloxy)hexyl)amino)octanoate;
(124) Heptadecan-9-yl 8-((2-hydroxy-5-(1*H*-pyrrole-3-carboxamido)pentyl)(6-oxo-6-(undecyloxy)hexyl)amino)octanoate;
(125) Heptadecan-9-yl 8-((2-hydroxy-7-(1*H*-pyrrole-3-carboxamido)heptyl)(6-oxo-6-(undecyloxy)hexyl)amino)octanoate;
(126) Heptadecan-9-yl 8-((2-hydroxy-8-(1*H*-pyrrole-3-carboxamido)octyl)(6-oxo-6-(undecyloxy)hexyl)amino)octanoate;
(127) Heptadecan-9-yl 8-((3-(2-(1*H*-pyrrole-3-carboxamido)ethoxy)-2-hydroxypropyl)(6-oxo-6-(u ndecyloxy)hexyl)amino)octanoate;
(128) Heptadecan-9-yl 10-hydroxy-1-oxo-12-(6-oxo-6-(undecyloxy)hexyl)-1-(1*H*-pyrrol-3-yl)-5,8-dioxa-2,12-diazacosan-20-oate;
(129) Heptadecan-9-yl 13-hydroxy-1-oxo-15-(6-oxo-6-(undecyloxy)hexyl)-1-(1*H*-pyrrol-3-yl)-5,8,11-trioxa-2,15-diazatricosan-23-oate;
(130) Heptadecan-9-yl 8-((2-(hydroxymethyl)-6-(1*H*-pyrrole-3-carboxamido)hexyl)(6-oxo-6-(undecyloxy)hexyl)amino)octanoate;
(131) Heptadecan-9-yl 8-((2-(2-hydroxyethyl)-6-(1*H*-pyrrole-3-carboxamido)hexyl)(6-oxo-6-(undecyloxy)hexyl)amino)octanoate;
(132) Heptadecan-9-yl 8-((2-(3-hydroxypropyl)-6-(1*H*-pyrrole-3-carboxamido)hexyl)(6-oxo-6-(undecyloxy)hexyl)amino)octanoate;
(133) Heptadecan-9-yl 8-((6-(1*H*-pyrrole-3-carboxamido)hexyl)(6-oxo-6-(undecyloxy)hexyl)amino)octanoate;
(134) Heptadecan-9-yl 8-((6-(dodecan-2-yloxy)-6-oxohexyl)(2-hydroxy-6-(1*H-*pyrrole-3-carboxamido)hexyl)amino)octanoate;
(135) Heptadecan-9-yl 8-((2-hydroxy-6-(1*H*-pyrrole-3-carboxamido)hexyl)(6-oxo-6-(tridecan-3-yloxy)hexyl)amino)octanoate;
(136) 5-((8-(Heptadecan-9-yloxy)-8-oxooctyl)(2-hydroxy-6-(1*H*-pyrrole-3-carboxamido)hexyl)amino)pentyl dodecanoate;
(137) (9Z,28Z)-heptatriaconta-9,28-dien-19-yl 4-((2-hydroxy-6-(1*H*-pyrrole-3-carboxamido)hexyl)(methyl) amino)butanoate;
(138) (6Z,9Z,28Z,31Z)-heptatriaconta-6,9,28,31-tetraen-19-yl 4-((2-hydroxy-6-(1*H*-pyrrole-3-carboxamido)hexyl)(methyl)amino)butanoate;
(139) Heptadecan-9-yl 8-((6-(heptan-2-yloxy)-6-oxohexyl)(2-hydroxy-6-(1*H-*pyrrole-3-carboxamido)hexyl)amino)octanoate;
(140) Heptadecan-9-yl 8-((2-hydroxy-6-(1*H*-pyrrole-3-carboxamido)hexyl)(6-(octan-2-yloxy)-6-oxohexyl)amino)octanoate;
(141) Heptadecan-9-yl 8-((2-hydroxy-6-(1*H*-pyrrole-3-carboxamido)hexyl)(6-(nonan-2-yloxy)-6-oxohexyl)amino)octanoate;
(142) Heptadecan-9-yl 8-((2-hydroxy-6-(1*H*-pyrrole-3-carboxamido)hexyl)(6-oxo-6-(tridecan-2-yloxy)hexyl)amino)octanoate;
(143) Heptadecan-9-yl 8-((2-hydroxy-6-(1*H*-pyrrole-3-carboxamido)hexyl)(6-oxo-6-(tetradecan-2-yloxy)hexyl)amino)octanoate;
(144) Heptadecan-9-yl 8-((2-hydroxy-6-(1*H*-pyrrole-3-carboxamido)hexyl)(6-(octan-3-yloxy)-6-oxohexyl)amino)octanoate;
(145) Heptadecan-9-yl 8-((2-hydroxy-6-(1*H*-pyrrole-3-carboxamido)hexyl)(6-oxo-6-(undecan-3-yloxy)hexyl)amino)octanoate;
(146) Heptadecan-9-yl 8-((2-hydroxy-6-(1*H*-pyrrole-3-carboxamido)hexyl)(6-(nonan-4-yloxy)-6-oxohexyl)amino)octanoate;
(147) Heptadecan-9-yl 8-((2-hydroxy-6-(1*H*-pyrrole-3-carboxamido)hexyl)(6-oxo-6-(undecan-4-yloxy)hexyl)amino)octanoate;
(148) Heptadecan-9-yl 8-((6-(decan-5-yloxy)-6-oxohexyl)(2-hydroxy-6-(1*H-*pyrrole-3-carboxamido)hexyl)amino)octanoate;
(149) Heptadecan-9-yl 8-((2-hydroxy-6-(1*H*-pyrrole-3-carboxamido)hexyl)(6-oxo-6-(undecan-5-yloxy)hexyl)amino)octanoate;
(150) Heptadecan-9-yl 8-((2-hydroxy-6-(1*H*-pyrrole-3-carboxamido)hexyl)(6-oxo-6-(undecan-6-yloxy)hexyl)amino)octanoate;
(151) Heptadecan-9-yl 8-((2-hydroxy-6-(1*H*-pyrrole-3-carboxamido)hexyl)(6-((2-methylpentyl)oxy)-6-oxohexyl)amino)octanoate;
(152) Heptadecan-9-yl 8-((2-hydroxy-6-(1*H*-pyrrole-3-carboxamido)hexyl)(6-((2-methyloctyl)oxy)-6-oxohexyl)amino)octanoate;
(153) Heptadecan-9-yl 8-((2-hydroxy-6-(1*H*-pyrrole-3-carboxamido)hexyl)(6-((2-methylnonyl)oxy)-6-oxohexyl)amino)octanoate;
(154) Heptadecan-9-yl 8-((2-hydroxy-6-(1*H*-pyrrole-3-carboxamido)hexyl)(6-((2-methyldecyl)oxy)-6-oxohexyl)amino)octanoate;
(155) Heptadecan-9-yl 8-((2-hydroxy-6-(1*H*-pyrrole-3-carboxamido)hexyl)(6-((2-methylundecyl)oxy)-6-oxohexyl)amino)octanoate;
(156) Heptadecan-9-yl 8-((2-hydroxy-6-(1*H*-pyrrole-3-carboxamido)hexyl)(6-((2-methyldodecyl)oxy)-6-oxohexyl)amino)octanoate;
(157) Heptadecan-9-yl 8-((2-hydroxy-6-(1*H*-pyrrole-3-carboxamido)hexyl)(6-((2-methyltridecyl)oxy)-6-oxohexyl)amino)octanoate;
(158) Heptadecan-9-yl 8-((2-hydroxy-6-(1*H*-pyrrole-3-carboxamido)hexyl)(6-((2-methyltetradecyl)oxy)-6-oxohexyl)amino)octanoate;
(159) Heptadecan-9-yl 8-((6-((2-ethylhexyl)oxy)-6-oxohexyl)(2-hydroxy-6-(1*H*-pyrrole-3-carboxamido)hexyl)amino)octanoate;
(160) Heptadecan-9-yl 8-((6-((2-butyloctyl)oxy)-6-oxohexyl)(2-hydroxy-6-(1*H*-pyrrole-3-carboxamido)hexyl)amino)octanoate;
(161) Heptadecan-9-yl 8-((6-((2-hexyloctyl)oxy)-6-oxohexyl)(2-hydroxy-6-(1*H*-pyrrole-3-carboxamido)hexyl)amino)octanoate;
(162) Heptadecan-9-yl 8-((2-hydroxy-6-(1*H*-pyrrole-3-carboxamido)hexyl)(6-((2-octyldodecyl)oxy)-6-oxohexyl)amino)octanoate;
(163) Heptadecan-9-yl 8-((2-hydroxy-6-(1*H*-pyrrole-3-carboxamido)hexyl)(6-((6-methylnonyl)oxy)-6-oxohexyl)amino)octanoate;
(164) Heptadecan-9-yl 8-((2-hydroxy-6-(1*H*-pyrrole-3-carboxamido)hexyl)(6-((6-methyloctyl)oxy)-6-oxohexyl)amino)octanoate;
(165) Heptadecan-9-yl 8-((2-hydroxy-6-(1*H*-pyrrole-3-carboxamido)hexyl)(6-((7-methylnonyl)oxy)-6-oxohexyl)amino)octanoate;
(166) Heptadecan-9-yl 8-((2-hydroxy-6-(1*H*-pyrrole-3-carboxamido)hexyl)(6-((8-methyldecyl)oxy)-6-oxohexyl)amino)octanoate;
(167) Heptadecan-9-yl 8-((2-hydroxy-6-(1*H*-pyrrole-3-carboxamido)hexyl)(6-((7-methyloctyl)oxy)-6-oxohexyl)amino)octanoate;
(168) Heptadecan-9-yl 8-((2-hydroxy-6-(1*H*-pyrrole-3-carboxamido)hexyl)(6-((8-methylnonyl)oxy)-6-oxohexyl)amino)octanoate;
(169) Heptadecan-9-yl 8-((2-hydroxy-6-(1*H*-pyrrole-3-carboxamido)hexyl)(6-((9-methyldecyl)oxy)-6-oxohexyl)amino)octanoate;
(170) Heptadecan-9-yl 8-((2-hydroxy-6-(1*H*-pyrrole-3-carboxamido)hexyl)(6-((10-methylundecyl)oxy)-6-oxohexyl)amino)octanoate;
(171) Heptadecan-9-yl 8-((2-hydroxy-6-(1*H*-pyrrole-3-carboxamido)hexyl)(6-((11-methyldodecyl)oxy)-6-oxohexyl)amino)octanoate;
(172) Heptadecan-9-yl 8-((2-hydroxy-6-(1*H*-pyrrole-3-carboxamido)hexyl)(6-((12-methyltridecyl)oxy)-6-oxohexyl)amino)octanoate;
(173) Heptadecan-9-yl 8-((2-hydroxy-6-(1*H*-pyrrole-3-carboxamido)hexyl)(6-((5-methylheptan-2-yl)oxy)-6-oxohexyl)amino)octanoate;
(174) Heptadecan-9-yl 8-((2-hydroxy-6-(1*H*-pyrrole-3-carboxamido)hexyl)(6-((5-methyloctan-2-yl)oxy)-6-oxohexyl)amino)octanoate;
(175) Heptadecan-9-yl 8-((2-hydroxy-6-(1*H*-pyrrole-3-carboxamido)hexyl)(6-((6-methylheptan-2-yl)oxy)-6-oxohexyl)amino)octanoate;
(176) Heptadecan-9-yl 8-((2-hydroxy-6-(1*H*-pyrrole-3-carboxamido)hexyl)(6-((2-methyloctan-3-yl)oxy)-6-oxohexyl)amino)octanoate;
(177) Heptadecan-9-yl 8-((6-((2,6-dimethylheptan-4-yl)oxy)-6-oxohexyl)(2-hydroxy-6-(1*H-*pyrrole-3-carboxamido)hexyl)amino)octanoate; and
(178) Heptadecan-9-yl 8-((6-((7-ethyl-2-methylundecan-4-yl)oxy)-6-oxohexyl) (2-hydroxy-6-(1*H*-pyrrole-3-carboxamido)hexyl)amino)octanoate.

5. A lipid nanoparticle composition, comprising:
the lipid compound, an isomer thereof, or a salt thereof of one of claims 1 to 4;
active substances;
phospholipids;
structural lipids; and
PEGylated lipids (PEG-lipid).

6. The lipid nanoparticle composition of claim 5, wherein
the active substances are one or more types selected from the group consisting
of chemical therapeutics, small-molecule drugs, proteins, and nucleic acids.

7. The lipid nanoparticle composition of claim 5 or 6, wherein
the active substances are nucleic acids.

8. The lipid nanoparticle composition of one of claim 5 to 7, wherein
the phospholipids are selected from the group consisting of 1,2-distearoyl-sn-glycero-3-phosphocholine (DSPC), 1,2-dioleoyl-sn-glycero-3-phosphoethanolamine (DOPE), 1,2-dilinoleoyl-sn-glycero-3-phosphocholine (DLPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-diundecanoyl-sn-glycero-3-phosphocholine (DUPC), 1-palmitoyl-2-oleoyl-sn-glycero-3-phosphocholine (POPC), 1,2-di-O-octadecenyl-sn-glycero-3-phosphocholine (18:0 diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoylphosphatidylglycerol (DPPG), palmitoyloleoylphosphatidylethanolamine (POPE), distearoyl-phosphatidylethanolamine (DSPE), dipalmitoyl phosphatidyl ethanolamine (DPPE), dimyristoylphosphoethanolamine (DMPE), 1-stearoyl-2-oleoyl-phosphatidylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine (LPE), and a mixture thereof.

9. The lipid nanoparticle composition of one of claim 5 to 8, wherein
the structural lipids are selected from the group consisting of cholesterol, pecosterol, cytosterol, ergosterol, stigmasterol, bracicasterol, tomatidine, ursolic acid, alpha-tocopherol, and a mixture thereof.

10. The lipid nanoparticle composition of one of claim 5 to 9, wherein
the PEGylated lipids are selected from the group consisting of PEGylated phosphatidylethanolamine, PEGylated phosphatidic acid, PEGylated ceramide (PEG-CER), PEGylated dialkylamine, PEGylated diacylglycerol (PEG-DEG), PEGylated dialkyl glycerol, and a mixture thereof, and for example, PEG-lipids are selected from the group consisting of PEG-c-DOMG, PEG-DMG, PEG-DLPE, PEG-DMPE, PEG-DPPC, PEG-DSPE, and a mixture thereof.

11. A method for preparing an active substance-lipid nanoparticle, the method comprising: mixing an organic phase with an aqueous phase, the organic phase in which the lipid compound, an isomer thereof, or a salt thereof according to one of claims 1 to 4, phospholipids, structural lipids, and PEGylated lipids are mixed at a molar ratio of (20-60):(0-25):(30-60):(0-5); and the aqueous phase in which the active substance is dissolved, wherein the ratio (N/P ratio) of the number of ionizable nitrogen atoms of the lipid compound, an isomer thereof, or a salt thereof according to one of claims 1 to 4 to the number of phosphorus atoms from the anionic phosphodiester group in the active substance is in the range of 1.5-15, and the mass ratio of the lipid compound, an isomer thereof, or a salt thereof according to one of claims 1 to 4 to the active substance is (4-30):1.

12. A pharmaceutical composition comprising: the lipid nanoparticle composition of one of claim 5 to 10; and a pharmaceutically acceptable carrier.

13. A method for preventing or treating a disease of one of claim 5 to 10, comprising a step of administering the lipid nanoparticle composition of one of claim 5 to 10 to a subject in need of the prevention or treatment of the disease.
